# EUROPEAN PATENT APPLICATION

(11) **EP 4 628 493 A1**
(43) Date of publication of application: **08.10.2025**
(21) Application number: 23896847.3
(22) Date of filing: 29.11.2023
(51) Int. Cl.: C07D 519/00, A61K 31/34, A61P 35/00

(54) **NOVEL IRAK4 INHIBITOR, AND COMPOUND FOR INHIBITING AND DEGRADING IRAK4 PROTEIN AND PREPARATION METHOD THEREFOR AND USE THEREOF**

(30) Priority: 30.11.2022 CN 202211529378
(71) Applicant: Shenzhen Zhongge Biological Technology Co., Ltd., Futian District Shenzhen, Guangdong 518017 (CN)
(72) Inventor: DU, Xinming, Shenzhen, Guangdong 518017 (CN); MA, Junjun, Shenzhen, Guangdong 518017 (CN); CHEN, Zhaoqiang, Shenzhen, Guangdong 518017 (CN); DU, Lifei, Shenzhen, Guangdong 518017 (CN); WANG, Shaohui, Shenzhen, Guangdong 518017 (CN); CHEN, Bin, Shenzhen, Guangdong 518017 (CN); ZHANG, Peiyu, Shenzhen, Guangdong 518017 (CN)
(74) Representative: Keil & Schaafhausen Patentanwälte PartGmbB
(86) International application number: PCT/CN2023/135225
(87) International publication number: WO 2024/114704

(57) **Abstract**

The present application relates to a novel IRAK4 inhibitor of formula (I) and a compound for inhibiting and degrading IRAK4 protein, a preparation method therefor and use thereof, and also relates to a pharmaceutical composition comprising the IRAK4 inhibitor or the compound for inhibiting and degrading IRAK4 protein.

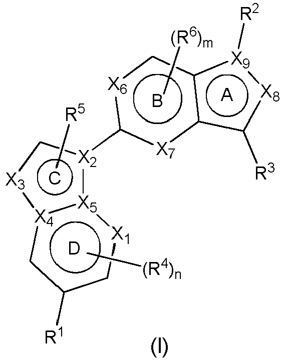

## Description

The present application claims priority to Chinese Patent Application No. CN202211529378.1 filed on November 30, 2022, which is incorporated herein by reference in its entirety.

### TECHNICAL FIELD

The present application relates to a novel IRAK4 inhibitor and a compound for inhibiting and degrading IRAK4 protein, a preparation method therefor and use thereof, and also relates to a pharmaceutical composition comprising the IRAK4 inhibitor or the compound for inhibiting and degrading IRAK4 protein.

### BACKGROUND

IRAK4 is a serine/threonine protein kinase and belongs to the Interleukin-1 receptor-associated kinase family, which includes four subtypes, i.e., IRAK1, IRAK2, IRAK3 (or IRAKM), and IRAK4. IRAK1, IRAK2, and IRAK4 promote the release of inflammatory factors, while IRAK3 is involved in the process of inflammation inhibition. Among the four subtypes, the biological function of IRAK4 has been clearly elucidated. When TLR or IL-1R senses external signal stimulation, the Myddosome complex formed by IRAK4 activates MAPK and NF-κB pathways, thereby releasing various inflammatory factors.

Research proves that IRAK4 is highly expressed in various tumor cells and inflammation models, and the development of an inhibitor targeting IRAK4 increasingly becomes an important direction for the treatment of autoimmune diseases and tumors. IRAK4 has two functions: kinase activity and skeleton activity, which play an important role in downstream signal regulation. Traditional small molecule IRAK4 kinase inhibitors can not achieve ideal treatment effect by solely inhibiting its kinase activity, while also facing subsequent challenges such as target protein mutation-induced drug resistance.

Proteolysis Targeting Chimera (PROTAC) technology is a new technology that has emerged in recent years. Since its introduction in 2001, this technology has attracted considerable interest, with multiple drugs developed based on this technology having entered clinical trials, some advancing to Phase II studies. PROTAC, as a heterogeneous bifunctional molecule, consists of a small molecule inhibitor capable of recognizing a target protein at one end, a linking group, and a ligand capable of recognizing an E3 ubiquitin ligase at the other end. The bifunctional molecule recognizes a target protein and binds a target protein to an E3 ubiquitin ligase to form a ternary complex in vivo, and then ubiquitination labeling is carried out on the target protein, thereby starting a ubiquitin-proteasome-dependent degradation pathway. Compared with traditional small-molecule inhibitors, the PROTAC technology realizes simultaneous inhibition of two functions of IRAK4 by degrading IRAK4 protein, thereby effectively solving the problems of insufficient activity of small-molecule inhibitors or mutation of target proteins. At present, no PROTAC drugs targeting IRAK4 have entered the clinical research stage in China.

There is a need to develop a novel small-molecule inhibitor of IRAK4 and a PROTAC molecule targeting IRAK4 for the treatment of a disease, a disorder, or a condition associated with IRAK4 protein kinase.

### SUMMARY

The present disclosure provides a novel small-molecule inhibitor of IRAK4 and a PROTAC molecule targeting IRAK4 for the treatment of a disease, a disorder, or a condition associated with IRAK4 protein kinase. The compounds of the present disclosure exhibit high selectivity for IRAK4 and can inhibit, or inhibit and degrade the IRAK4 protein kinase. In addition, the compounds of the present disclosure show more excellent properties such as better physicochemical properties (e.g., solubility and physical and/or chemical stability), improved pharmacokinetic properties (e.g., improved bioavailability, improved metabolic stability, and suitable half-life and duration of action), improved safety (less toxicity (e.g., reduced cardiotoxicity) and/or fewer side effects), and less susceptibility to drug resistance.

In one aspect, the present disclosure provides a compound of formula (I) as defined below: 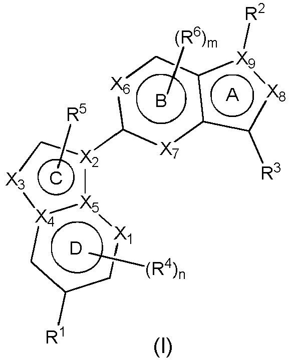 or a stereoisomer, a tautomer, a diastereoisomer, a racemate, a cis-trans isomer, an isotope-labeled compound (preferably a deuteride), an N-oxide, a metabolite, an ester, a prodrug, a crystalline form, a hydrate, a solvate, or a pharmaceutically acceptable salt thereof.

In another aspect, the present disclosure provides use of the compound of formula (I) or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof in the preparation of a proteolysis targeting chimera (PROTAC).

In another aspect, the present disclosure provides a proteolysis targeting chimera (PROTAC) comprising a moiety with IRAK4 protein kinase inhibitory activity, wherein the moiety is derived from the compound of formula (I) or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure.

In another aspect, the present disclosure provides a pharmaceutical composition comprising the compound of formula (I) or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure or the PROTAC molecule of the present disclosure, and a pharmaceutically acceptable excipient, carrier, or diluent. The pharmaceutical composition is preferably a solid formulation, a liquid formulation, or a transdermal formulation.

In another aspect, the present disclosure provides use of the compound of formula (I) or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure, or the PROTAC molecule of the present disclosure, or the pharmaceutical composition of the present disclosure for the preparation of a medicament for treating a disease, a disorder, or a condition associated with IRAK4 protein kinase.

In another aspect, the present disclosure provides a method for treating a disease, a disorder, or a condition associated with IRAK4 protein kinase, which comprises administering to an individual in need thereof a therapeutically effective amount of the compound of formula (I) or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure, or the PROTAC molecule of the present disclosure, or the pharmaceutical composition of the present disclosure.

In another aspect, the present disclosure provides a method for preparing the compound of formula (I) of the present disclosure and the PROTAC molecule of the present disclosure.

### DETAILED DESCRIPTION

### Definitions

Unless otherwise defined below, all technical and scientific terms used herein are intended to have the same meaning as commonly understood by those of ordinary skill in the art. Reference to the techniques used herein is intended to refer to techniques commonly understood in the art, including those variations of or alternatives to techniques that would be apparent to those of ordinary skill in the art. Although the following terms are believed to be well understood by those skilled in the art, the following definitions are set forth to better explain the present disclosure.

The terms "include", "comprise", "have", "contain" or "involve", and other variant forms thereof herein, are inclusive or open-ended and do not exclude other unrecited elements or method steps (that is, these terms also encompass the terms "substantially consist of' and "consist of').

As used herein, the term "alkylene" refers to saturated divalent hydrocarbyl, preferably saturated divalent hydrocarbyl having 1, 2, 3, 4, 5, or 6 carbon atoms, e.g., methylene, ethylene, propylene, or butylene.

As used herein, the term "alkyl" is defined as a linear or branched saturated aliphatic hydrocarbon. In some embodiments, the alkyl has 1-12, e.g., 1-6, carbon atoms. For example, as used herein, the term "C₁₋₆ alkyl" refers to a linear or branched group of 1-6 carbon atoms (e.g., methyl, ethyl, n-propyl, isopropyl, n-butyl, isobutyl, sec-butyl, tert-butyl, n-pentyl, or n-hexyl) that is optionally substituted with one or more (such as 1-3) suitable substituents such as halogen (in which case the group is referred to as "haloalkyl") (e.g., CF₃, C₂F₅, CHF₂, CH₂F, CH₂CF₃, CH₂Cl, -CH₂CH₂CF₃, or the like). The term "C₁₋₄ alkyl" refers to a linear or branched aliphatic hydrocarbon chain of 1-4 carbon atoms (i.e., methyl, ethyl, n-propyl, isopropyl, n-butyl, isobutyl, sec-butyl, or tert-butyl).

As used herein, the term "alkoxy" refers to -O-alkyl, wherein the alkyl is as defined above. The term "C₁₋₆ alkoxy" refers to a linear or branched alkoxy having 1-6 carbon atoms (e.g., methoxy, ethoxy, n-propoxy, isopropoxy, tert-butoxy, n-pentoxy, or n-hexoxy) that is optionally substituted with one or more (such as 1-3) suitable substituents such as halogen (in which case the group is referred to as "haloalkoxy") (e.g., -OCF₃, -OC₂F₅, -OCHF₂, -OCH₂F, -OCH₂CF₃, -OCH₂Cl, -OCH₂CH₂CF₃, or the like). The term "C₁₋₄ alkoxy" refers to a linear or branched alkoxy of 1-4 carbon atoms (e.g., methoxy, ethoxy, n-propoxy, isopropoxy, or tert-butoxy).

As used herein, the term "alkylthio" refers to -S-alkyl, wherein the alkyl is as defined above. The term "C₁₋₆ alkylthio" refers to a linear or branched alkylthio having 1-6 carbon atoms (e.g., methylthio, ethylthio, or propylthio) that is optionally substituted with one or more (such as 1-3) suitable substituents such as halogen (in which case the group is referred to as "haloalkylthio") (e.g., -SCF₃, -SC₂F₅, -SCHF₂, -SCH₂F, - SCH₂CF₃, -SCH₂Cl, -SCH₂CH₂CF₃, or the like). The term "C₁₋₄ alkylthio" refers to a linear or branched alkylthio having 1-4 carbon atoms (e.g., methylthio, ethylthio, or propylthio).

As used herein, the terms "cyclohydrocarbyl", "hydrocarbon ring", and "cyclohydrocarbylene" refer to saturated (i.e., "cycloalkyl" and "cycloalkylene") or partially unsaturated (i.e., having one or more double and/or triple bonds within the ring) monocyclic or polycyclic hydrocarbon rings having, e.g., 3-10 (suitably 3-8, and more suitably 3-7 or 3-6) ring carbon atoms, including, but not limited to, cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), and the like.

As used herein, the term "cycloalkyl" refers to a saturated monocyclic or polycyclic (such as bicyclic) hydrocarbon ring (e.g., a monocyclic ring, such as cyclopropyl, cyclobutyl, cyclopentyl, cyclohexyl, cycloheptyl, cyclooctyl, or cyclononyl, or a bicyclic ring, including spirocycle, fused, or bridged systems (such as bicyclo[1.1.1]pentyl, bicyclo[2.2.1]heptyl, bicyclo[3.2.1]octyl, bicyclo[5.2.0]nonyl, decahydronaphthyl, and the like), which is optionally substituted with one or more (such as 1-3) suitable substituents. The cycloalkyl has 3-15 carbon atoms, suitably 3-10 carbon atoms. For example, the term "C₃₋₆ cycloalkyl" refers to a saturated or partially unsaturated non-aromatic monocyclic or polycyclic (such as bicyclic) hydrocarbon ring of 3-6 ring-forming carbon atoms (e.g., cyclopropyl, cyclobutyl, cyclopentyl, or cyclohexyl). The cycloalkyl is optionally substituted with one or more (such as 1-3) suitable substituents, for example methyl-substituted cyclopropyl.

As used herein, the term "heterocyclyl" refers to a saturated (i.e., "heterocycloalkyl") or partially unsaturated monovalent monocyclic or bicyclic group having 2, 3, 4, 5, 6, 7, 8, or 9 carbon atoms and one or more (e.g., one, two, three, or four) heteroatom-containing groups selected from C(=O), O, S, S(=O), S(=O)₂, and NR^{a} in the ring, wherein R^{a} represents a hydrogen atom or C₁₋₆ alkyl or C₁₋₆ haloalkyl. The heterocyclyl may be attached to the rest of the molecule through any one of the carbon atoms or the nitrogen atom (if present). In particular, 3-10 membered heterocyclyl is a group having 3-10 (e.g., 3-7, 4-6, or 5-6) carbon atoms and heteroatoms in the ring, such as, but not limited to, oxiranyl, aziridinyl, azetidinyl, oxetanyl, tetrahydrofuranyl, dioxolinyl, pyrrolidinyl, pyrrolidinonyl, imidazolidinyl, pyrazolidinyl, pyrrolinyl, tetrahydropyranyl, piperidinyl, morpholinyl, dithianyl, thiomorpholinyl, piperazinyl, or trithianyl.

As used herein, the term "heterocyclyl" encompasses a fused ring structure, the attachment point of which to other groups may be on any one ring of the fused ring structure. Thus, the heterocyclyl of the present disclosure also includes, but is not limited to, heterocyclyl-fused heterocyclyl, heterocyclyl-fused cycloalkyl, monoheterocyclyl-fused monoheterocyclyl, and monoheterocyclyl-fused monocycloalkyl, such as 3-7 membered (mono)heterocyclyl-fused 3-7 membered (mono)heterocyclyl, 3-7 membered (mono)heterocyclyl-fused (mono)cycloalkyl, and 3-7 membered (mono)heterocyclyl-fused C₄₋₆ (mono)cycloalkyl, examples of which include, but are not limited to, pyrrolidinyl-fused cyclopropyl, cyclopentyl-fused azacyclopropyl, pyrrolidinyl-fused cyclobutyl, pyrrolidinyl-fused pyrrolidinyl, pyrrolidinyl-fused piperidinyl, pyrrolidinyl-fused piperazinyl, piperidinyl-fused morpholinyl,

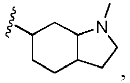

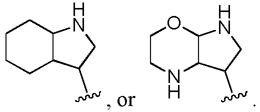

As used herein, the term "heterocyclyl" encompasses bridged heterocyclyl and spiroheterocyclyl.

As used herein, the term "bridged heterocycle" refers to a ring structure containing one or more (e.g., 1, 2, 3, or 4) heteroatoms (e.g., oxygen atoms, nitrogen atoms, and/or sulfur atoms) formed by two saturated rings sharing two ring atoms which are not directly linked, including, but not limited to, 7-10 membered bridged heterocycle, 8-10 membered bridged heterocycle, 7-10 membered nitrogen-containing bridged heterocycle, 7-10 membered oxygen-containing bridged heterocycle, 7-10 membered sulfur-containing bridged heterocycle, and the like, such as 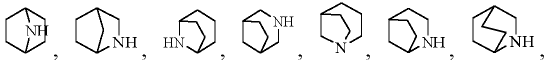 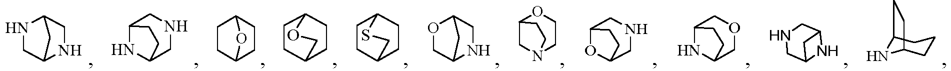 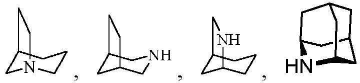 , etc. The "nitrogen-containing bridged heterocycle", "oxygen-containing bridged heterocycle", and "sulfur-containing bridged heterocycle" optionally further comprise one or more other heteroatoms selected from oxygen, nitrogen, and sulfur.

As used herein, the term "monospiroheterocyclyl" refers to a ring structure containing one or more (e.g., 1, 2, 3, or 4) heteroatoms (e.g., oxygen atoms, nitrogen atoms, or sulfur atoms) formed by two or more saturated or partially unsaturated rings sharing one ring atom. "Monospiroheterocycloalkyl" is monospiroheterocyclyl in which each ring forming the spirocycle is a saturated ring. Monospiroheterocycloalkyl includes, but is not limited to, 5-11 membered monospiroheterocycloalkyl, 6-10 membered monospiroheterocycloalkyl, 7-10 membered monocyclic heterocycloalkyl, 6-10 membered nitrogen-containing spiroheterocycloalkyl, 6-10 membered oxygen-containing spiroheterocycloalkyl, 6-10 membered sulfur-containing spiroheterocycloalkyl, and the like. Monospiroheterocycloalkyl may include, e.g., 3-membered/5-membered ring systems, 4-membered/4-membered ring systems, 4-membered/5-membered ring systems, 4-membered/6-membered ring systems, 5-membered/5-membered ring systems, 5-membered/6-membered ring systems, and 6-membered/6-membered ring systems, wherein the number of each ring includes the spiro atom. Examples include, but are not limited to, 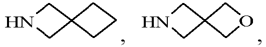 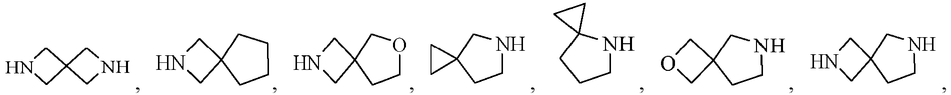 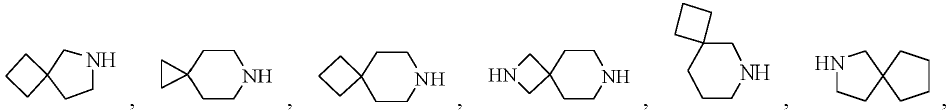 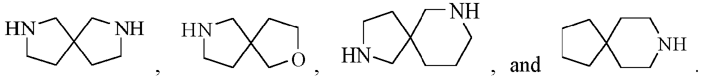 The "nitrogen-containing monospiroheterocycloalkyl", "oxygen-containing monospiroheterocycloalkyl", or "sulfur-containing monospiroheterocycloalkyl" optionally further contains one or more other heteroatoms selected from oxygen, nitrogen, and sulfur. The term "6-10 membered nitrogen-containing monospiroheterocycloalkyl" refers to spiroheterocyclyl containing a total of 6-10 ring atoms, at least one of which is a nitrogen atom.

As used herein, "○" shown in ring structures, such as ring A, ring B, ring C, and ring D in formula (I), formula (I-1), and formula (I-2) herein, means that each of the corresponding ring structures is aromatic (i.e., aryl or heteroaryl).

As used herein, the term "aryl" refers to an all-carbon monocyclic or fused polycyclic aromatic group having a conjugated π-electron system. For example, as used herein, the term "C₆₋₁₄ aryl" refers to an aromatic group containing 6-14 (e.g., 6-12) carbon atoms, such as phenyl or naphthyl. The aryl is optionally substituted with one or more (such as 1-3) suitable substituents (e.g., halogen, -OH, -CN, -NO₂, C₁₋₆ alkyl, and the like).

As used herein, the term "heteroaryl" refers to a monovalent monocyclic, bicyclic, or tricyclic aromatic ring system that has 5, 6, 8, 9, 10, 11, 12, 13, or 14 ring atoms, in particular 1, 2, 3, 4, 5, 6, 9, or 10 carbon atoms, and contains at least one heteroatom that may be identical or different (which is, e.g., oxygen, nitrogen, or sulfur). In addition, in each case, the heteroaryl may be a benzo-fused group. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl (including 1,2,3-triazolyl and 1,2,4-triazolyl), thiadiazolyl, etc., and benzo derivatives thereof; or pyridinyl, pyridazinyl, pyrimidinyl, pyrazinyl, triazinyl, etc., and benzo derivatives thereof.

As used herein, the term "halo" or "halogen" group is defined to include F, Cl, Br, or I.

As used herein, the term "haloalkyl" refers to alkyl substituted with one or more (such as 1-3) identical or different halogen atoms, and the alkyl is as defined herein. The terms "C₁₋₈ haloalkyl," "C₁₋₆ haloalkyl," and "C₁₋₄ haloalkyl" refer to haloalkyl groups having 1-8 carbon atoms, 1-6 carbon atoms, and 1-4 carbon atoms, respectively, such as -CF₃, -C₂F₅, -CHF₂, -CH₂F, -CH₂CF₃, -CH₂Cl, -CH₂CH₂CF₃, or the like.

As used herein, the term "nitrogen-containing heterocycle" refers to a saturated or unsaturated monocyclic or bicyclic group having 2, 3, 4, 5, 6, 7, 8, 9, 10, 11, 12, or 13 carbon atoms and at least one nitrogen atom in the ring, which may also optionally contain one or more (e.g., one, two, three, or four) ring members selected from N, O, C=O, S, S=O, and S(=O)₂. The nitrogen-containing heterocycle is attached to the rest of the molecule through a nitrogen atom. The nitrogen-containing heterocycle is preferably a saturated nitrogen-containing monocyclic ring. In particular, a 3-14 membered nitrogen-containing heterocycle is a group having 3-14 carbon atoms and heteroatoms (at least one of which is a nitrogen atom) in the ring, including, but not limited to, a three-membered nitrogen-containing heterocycle (e.g., aziridinyl), a four-membered nitrogen-containing heterocycle (e.g., azetidinyl), a five-membered nitrogen-containing heterocycle (e.g., pyrrolyl, pyrrolidinyl (pyrrolidine ring), pyrrolinyl, pyrrolidinonyl, imidazolyl, imidazolidinyl, imidazolinyl, pyrazolyl, or pyrazolinyl), a six-membered nitrogen-containing heterocycle (e.g., piperidinyl (piperidine ring), morpholinyl, thiomorpholinyl, or piperazinyl), a seven-membered nitrogen-containing heterocycle, and the like.

The term "substitute" means that one or more (e.g., one, two, three, or four) hydrogen atoms on a specified atom are replaced with a selection from the designated groups, provided that the normal valency of the atom specified under the present circumstances is not exceeded and that the replacement results in a stable compound. A combination of substituents and/or variables is permissible only if the combination results in a stable compound.

If a substituent is described as "optionally substituted with...", the substituent may be (1) unsubstituted or (2) substituted. If a carbon of a substituent is described as being optionally substituted with one or more of a list of substituents, one or more hydrogen atoms on the carbon (to the extent of any hydrogen atoms present) may be replaced individually and/or together with an independently selected optional substituent. If a nitrogen of a substituent is described as being optionally substituted with one or more of a list of substituents, one or more hydrogen atoms on the nitrogen (to the extent of any hydrogen atoms present) may each be replaced with an independently selected optional substituent.

If a substituent is described as being "independently selected from" one group, each substituent is selected independently of the other. Thus, each substituent may be identical to or different from another (other) substituent(s).

As used herein, the term "one or more" refers to one or more than one, such as 2, 3, 4, 5, or 10, under reasonable conditions.

Unless otherwise specified, as used herein, the attachment point of a substituent may be from any suitable position of the substituted group.

When a bond of a substituent is shown to pass through a bond (a floating bond) connecting two atoms in a ring, such a substituent may be bonded to any one of the ring-forming atoms in the substitutable ring, unless otherwise specified. In the case where it is shown that an available ring member carries a substitutable hydrogen atom, the substitutable hydrogen atom is substantially substituted (i.e., absent) when the floating bond is bonded to the available ring member.

The present disclosure also includes all pharmaceutically acceptable isotope-labeled compounds, which are identical to the compounds of the present disclosure, except that one or more atoms are replaced by an atom having the same atomic number, but an atomic mass or mass number different from the atomic mass or mass number predominant in nature. Examples of isotopes suitable for incorporation into the compounds of the present disclosure include (but are not limited to) isotopes of hydrogen (e.g., deuterium (D, ²H) and tritium (T, ³H)), isotopes of carbon (e.g., ¹¹C, ¹³C, and ¹⁴C), isotopes of chlorine (e.g., ³⁶Cl), isotopes of fluorine (e.g., ¹⁸F), isotopes of iodine (e.g., ¹²³I and ¹²⁵I), isotopes of nitrogen (e.g., ¹³N and ¹⁵N), isotopes of oxygen (e.g., ¹⁵O, ¹⁷O, and ¹⁸O), isotopes of phosphorus (e.g., ³²P), and isotopes of sulfur (e.g., ³⁵S). Certain isotope-labeled compounds of the present disclosure (e.g., those into which a radioactive isotope is incorporated) can be used in drug and/or substrate tissue distribution studies (e.g., assays). The radioactive isotopes tritium (i.e., ³H) and carbon-14 (i.e., ¹⁴C) are particularly useful for this purpose because of their ease of incorporation and detection. Substitution with positron emitting isotopes (e.g., ¹¹C, ¹⁸F, ¹⁵O, and ¹³N) can be used to examine substrate receptor occupancy in positron emission tomography (PET) studies. The isotope-labeled compounds of the present disclosure can be prepared by processes analogous to those described in the accompanying routes and/or in the examples and preparations by using an appropriate isotopically labeled reagent in place of the non-labeled reagent previously employed. The pharmaceutically acceptable solvates of the present disclosure include those in which the crystallization solvent may be isotopically substituted, e.g., D₂O, acetone-*d₆*, or DMSO-*d₆*.

The term "stereoisomer" refers to an isomer formed due to at least one asymmetric center. In compounds having one or more (e.g., 1, 2, 3, or 4) asymmetric centers, racemic mixtures, single enantiomers, mixtures of diastereoisomers, and individual diastereoisomers may be produced. Certain individual molecules may also exist as geometric isomers (cis/trans). Similarly, the compounds of the present disclosure may exist as a mixture of two or more structurally different forms in rapid equilibrium (commonly referred to as tautomers). Representative examples of tautomers include keto-enol tautomers, phenol-keto tautomers, nitroso-oxime tautomers, imine-enamine tautomers, etc. It should be understood that the scope of the present application encompasses all such isomers or mixtures thereof in any proportion (e.g., 60%, 65%, 70%, 75%, 80%, 85%, 90%, 95%, 96%, 97%, 98%, or 99%).

A solid line (-), a solid wedge ( 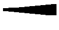 ), or a dashed wedge ( 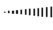 ) may be used herein to depict chemical bonds of the compounds of the present disclosure. Using the solid line to depict a bond that is bonded to an asymmetric carbon atom is intended to indicate that all possible stereoisomers (e.g., particular enantiomers, racemic mixtures, etc.) at the carbon atom are included. Using the solid or dashed wedge to depict a bond that is bonded to an asymmetric carbon atom is intended to indicate that the stereoisomers shown are present. When present in a racemic mixture, the solid and dashed wedges are used to define the relative stereochemistry rather than the absolute stereochemistry. Unless otherwise indicated, the compounds of the present disclosure are intended to be present in the form of stereoisomers (including cis and trans isomers, optical isomers (e.g., R and S enantiomers), diastereoisomers, geometric isomers, rotamers, conformers, atropisomers, and mixtures thereof). The compounds of the present disclosure may exhibit no less than one type of isomerism and consist of mixtures thereof (e.g., racemic mixtures and diastereoisomer pairs).

It should be understood that certain compounds of the present disclosure may be present in free form for use in therapy or, where appropriate, in the form of a pharmaceutically acceptable derivative thereof. In the present disclosure, the pharmaceutically acceptable derivatives include, but are not limited to, pharmaceutically acceptable salts, esters, solvates, metabolites, or prodrugs, which, upon administration to a patient in need thereof, are capable of providing, directly or indirectly, the compound of the present disclosure or a metabolite or residue thereof. Thus, when reference is made herein to "the compound of the present disclosure", it is also intended to encompass the various derivative forms of the compound described above.

The pharmaceutically acceptable salts of the compounds of the present disclosure include acid addition salts and base addition salts thereof.

Suitable acid addition salts are formed from acids that form pharmaceutically acceptable salts. Examples include aspartate, benzoate, bicarbonate/carbonate, bisulfate/sulfate, fumarate, glucoheptonate, gluconate, glucuronate, hexafluorophosphate, hydrobromide/bromide, hydroiodide/iodide, maleate, malonate, methyl sulfate, naphthylate, nicotinate, nitrate, orotate, oxalate, palmitate, and other similar salts.

Suitable base addition salts are formed from bases that form pharmaceutically acceptable salts. Examples include aluminum salt, arginine salt, choline salt, diethylamine salt, lysine salt, magnesium salt, meglumine salt, potassium salt, and other similar salts.

A review of suitable salts is found in Stahl and Wermuth, "Handbook of Pharmaceutical Salts: Properties, Selection, and Use" (Wiley-VCH, 2002). Methods for preparing the pharmaceutically acceptable salts of the compounds of the present disclosure are known to those skilled in the art.

As used herein, the term "ester" refers to an ester derived from the compounds of formulas in the present application, including physiologically hydrolyzable esters (which can be hydrolyzed under physiological conditions to release the compounds of the present disclosure in the form of free acids or alcohols). The compounds of the present disclosure may themselves also be esters.

The present disclosure encompasses all possible crystalline forms or polymorphs of the compounds of the present disclosure, which may be single polymorphs or mixtures of more than one polymorph in any ratio.

The compounds of the present disclosure may be present in the form of solvates (preferably hydrates), and the compounds of the present disclosure contain a polar solvent as a structural element of the crystal lattice of the compound, particularly, for example, water, methanol, or ethanol. The amount of the polar solvent, especially water, may be present in a stoichiometric or non-stoichiometric ratio.

Also included within the scope of the present disclosure are metabolites of the compounds of the present disclosure, i.e., substances formed in vivo upon administration of the compounds of the present disclosure. Such products may result, for example, from oxidation, reduction, hydrolysis, amidation, deamidation, esterification, defatting, enzymatic digestion, and the like of the administered compound. Thus, the present disclosure includes metabolites of the compounds of the present disclosure, including compounds made by the process of contacting the compounds of the present disclosure with a mammal for a time sufficient to produce metabolites thereof.

Also included within the scope of the present disclosure are prodrugs of the compounds of the present disclosure, which are certain derivatives of the compounds of the present disclosure that may themselves have little or no pharmacological activity and, when administered into or onto the body, can be converted to the compounds of the present disclosure having the desired activity, for example, by hydrolysis. Generally, such prodrugs will be functional derivatives of the compounds, which are readily converted into desired therapeutically active compounds in vivo. For additional information on the use of prodrugs, reference may be made to "Pro-drugs as Novel Delivery Systems", volume 14, ACS Symposium Series (T. Higuchi and V. Stella) and "Bioreversible Carriers in Drug Design," Pergamon Press, 1987 (E. B. Roche Ed., American Pharmaceutical Association). The prodrugs of the present disclosure may be prepared, for example, by replacing appropriate functional groups present in the compounds of the present disclosure with certain moieties known to those skilled in the art as "pro-moieties" (for example, as described in "Design of Prodrugs", H. Bundgaard (Elsevier, 1985)).

The present disclosure further encompasses the compounds of the present disclosure containing a protective group. In any process of preparing the compounds of the present disclosure, it may be necessary and/or desirable to protect sensitive or reactive groups on any of the molecules concerned, thereby forming a chemically protected form of the compounds of the present disclosure. This may be realized by conventional protective groups, such as those described in Protective Groups in Organic Chemistry, ed. J.F.W McOmie, Plenum Press, 1973; and T.W. Greene & P.G.M. Wuts, Protective Groups in Organic Synthesis, John Wiley & Sons, 1991, which are incorporated herein by reference. The protective groups may be removed at an appropriate subsequent stage by methods known in the art.

As used herein, the term "about" means within ±10%, preferably within ±5%, and more preferably within ±2% of the stated numerical value.

### Compound as IRAK4 Inhibitor

In one general aspect, the present disclosure provides a compound of formula (I): 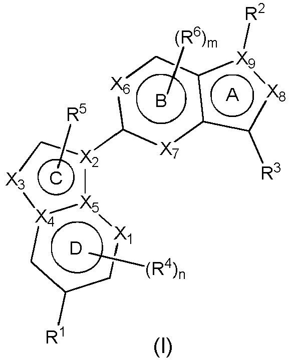
or a stereoisomer, a tautomer, a diastereoisomer, a racemate, a cis-trans isomer, an isotope-labeled compound (preferably a deuteride), an N-oxide, a metabolite, an ester, a prodrug, a crystalline form, a hydrate, a solvate, or a pharmaceutically acceptable salt thereof as an IRAK4 inhibitor,
wherein:
   X₁, X₂, X₃, X₄, X₅, X₆, X₇, X₈, and X₉ may each independently be C, CR, or N, provided that the valency of all atoms is met and X₄ and X₅ are not both N at the same time;
   R, R¹, R⁴, and R⁵ are each independently selected from: hydrogen, deuterium, R⁷, halogen, CN, NO₂, C₁₋₆ alkyl, -OR^{1f}, -SR^{1f}, -NR^{1d}R^{1e}, -S(O)₂R^{1f}, -S(O)R^{1f}, -S(O)₂-NR^{1d}R^{1e}, -S(O)-NR^{1d}R^{1e}, -P(O)(OR^{1f})₂, - P(O)(NR^{1d}R^{1e})₂, -CF(R^{1f})₂, -CF₂(R^{1f}), -CF₃, -CCl(R^{1f})₂, -CCl₂(R^{1f}), -CCl₃, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, -C(O)R^{1f}, -C(O)OR^{1f}, -C(O)NR^{1d}R^{1e}, -C(O)NR^{1f}-OR^{1f}, -OC(O)R^{1f}, - OC(O)NR^{1d}R^{1e}, -NR^{1f}-C(O)OR^{1f}, -NR^{1f}-C(O)R^{1f}, -NR^{1f}-C(O)NR^{1d}R^{1e}, and -NR^{1f}-S(O)₂R^{1f}, wherein
   p is 1, 2, or 3;
   n is 0, 1, or 2, wherein when n is 1 or 2, R⁴ is attached to an available ring member of ring D; and
   R⁵ is attached to an available ring member of ring C;
   R² is selected from: saturated or partially unsaturated C₃₋₇ cyclohydrocarbyl, 3-7 membered saturated or partially unsaturated heterocyclyl having 1-3 heteroatoms independently selected from nitrogen, oxygen, and sulfur, C₆₋₁₀ aryl, and 5-10 membered heteroaryl having **1-4** heteroatoms independently selected from nitrogen, oxygen, and sulfur, wherein the cyclohydrocarbyl, heterocyclyl, aryl, and heteroaryl are optionally substituted with 1, 2, or more substituents independently selected from halogen, CN, OH, SH, NO₂, C₁₋₆ alkyl, C₁₋₆ haloalkyl, C₁₋₆ alkoxy, C₁₋₆ alkylthio, -NR^{2d}R^{2e}, -C(O)OR^{2f}, and -C(O)NR^{2d}R^{2e}, and in the case where two substituents are attached to the same ring carbon atom of the cyclohydrocarbyl or heterocyclyl, the two substituents, together with the ring carbon atom to which they are attached, optionally form saturated or partially unsaturated optionally substituted C₃₋₇ cyclohydrocarbyl or 3-7 membered saturated or partially unsaturated optionally substituted heterocyclyl having 1-3 heteroatoms independently selected from nitrogen, oxygen, and sulfur;
   R³ and R⁶ are each independently selected from: H, R⁸, halogen, CN, NO₂, C₁₋₆ alkyl, -OR^{3f}, -SR^{3f}, - NR^{3d}R^{3e}, -S(O)₂R^{3f}, -S(O)R^{3f}, -S(O)₂-NR^{3d}R^{3e}, -S(O)-NR^{3d}R^{3e}, -P(O)(OR^{3f})₂, -P(O)(NR^{3d}R^{3e})₂, -CF(R^{3f})₂,-CF₂(R^{3f}), -CF₃, -CCl(R^{3f})₂, -CCl₂(R^{3f}), -CCl₃, -(CR^{3a}R^{3b})_{q}-OR^{3f}, -(CR^{3a}R^{3b})_{q}-C(O)OR^{3f}, -(CR^{3a}R^{3b})_{q}-NR^{3d}R^{3e}, -C(O)R^{3f}, -C(O)OR^{3f}, and -C(O)NR^{3d}R^{3e}, wherein
   q is 1, 2, or 3;
   m is 0, 1, 2, or 3, wherein when m is 1, 2, or 3, R⁶ is attached to an available ring member of ring B;
   R^{1a}, R^{1b}, R^{3a}, and R^{3b} are each independently selected from hydrogen, deuterium, halogen, CN, OH, SH, NO₂, C₁₋₆ alkyl, C₁₋₆ haloalkyl, C₁₋₆ alkoxy, C₁₋₆ alkylthio, and -NR^{7d}R^{7e}; or, R^{1a} and R^{1b}, or R^{3a} and R^{3b}, together with the carbon atom to which they are both attached, form R⁹;
   R^{1d}, R^{1e}, R^{2d}, R^{2e}, R^{3d}, R^{3e}, R^{7d}, and R^{7e} are each independently selected from hydrogen, deuterium, R¹¹, C₁₋₆ alkyl, and C₁₋₆ haloalkyl; or, R^{1d} and R^{1e}, or R^{2d} and R^{2e}, or R^{3d} and R^{3e}, or R^{7d} and R^{7e}, together with the nitrogen atom to which they are both attached, form R¹⁰;
   R^{1f}, R^{2f}, and R^{3f} are independently selected from hydrogen, deuterium, C₁₋₆ alkyl, C₁₋₆ haloalkyl, and R¹²;
   R⁷, R⁸, R⁹, R¹¹, and R¹² are each independently selected from: saturated or partially unsaturated C₃₋₇ cyclohydrocarbyl, 3-10 membered saturated or partially unsaturated heterocyclyl having 1-3 heteroatoms independently selected from nitrogen, oxygen, and sulfur, C₆₋₁₀ aryl, and 5-10 membered heteroaryl having 1-4 heteroatoms independently selected from nitrogen, oxygen, and sulfur, wherein the cyclohydrocarbyl, heterocyclyl, aryl, and heteroaryl are optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e};
   R¹⁰ is selected from: 3-7 membered saturated or partially unsaturated heterocyclyl having 1 nitrogen heteroatom and optionally 1-2 additional heteroatoms independently selected from nitrogen, oxygen, and sulfur, and 5-10 membered heteroaryl having 1 nitrogen heteroatom and optionally 1-3 additional heteroatoms independently selected from nitrogen, oxygen, and sulfur, wherein the heterocyclyl and heteroaryl are optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{10d}R^{10e}; and
   R^{8d}, R^{8e}, R^{10d}, and R^{10e} are each independently selected from hydrogen, deuterium, C₁₋₆ alkyl, and C₁₋₆ haloalkyl.

In some embodiments, the present disclosure provides the compound of formula (I), wherein R¹ and R⁴ are not both hydrogen or deuterium at the same time.

In some embodiments, the present disclosure provides the compounds of formula (I), wherein R^{1a}, R^{1b}, R^{3a}, and R^{3b} are each independently selected from hydrogen, halogen, CN, OH, SH, NO₂, C₁₋₄ alkyl, C₁₋₄ haloalkyl, C₁₋₄ alkoxy, C₁₋₄ alkylthio, and-NR^{7d}R^{7e}; or, R^{1a} and R^{1b}, or R^{3a} and R^{3b}, together with the carbon atom to which they are both attached, form R⁹.

In some embodiments, the present disclosure provides the compound of formula (I) wherein R^{1d}, R^{1e}, R^{2d}, R^{2e}, R^{3d}, R^{3e}, R^{7d}, and R^{7e} are each independently selected from hydrogen, R¹¹, C₁₋₄ alkyl, and C₁₋₄ haloalkyl; or, R^{1d} and R^{1e}, or R^{2d} and R^{2e}, or R^{3d} and R^{3e}, or R^{7d} and R^{7e}, together with the nitrogen atom to which they are both attached, form R¹⁰.

In some embodiments, the present disclosure provides the compound of formula (I), wherein R^{1f}, R^{2f}, and R^{3f} are independently selected from hydrogen, C₁₋₄ alkyl, C₁₋₄ haloalkyl, and R¹².

In some embodiments, the present disclosure provides the compound of formula (I) wherein R⁷, R⁸, R⁹, R¹¹, and R¹² are each independently selected from: saturated or partially unsaturated C₃₋₆ cyclohydrocarbyl, 3-10 membered saturated or partially unsaturated heterocyclyl having 1-3 heteroatoms independently selected from nitrogen, oxygen, and sulfur, C₆₋₁₀ aryl, and 5-6 membered heteroaryl having 1-3 heteroatoms independently selected from nitrogen, oxygen, and sulfur, wherein the cyclohydrocarbyl, heterocyclyl, aryl, and heteroaryl are optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e}.

In some embodiments, the present disclosure provides the compound of formula (I), wherein R¹⁰ is selected from: 3-6 membered saturated or partially unsaturated heterocyclyl having 1 nitrogen heteroatom and optionally 1-2 additional heteroatoms independently selected from nitrogen, oxygen, and sulfur, and 5-6 membered heteroaryl having 1 nitrogen heteroatom and optionally 1-2 additional heteroatoms independently selected from nitrogen, oxygen, and sulfur, wherein the heterocyclyl and heteroaryl are optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{10d}R^{10e}.

In some embodiments, the present disclosure provides the compound of formula (I), wherein R^{8d}, R^{8e}, R^{10d}, and R^{10e} are each independently selected from hydrogen, C₁₋₆ alkyl, and C₁₋₆ haloalkyl.

In a first sub-aspect, the present disclosure provides the compound of formula (I) described above, wherein R¹ is not hydrogen.

In some embodiments, R¹ is selected from: R⁷, halogen, CN, NO₂, C₁₋₄ alkyl, -OR^{1f}, -SR^{1f}, -NR^{1d}R^{1e}, - S(O)₂R^{1f}, -S(O)R^{1f}, -S(O)₂-NR^{1d}R^{1e}, -S(O)-NR^{1d}R^{1e}, -P(O)(OR^{1f})₂, -P(O)(NR^{1d}R^{1e})₂, -CF(R^{1f})₂, -CF₂(R^{1f}), - CF₃, -CCl(R^{1f})₂, -CCl₂(R^{1f}), -CCl₃, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, - C(O)R^{1f}, -C(O)OR^{1f}, -C(O)NR^{1d}R^{1e}, -C(O)NR^{1f}-OR^{1f}, -OC(O)R^{1f}, -OC(O)NR^{1d}R^{1e}, -NR^{1f}-C(O)OR^{1f}, -NR^{1f}-C(O)R^{1f}, -NR^{1f}-C(O)NR^{1d}R^{1e}, or -NR^{1f}-S(O)₂R^{1f}. In some embodiments, R¹ is selected from: R⁷, halogen, CN, NO₂, C₁₋₄ alkyl, -OR^{1f}, -SR^{1f}, -NR^{1d}R^{1e}, -CF(R^{1f})₂, -CF₂(R^{1f}), -CF₃, -CCl(R^{1f})₂, -CCl₂(R^{1f}), -CCl₃, - (CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, -C(O)OR^{1f}, or -C(O)NR^{1d}R^{1e}. In some embodiments, R¹ is selected from: R⁷, halogen, CN, -OR^{1f}, -NR^{1d}R^{1e}, -(CR^{1a}R^{1b})ₚ-OR^{1f}, -(CR^{1a}R^{1b})ₚ-C(O)OR^{1f}, -C(O)OR^{1f}, or -C(O)NR^{1d}R^{1e}. In some such embodiments, R⁷ is selected from: C₃₋₆ cycloalkyl, 4-6 membered saturated or partially unsaturated heterocyclyl having 1-3 heteroatoms independently selected from nitrogen, oxygen, and sulfur, phenyl, and 5-6 membered heteroaryl having 1-3 heteroatoms independently selected from nitrogen, oxygen, and sulfur, wherein the cycloalkyl, heterocyclyl, phenyl, and heteroaryl are optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e}. In some such embodiments, R⁷ is selected from: C₃₋₆ cycloalkyl, 4-6 membered heterocycloalkyl having 1-3 heteroatoms independently selected from nitrogen, oxygen, and sulfur, phenyl, and 5-6 membered heteroaryl having 1-3 heteroatoms independently selected from nitrogen, oxygen, and sulfur, wherein the cycloalkyl, heterocycloalkyl, phenyl, and heteroaryl are optionally substituted with one or more substituents independently selected from halogen, CN, OH, NO₂, C₁₋₄ alkyl, C₁₋₄ haloalkyl, C₁₋₄ alkoxy, and -NR^{8d}R^{8e}. In some such embodiments, R^{8d} and R^{8e} are preferably each independently selected from hydrogen and C₁₋₆ alkyl. Preferably, R^{8d} and R^{8e} are each independently selected from hydrogen and C₁₋₄ alkyl.

In some such embodiments, R⁷ is selected from: 4-6 membered heterocycloalkyl having 1 N atom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some such embodiments, R⁷ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R⁷ is selected from: azetidinyl, pyrrolidinyl, and piperidinyl.

In some such embodiments, p is 1.

In some such embodiments, R^{1a} and R^{1b} are each independently selected from hydrogen and C₁₋₆ alkyl.

In some such embodiments, R^{1a} and R^{1b} are each independently selected from hydrogen and C₁₋₄ alkyl. In some such embodiments, R^{1a} and R^{1b} are each independently hydrogen.

In some such embodiments, R^{1a} and R^{1b}, together with the carbon atom to which they are both attached, form R⁹.

In some such embodiments, R⁹ is preferably selected from: C₃₋₆ cycloalkyl, 3-6 membered saturated or partially unsaturated heterocyclyl having 1-3 heteroatoms independently selected from nitrogen, oxygen, and sulfur, C₆₋₁₀ aryl, and 5-6 membered heteroaryl having 1-3 heteroatoms independently selected from nitrogen, oxygen, and sulfur, wherein the cycloalkyl, heterocyclyl, aryl, and heteroaryl are optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e}. In some such embodiments, R^{8d} and R^{8e} are preferably each independently selected from hydrogen and C₁₋₆ alkyl. More preferably, R^{8d} and R^{8e} are each independently selected from hydrogen and C₁₋₄ alkyl.

In some such embodiments, R⁹ is preferably selected from: C₃₋₆ cycloalkyl, wherein the cycloalkyl is optionally substituted with one or more substituents independently selected from F, Cl, Br, CN, OH, NO₂, C₁₋₄ alkyl, C₁₋₄ haloalkyl, C₁₋₄ alkoxy, NH₂, -NH(C₁₋₄ alkyl) and N(C₁₋₄ alkyl)₂.

In some such embodiments, R⁹ is preferably selected from: cyclopropyl, cyclobutyl, cyclopentyl, and cyclohexyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R⁹ is preferably cyclopropyl.

In some such embodiments, R^{1d} and R^{1e} are each independently selected from hydrogen, R¹¹, and C₁₋₆ alkyl. In some such embodiments, R^{1d} and R^{1e} are each independently selected from hydrogen, R¹¹, and C₁₋₄ alkyl. In some such embodiments, R^{1d} and R^{1e} are each independently selected from hydrogen, R¹¹, methyl, ethyl, propyl, isopropyl, n-butyl, isobutyl, sec-butyl, and tert-butyl.

In some such embodiments, R^{1d} and R^{1e}, together with the nitrogen atom to which they are both attached, form R¹⁰.

In some such embodiments, R¹¹ is preferably selected from: C₃₋₆ cycloalkyl, 3-6 membered saturated or partially unsaturated heterocyclyl having 1-3 heteroatoms independently selected from nitrogen, oxygen, and sulfur, C₆₋₁₀ aryl, and 5-6 membered heteroaryl having 1-3 heteroatoms independently selected from nitrogen, oxygen, and sulfur, wherein the cycloalkyl, heterocyclyl, aryl, and heteroaryl are optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e}. In some such embodiments, R^{8d} and R^{8e} are preferably each independently selected from hydrogen and C₁₋₆ alkyl. Preferably, R^{8d} and R^{8e} are each independently selected from hydrogen and C₁₋₄ alkyl.

In some such embodiments, R¹¹ is selected from: C₃₋₆ cycloalkyl, and 3-6 membered heterocycloalkyl having 1 nitrogen heteroatom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some such embodiments, R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R¹¹ is selected from: cyclopropyl, cyclobutyl, cyclopentyl, cyclohexyl, azetidinyl, pyrrolidinyl, and piperidinyl, each of which is optionally substituted with one or more substituents independently selected from F and Cl.

In some such embodiments, R¹¹ is selected from cyclopropyl and

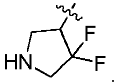

In some such embodiments, R¹⁰ is preferably selected from: 3-6 membered heterocycloalkyl having 1 nitrogen heteroatom and optionally 1-2 additional heteroatoms independently selected from nitrogen, oxygen, and sulfur, and 5-6 membered heteroaryl having 1 nitrogen heteroatom and optionally 1-2 additional heteroatoms independently selected from nitrogen, oxygen, and sulfur, wherein the heterocycloalkyl and heteroaryl are optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some such embodiments, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄)alkyl, and N(C₁₋₄ alkyl)₂.

In some such embodiments R¹⁰ is selected from: 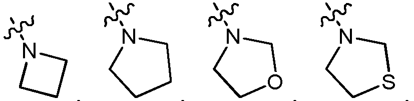 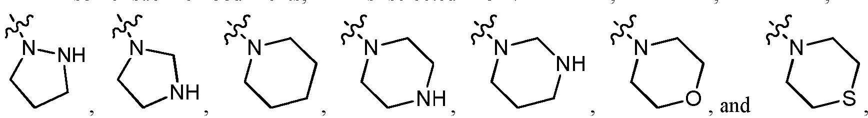 each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R¹⁰ is selected from: 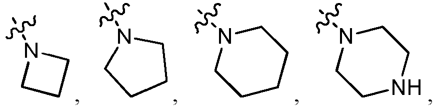 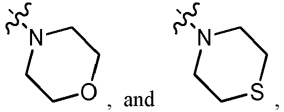 each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R^{1f} is selected from hydrogen and C₁₋₆ alkyl. In some such embodiments, R^{1f} is selected from hydrogen and C₁₋₄ alkyl. In some such embodiments, R^{1f} is hydrogen.

In some particular embodiments, R¹ is selected from: F, Cl, Br, CN, OH, NH₂, -NHCH₃, -C(O)OH, - C(O)NH₂, -CH₂NH₂, -CH₂OH,

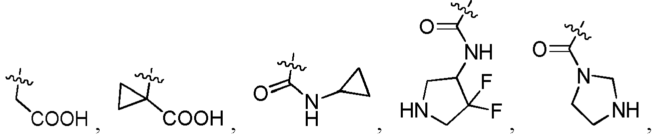

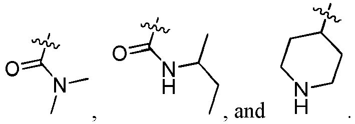

In some particular embodiments, R¹ is selected from: -CN, -C(O)NH₂, F, -NHCH₃, -C(O)OH,

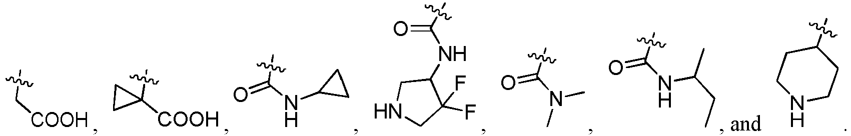

In some embodiments according to the first sub-aspect, the compound of formula (I) has a structure of formula (I-1): 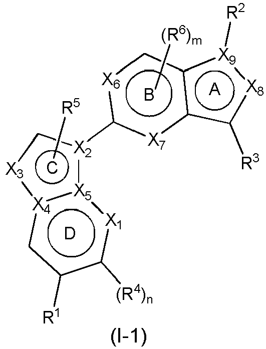 , wherein n is 0 or 1.

In a second sub-aspect, the present disclosure provides the compound of formula (I) described above, wherein R¹ is selected from hydrogen and deuterium, and n is 1 or 2.

In some embodiments, R¹ is hydrogen and n is 1.

In some embodiments according to the second sub-aspect, the compound of formula (I) has a structure of formula (I-2): 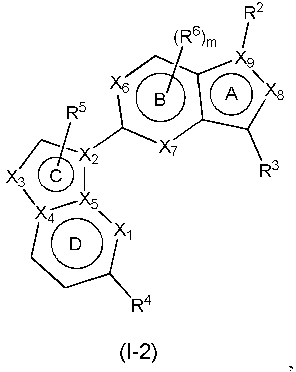 provided that R⁴ is not hydrogen or deuterium.

In some embodiments, the present disclosure provides the compound according to any of the embodiments described above, wherein R⁴ is selected from: hydrogen, R⁷, halogen, CN, NO₂, -OR^{1f}, -SR^{1f}, and -NR^{1d}R^{1e}, provided that when R¹ is hydrogen or deuterium, R⁴ is not hydrogen. In some such embodiments, R^{1d} and R^{1e} are preferably each independently selected from hydrogen and C₁₋₆ alkyl. Preferably, R^{1d} and R^{1e} are each independently selected from hydrogen and C₁₋₄ alkyl. In some such embodiments, R^{1f} is preferably selected from hydrogen and C₁₋₆ alkyl. Preferably, R^{1f} is selected from hydrogen and C₁₋₄ alkyl.

In some embodiments, R⁴ is selected from: hydrogen, R⁷, F, Cl, Br, CN, NO₂, OH, and NH₂, provided that when R¹ is hydrogen or deuterium, R⁴ is not hydrogen. In some embodiments, R⁴ is selected from: hydrogen, R⁷, and CN, provided that when R¹ is hydrogen or deuterium, R⁴ is not hydrogen.

In some such embodiments, R⁷ is preferably selected from: 4-6 membered saturated monocyclic heterocyclyl, 8-10 membered saturated fused bicyclic heterocyclyl, 6-11 membered saturated monospiroheterocyclyl, and 7-10 membered saturated bridged heterocyclyl, each of which has 1-3 heteroatoms independently selected from nitrogen, oxygen, and sulfur and is optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e}. In some such embodiments, R⁷ is selected from: 4-6 membered saturated monocyclic heterocyclyl and 7-10 membered saturated bridged heterocyclyl, wherein the monocyclic heterocyclyl and bridged heterocyclyl have 1 nitrogen heteroatom and optionally 1-2 additional heteroatoms independently selected from nitrogen, oxygen, and sulfur and are optionally substituted with one or more substituents independently selected from halogen, CN, OH, NO₂, C₁₋₄ alkyl, C₁₋₄ haloalkyl, C₁₋₄ alkoxy, and -NR^{8d}R^{8e}. In some such embodiments, R^{8d} and R^{8e} are preferably each independently selected from hydrogen and C₁₋₆ alkyl. Preferably, R^{8d} and R^{8e} are each independently selected from hydrogen and C₁₋₄ alkyl.

In some such embodiments, R⁷ is selected from: 4-6 membered saturated monocyclic heterocyclyl (e.g., azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, or thiomorpholinyl) and 7-10 membered saturated bridged heterocyclyl, wherein the monocyclic heterocyclyl and bridged heterocyclyl have 1 nitrogen heteroatom and optionally 1-2 additional heteroatoms independently selected from nitrogen, oxygen, and sulfur and are optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄)alkyl, and N(C₁₋₄ alkyl)₂.

In some such embodiments, R⁷ is selected from: 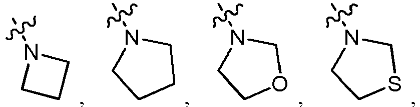 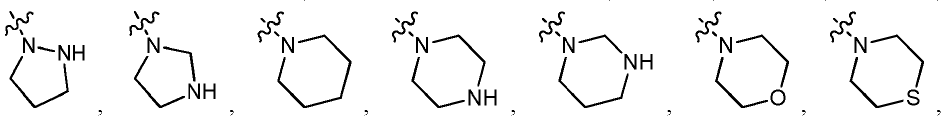 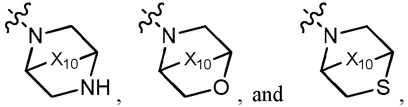 each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂, wherein X₁₀ is CH₂, (CH₂)₂, or (CH₂)₃.

In some such embodiments, R⁷ is selected from:

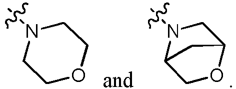

In some particular embodiments, R⁴ is selected from: hydrogen, CN 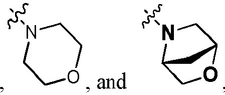 provided that when R¹ is hydrogen or deuterium, R⁴ is not hydrogen.

In some embodiments, the present disclosure provides the compound according to any of the embodiments described above, wherein:
R and R⁵ are each independently selected from hydrogen.

In some embodiments, the present disclosure provides the compound according to any of the embodiments described above, wherein:
R² is selected from: C₃₋₆ cycloalkyl, 4-6 membered saturated or partially unsaturated heterocyclyl having 1-3 heteroatoms independently selected from nitrogen, oxygen, and sulfur, C₆₋₁₀ aryl, and 5-6 membered heteroaryl having 1-3 heteroatoms independently selected from nitrogen, oxygen, and sulfur, wherein the cycloalkyl, heterocyclyl, aryl, and heteroaryl are optionally substituted with 1, 2, or more substituents independently selected from halogen, CN, OH, SH, NO₂, C₁₋₆ alkyl, C₁₋₆ haloalkyl, C₁₋₆ alkoxy, C₁₋₆ alkylthio, -NR^{2d}R^{2e}, -C(O)OR^{2f}, and -C(O)NR^{2d}R^{2e}, and in the case where two substituents are attached to the same ring carbon atom of the cycloalkyl or heterocyclyl, the two substituents, together with the ring carbon atom to which they are attached, optionally form optionally substituted C₃₋₆ cycloalkyl or optionally substituted 4-6 membered heterocycloalkyl having 1-3 heteroatoms independently selected from nitrogen, oxygen, and sulfur. In some such embodiments, R^{2d} and R^{2e} are preferably each independently selected from hydrogen, C₁₋₆ alkyl, and C₁₋₆ haloalkyl. In some such embodiments, R^{2f} is selected from hydrogen and C₁₋₆ alkyl.

In some embodiments, R² is selected from: C₃₋₆ cycloalkyl, 4-6 membered heterocycloalkyl having 1 nitrogen heteroatom and optionally 1-2 additional heteroatoms independently selected from nitrogen, oxygen, and sulfur, phenyl, and 5-6 membered heteroaryl having 1 nitrogen heteroatom and optionally 1-2 additional heteroatoms independently selected from nitrogen, oxygen, and sulfur, wherein the cycloalkyl, heterocycloalkyl, phenyl, and heteroaryl are optionally substituted with 1, 2, or more substituents independently selected from halogen, CN, OH, NH₂, NO₂, C₁₋₄ alkyl, -C(O)OH, -C(O)O-C₁₋₄ alkyl, - C(O)NH₂, -C(O)NH(C₁₋₄ alkyl), and -C(O)NH(C₁₋₄ alkyl)₂, and in the case where two substituents are attached to the same ring carbon atom of the cycloalkyl or heterocycloalkyl, the two substituents, together with the ring carbon atom to which they are attached, optionally form optionally substituted C₃₋₆ cycloalkyl.

In some embodiments, R² is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, thiomorpholinyl, phenyl, pyrazolyl, oxazolyl, isoxazolyl, thiazolyl, isothiazolyl, oxadiazolyl, thiadiazolyl, pyridinyl, pyrazinyl, pyridazinyl, and pyrimidinyl, each of which is optionally substituted with 1, 2, or more substituents independently selected from F, Cl, Br, CN, OH, NH₂, NO₂, methyl, ethyl, isopropyl, tert-butyl, C(O)OH, -C(O)OCH₃, -C(O)OCH₂CH₃, -C(O)NH₂, - C(O)NHCH₃, and -C(O)N(CH₃)₂, and in the case where two substituents are attached to the same ring carbon atom of the cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, morpholinyl, or thiomorpholinyl, the two substituents, together with the ring carbon atom to which they are attached, optionally form optionally substituted C₃₋₆ cycloalkyl.

In some embodiments, R² is selected from: cyclohexyl, pyrrolidinyl, piperidinyl, phenyl, and pyridinyl, each of which is optionally substituted with 1 or 2 substituents independently selected from F, Cl, Br, OH, NH₂, and methyl, and in the case where two substituents are attached to the same ring carbon atom of the pyrrolidinyl or piperidinyl, the two substituents, together with the ring carbon atom to which they are attached, optionally form optionally substituted C₃₋₆ cycloalkyl.

In some embodiments, R² is selected from:

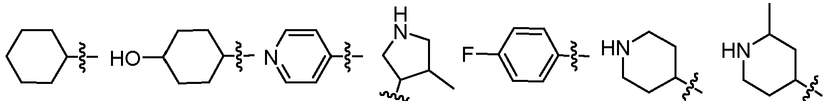

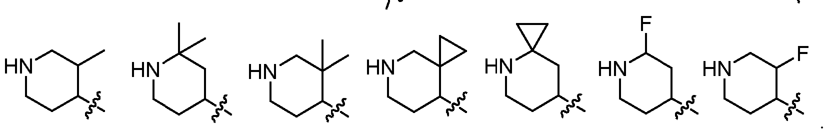

In some embodiments, R² is selected from:

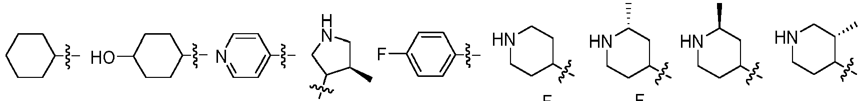

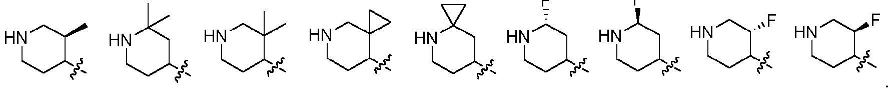

In some embodiments, the present disclosure provides the compound according to any of the embodiments described above, wherein R³ is selected from: halogen, CN, NO₂, C₁₋₆ alkyl, -OR^{3f}, -SR^{3f}, - NR^{3d}R^{3e}, -S(O)₂-NR^{3d}R^{3e}, -S(O)-NR^{3d}R^{3e}, -CF(R^{3f})₂, -CF₂(R^{3f}), -CF₃, -CCl(R^{3f})₂, -CCl₂(R^{3f}), -CCl₃,-C(O)OR^{3f}, and -C(O)NR^{3d}R^{3e}. In some embodiments, R³ is selected from: halogen, CN, C₁₋₄ alkyl, -OR^{3f}, - NR^{3d}R^{3e}, -CF(R^{3f})₂, -CF₂(R^{3f}), -CF₃, -CCl(R^{3f})₂, -CCl₂(R^{3f}), -CCl₃, -C(O)OR^{3f}, and -C(O)NR^{3d}R^{3e}.

In some embodiments, R³ is selected from: halogen, CN, C₁₋₄ alkyl, -OR^{3f}, -NR^{3d}R^{3e}, -CF(R^{3f})₂, - CF₂(R^{3f}), -CF₃, -CCl(R^{3f})₂, -CCl₂(R^{3f}), -CCl₃, and -C(O)NR^{3d}R^{3e}.

In some such embodiments, R^{3d} and R^{3e} are preferably each independently selected from hydrogen, deuterium, R¹¹, C₁₋₄ alkyl, and C₁₋₄ haloalkyl.

In some such embodiments, R¹¹ is preferably selected from: C₃₋₆ cycloalkyl, 4-6 membered saturated or partially unsaturated heterocyclyl having 1-3 heteroatoms independently selected from nitrogen, oxygen, and sulfur, C₆₋₁₀ aryl, and 5-6 membered heteroaryl having 1-3 heteroatoms independently selected from nitrogen, oxygen, and sulfur, wherein the cycloalkyl, heterocyclyl, aryl, and heteroaryl are optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e}.

In some such embodiments, R^{8d} and R^{8e} are preferably each independently selected from hydrogen, C₁₋₄ alkyl, and C₁₋₄ haloalkyl.

In some particular embodiments, R^{3d} and R^{3e} are each independently selected from hydrogen, methyl, and ethyl.

In some such embodiments, R^{3f} is selected from hydrogen and C₁₋₆ alkyl. Preferably, R^{3f} is selected from hydrogen and C₁₋₄ alkyl.

In some such embodiments, R^{3d} and R^{3e}, together with the nitrogen atom to which they are both attached, form R¹⁰.

In some such embodiments, R¹⁰ is preferably selected from: 3-6 membered heterocycloalkyl having 1 nitrogen heteroatom and optionally 1-2 additional heteroatoms independently selected from nitrogen, oxygen, and sulfur, and 5-6 membered heteroaryl having 1 nitrogen heteroatom and optionally 1-2 additional heteroatoms independently selected from nitrogen, oxygen, and sulfur, wherein the heterocycloalkyl and heteroaryl are optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some particular embodiments, R³ is selected from: halogen, OH, SH, NH₂, CN, C₁₋₄ alkyl, -OC₁₋₄ alkyl, -NH(C₁₋₄ alkyl), -N(C₁₋₄ alkyl)₂, -CHF₂, -CH₂F, -CF₃, -CH₂Cl, -CHCl₂, -CCl₃, -C(O)NH₂, - C(O)NH(C₁₋₄ alkyl), and -C(O)N(C₁₋₄ alkyl)₂.

In some particular embodiments, R³ is selected from: F, Cl, Br, OH, NH₂, CN, methyl, ethyl, propyl, isopropyl, n-butyl, isobutyl, sec-butyl, tert-butyl, methoxy, ethoxy, -N(CH₃)₂, -CHF₂, -CHCl₂, and -C(O)NH₂.

In some embodiments, the present disclosure provides the compound according to any of the embodiments described above, wherein R⁶ is selected from: halogen, CN, NO₂, C₁₋₄ alkyl, -OR^{3f}, -SR^{3f}, - NR^{3d}R^{3e}, -CF(R^{3f})₂, -CF₂(R^{3f}), -CF₃, -CCl(R^{3f})₂, -CCl₂(R^{3f}), and -CCl₃. In some such embodiments, R^{3f} is preferably selected from hydrogen and C₁₋₆ alkyl. Preferably, R^{3f} is selected from hydrogen and C₁₋₄ alkyl.

In some embodiments, R⁶ is selected from: F, Cl, Br, OH, NH₂, CN, NO₂, methyl, ethyl, propyl, isopropyl, n-butyl, isobutyl, sec-butyl, tert-butyl, methoxy, ethoxy, -CH₂F, -CHF₂, -CF₃, -CH₂Cl, -CHCl₂, and -CCl₃.

In some embodiments, the present disclosure provides the compound according to any of the embodiments described above, wherein m is 0.

In some embodiments, the present disclosure provides the compound according to any of the embodiments described above, wherein at least 2 of X₁, X₂, X₃, X₄, and X₅ are N, provided that X₄ and X₅ are not both N at the same time.

In some embodiments, 3 of X₁, X₂, X₃, X₄, and X₅ are N, provided that X₄ and X₅ are not both N at the same time.

In some embodiments, 4 of X₁, X₂, X₃, X₄, and X₅ are N, provided that X₄ and X₅ are not both N at the same time.

In some embodiments, X₁ is N.

In some embodiments, the present disclosure provides the compound according to any of the embodiments described above, wherein at least 2 of X₆, X₇, X₈, and X₉ are N.

In some embodiments, at least 3 of X₆, X₇, X₈, and X₉ are N.

In some embodiments, at least 4 of X₆, X₇, X₈, and X₉ are N.

In some embodiments, X₈ and X₉ are each N.

In some embodiments, the present disclosure provides the compound according to any of the embodiments described above, wherein 2 or 3 of X₁, X₂, X₃, X₄, and X₅ are N, and X₁ is N, provided that X₄ and X₅ are not both N at the same time; and
3 or 4 of X₆, X₇, X₈, and X₉ are N, and X₈ and X₉ are each N.

In a third sub-aspect, the present disclosure provides the compounds of formula (I), formula (I-1), and formula (I-2) described above, wherein X₁ and X₂ are each N, X₃ is CH, X₄ and X₅ are each C, X₆, X₈, and X₉ are each N, and X₇ is CH.

In some embodiments, the compound of the present disclosure has a structure of formula (I-i) or formula (I-ii): 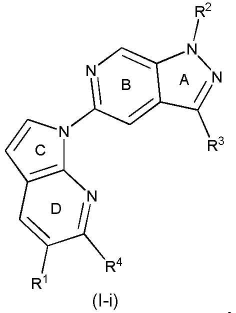 wherein R¹, R², R³, and R⁴ are each as defined in any of the embodiments described above for the compounds of formula (I), formula (I-1), and formula (I-2), provided that R¹ is not hydrogen or deuterium; 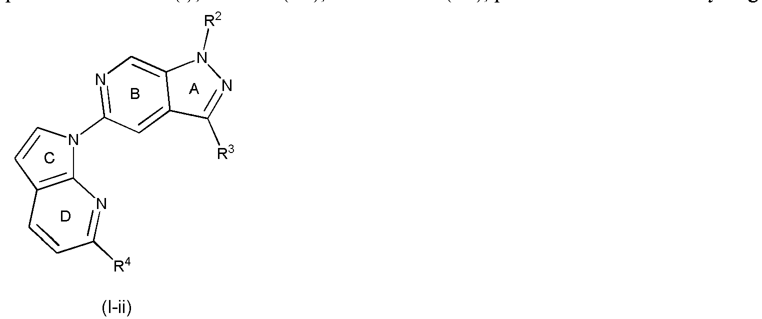 wherein R², R³, and R⁴ are each as defined in any of the embodiments described above for the compounds of formula (I), formula (I-1), and formula (I-2), provided that R⁴ is not hydrogen or deuterium.

In some embodiments, R¹ is selected from: halogen, CN, NO₂, -OR^{1f}, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, -C(O)OR^{1f}, or -C(O)NR^{1d}R^{1e}.

In some embodiments, R¹ is selected from: halogen, CN, -(CR^{1a}R^{1b})ₚ-OR^{1f}, -(CR^{1a}R^{1b})ₚ-C(O)OR^{1f}, - C(O)OR^{1f}, or -C(O)NR^{1d}R^{1e}.

In some such embodiments, p is preferably 1.

In some such embodiments, R^{1a} and R^{1b} are preferably each independently selected from hydrogen and C₁₋₄ alkyl, preferably each independently hydrogen.

In some such embodiments, R^{1a} and R^{1b}, together with the carbon atom to which they are both attached, form R⁹.

In some such embodiments, R⁹ is preferably selected from: C₃₋₆ cycloalkyl, wherein the cycloalkyl is optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl) and N(C₁₋₄ alkyl)₂.

In some such embodiments, R⁹ is selected from: cyclopropyl, cyclobutyl, cyclopentyl, and cyclohexyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R⁹ is cyclopropyl.

In some such embodiments, R^{1d} and R^{1e} are preferably each independently selected from hydrogen and R¹¹; or R^{1d} and R^{1e}, together with the nitrogen atom to which they are both attached, form R¹⁰.

In some such embodiments, R¹¹ is preferably selected from: C₃₋₆ cycloalkyl, and 3-6 membered heterocycloalkyl having 1-3 heteroatoms independently selected from nitrogen, oxygen, and sulfur, wherein the cycloalkyl and heterocycloalkyl are optionally substituted with one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂.

In some such embodiments, R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, and NH₂.

In some such embodiments, R¹¹ is selected from: cyclopropyl, cyclobutyl, cyclopentyl, cyclohexyl, azetidinyl, pyrrolidinyl, piperidinyl, piperazinyl, and morpholinyl, each of which is optionally substituted with one or more substituents independently selected from F, Cl, Br, CN, OH, NO₂, and NH₂.

In some such embodiments, R¹¹ is selected from cyclopropyl and

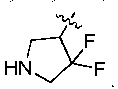

In some such embodiments, R¹⁰ is preferably selected from: 3-6 membered heterocycloalkyl having 1 nitrogen heteroatom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some such embodiments, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R¹⁰ is selected from: 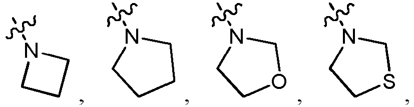 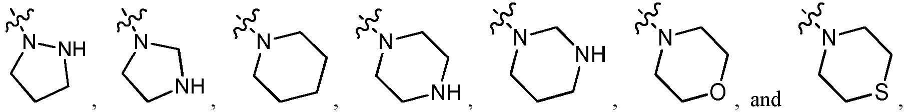 and preferably 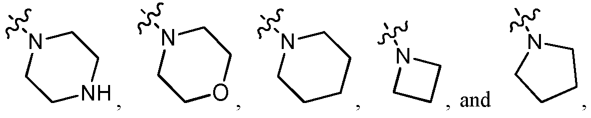 each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R^{1f} is selected from hydrogen and C₁₋₄ alkyl, preferably hydrogen.

In some particular embodiments, R¹ is selected from: F, Cl, Br, CN, OH, NH₂, C(O)OH, -C(O)NH₂,

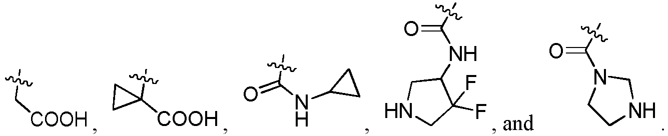

In some particular embodiments, R¹ is selected from CN and -C(O)NH₂.

In some embodiments, R² is selected from: C₃₋₆ cycloalkyl, 4-6 membered heterocycloalkyl having 1 nitrogen heteroatom and optionally 1-2 additional heteroatoms independently selected from nitrogen, oxygen, and sulfur, phenyl, and 5-6 membered heteroaryl having 1 nitrogen heteroatom and optionally 1-2 additional heteroatoms independently selected from nitrogen, oxygen, and sulfur, wherein the cycloalkyl, heterocycloalkyl, phenyl, and heteroaryl are optionally substituted with 1, 2, or more substituents independently selected from halogen, CN, OH, NH₂, NO₂, C₁₋₆ alkyl, and C₁₋₆ alkoxy, and in the case where two substituents are attached to the same ring carbon atom of the cycloalkyl or heterocycloalkyl, the two substituents, together with the ring carbon atom to which they are attached, optionally form optionally substituted C₃₋₆ cycloalkyl.

In some embodiments, R² is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, thiomorpholinyl, phenyl, pyrazolyl, oxazolyl, isoxazolyl, thiazolyl, isothiazolyl, oxadiazolyl, thiadiazolyl, pyridinyl, pyrazinyl, pyridazinyl, and pyrimidinyl, each of which is optionally substituted with 1 or 2 substituents independently selected from F, Cl, Br, CN, OH, NH₂, NO₂, and C₁-C₄ alkyl, and in the case where two substituents are attached to the same ring carbon atom of the cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, or thiomorpholinyl, the two substituents, together with the ring carbon atom to which they are attached, optionally form optionally substituted C₃₋₆ cycloalkyl.

In some embodiments, R² is selected from: cyclopentyl, cyclohexyl, pyrrolidinyl, piperidinyl, phenyl, and pyridinyl, each of which is optionally substituted with 1 or 2 substituents independently selected from F, Cl, Br, CN, OH, NH₂, NO₂, methyl, ethyl, and isopropyl, and in the case where two substituents are attached to the same ring carbon atom of the pyrrolidinyl or piperidinyl, the two substituents, together with the ring carbon atom to which they are attached, optionally form optionally substituted C₃₋₆ cycloalkyl.

In some embodiments, R² is selected from: 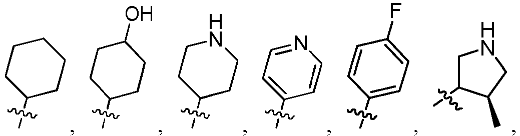 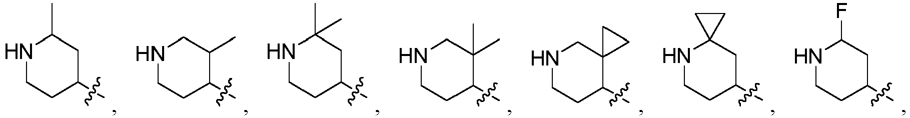 and

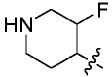

In some embodiments, R² is selected from: 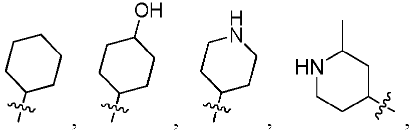 and

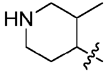

In some embodiments, R² is

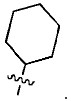

In some embodiments, R³ is selected from: halogen, CN, NO₂, C₁₋₆ alkyl, OH, NH₂, -CH₂F, -CHF₂, - CF₃, -CH₂Cl, -CHCl₂, and -CCl₃.

In some embodiments, R³ is -CHF₂.

In some embodiments, R⁴ is selected from: hydrogen, R⁷, halogen, CN, NO₂, OH, and NH₂.

In some embodiments, R⁴ is selected from: hydrogen, R⁷, F, Cl, Br, CN, NO₂, OH, and NH₂.

In some such embodiments, R⁷ is preferably selected from: 4-6 membered saturated monocyclic heterocyclyl, wherein the heterocyclyl has 1 nitrogen heteroatom and optionally 1-2 additional heteroatoms independently selected from nitrogen, oxygen, and sulfur and is optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, NH₂, NH(C₁₋₄ alkyl), and N(C₁₋₄ alkyl)₂.

In some such embodiments, R⁷ is selected from:

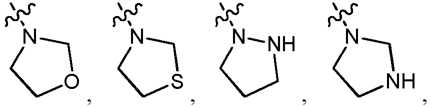

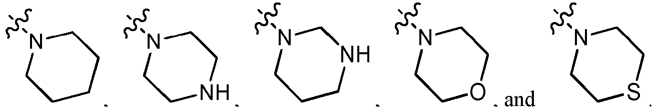

In some particular embodiments, R⁴ is selected from: hydrogen, CN, and 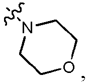 provided that when R¹ is hydrogen, R⁴ is not hydrogen.

In a fourth sub-aspect, the present disclosure provides the compounds of formula (I), formula (I-1), and formula (I-2) described above, wherein X₁ and X₅ are each N, X₂ and X₄ are C, X₃ is CH, X₆, X₈, and X₉ are each N, and X₇ is CH.

In some embodiments, the compound of the present disclosure has a structure of formula (I-iii): 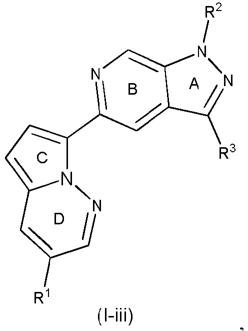 wherein R¹, R², and R³ are each as defined in any of the embodiments described above for the compounds of formula (I), formula (I-1), and formula (I-2), provided that R¹ is not hydrogen or deuterium.

In some embodiments, R¹ is selected from: R⁷, halogen, NO₂, -OR^{1f}, -NR^{1d}R^{1e}, -(CR^{1a}R^{1b})ₚ-OR^{1f}, - (CR^{1a}R^{1b})ₚ-C(O)OR^{1f}, -(CR^{1a}R^{1b})ₚ-NR^{1d}R^{1e}, -C(O)OR^{1f}, or -C(O)NR^{1d}R^{1e}.

In some embodiments, R¹ is selected from: R⁷, halogen, -NR^{1d}R^{1e}, -(CR^{1a}R^{1b})ₚ-OR^{1f}, -(CR^{1a}R^{1b})ₚ-C(O)OR^{1f}, -C(O)OR^{1f}, or -C(O)NR^{1d}R^{1e}.

In some such embodiments, p is 1.

In some such embodiments, R⁷ is selected from: 4-6 membered heterocycloalkyl having 1 N atom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some such embodiments, R⁷ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R⁷ is piperidinyl.

In some such embodiments, R^{1a} and R^{1b} are each independently selected from hydrogen and C₁₋₄ alkyl, preferably each independently hydrogen.

In some such embodiments, R^{1a} and R^{1b}, together with the carbon atom to which they are both attached, form R⁹.

In some such embodiments, R⁹ is selected from: C₃₋₆ cycloalkyl, wherein the cycloalkyl is optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl), and N(C₁₋₄ alkyl)₂.

In some such embodiments, R⁹ is selected from: cyclopropyl, cyclobutyl, cyclopentyl, and cyclohexyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R^{1d} and R^{1e} are each independently selected from hydrogen, C₁₋₄ alkyl and R¹¹; or R^{1d} and R^{1e}, together with the nitrogen atom to which they are both attached, form R¹⁰.

In some such embodiments, R¹¹ is selected from: C₃₋₆ cycloalkyl, and 3-6 membered heterocycloalkyl having 1-3 heteroatoms independently selected from nitrogen, oxygen, and sulfur, wherein the cycloalkyl and heterocycloalkyl are optionally substituted with one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂.

In some such embodiments, R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, and NH₂.

In some such embodiments, R¹⁰ is selected from: 3-6 membered heterocycloalkyl having 1 nitrogen heteroatom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some such embodiments, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R^{1f} is selected from hydrogen and C₁₋₄ alkyl, preferably hydrogen.

In some particular embodiments, R¹ is selected from: F, Cl, Br, OH, NH₂, -NHCH₃, C(O)OH, -C(O)NH₂, -CH₂OH,

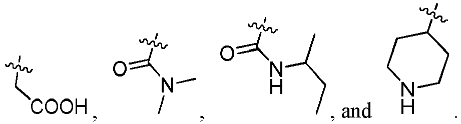

In some particular embodiments, R¹ is selected from-CN and -C(O)NH₂.

In some embodiments, R² is selected from: C₃₋₆ cycloalkyl, 4-6 membered heterocycloalkyl having 1 nitrogen heteroatom and optionally 1-2 additional heteroatoms independently selected from nitrogen, oxygen, and sulfur, phenyl, and 5-6 membered heteroaryl having 1 nitrogen heteroatom and optionally 1-2 additional heteroatoms independently selected from nitrogen, oxygen, and sulfur, wherein the cycloalkyl, heterocycloalkyl, phenyl, and heteroaryl are optionally substituted with 1, 2, or more substituents independently selected from halogen, CN, OH, NH₂, NO₂, C₁₋₆ alkyl, and C₁₋₆ alkoxy, and in the case where two substituents are attached to the same ring carbon atom of the cycloalkyl or heterocycloalkyl, the two substituents, together with the ring carbon atom to which they are attached, optionally form optionally substituted C₃₋₆ cycloalkyl.

In some embodiments, R² is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, thiomorpholinyl, phenyl, pyrazolyl, oxazolyl, isoxazolyl, thiazolyl, isothiazolyl, oxadiazolyl, thiadiazolyl, pyridinyl, pyrazinyl, pyridazinyl, and pyrimidinyl, each of which is optionally substituted with 1 or 2 substituents independently selected from F, Cl, Br, CN, OH, NH₂, NO₂, and C₁-C₄ alkyl, and in the case where two substituents are attached to the same ring carbon atom of the cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, or thiomorpholinyl, the two substituents, together with the ring carbon atom to which they are attached, optionally form optionally substituted C₃₋₆ cycloalkyl.

In some embodiments, R² is selected from: cyclopentyl, cyclohexyl, pyrrolidinyl, piperidinyl, phenyl, and pyridinyl, each of which is optionally substituted with 1 or 2 substituents independently selected from F, Cl, Br, CN, OH, NH₂, NO₂, methyl, ethyl, and isopropyl, and in the case where two substituents are attached to the same ring carbon atom of the pyrrolidinyl or piperidinyl, the two substituents, together with the ring carbon atom to which they are attached, optionally form optionally substituted C₃₋₆ cycloalkyl.

In some embodiments, R² is selected from:

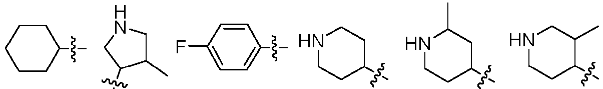

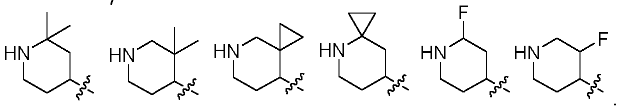

In some embodiments, R² is selected from:

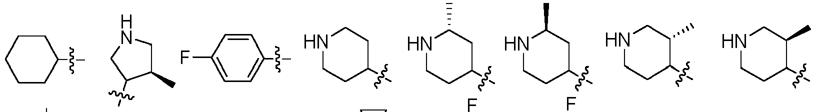

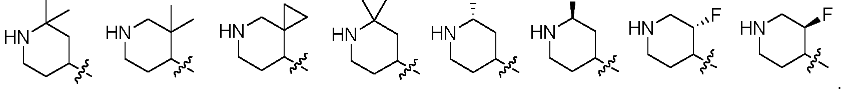

In some embodiments, R³ is selected from: halogen, CN, C₁₋₄ alkyl, -OR^{3f}, -SR^{3f}, -NR^{3d}R^{3e}, -CF(R^{3f})₂, -CF₂(R^{3f}), -CF₃, -CCl(R^{3f})₂, -CClz(R^{3f}), -CCl₃, and -C(O)NR^{3d}R^{3e}. In some such embodiments, R^{3d} and R^{3e} are each independently selected from hydrogen and C₁₋₄ alkyl, preferably hydrogen and methyl. In some such embodiments, R^{3f} is selected from hydrogen and C₁₋₄ alkyl, preferably hydrogen and methyl.

In some particular embodiments, R³ is selected from: halogen, OH, SH, NH₂, CN, C₁₋₄ alkyl, -OC₁₋₄ alkyl, -NH(C₁₋₄ alkyl), -N(C₁₋₄ alkyl)₂, -CHF₂, -CH₂F, -CF₃, -CH₂Cl, -CHCl₂, -CCl₃, -C(O)NH₂, - C(O)NH(C₁₋₄ alkyl), and -C(O)N(C₁₋₄ alkyl)₂.

In some embodiments, R³ is selected from: F, OH, NH₂, CN, methyl, ethyl, isopropyl, tert-butyl, methoxy, -N(CH₃)₂, -CHF₂, and -C(O)NH₂.

In some embodiments, R³ is selected from: F, CN, methyl, isopropyl, methoxy, -N(CH₃)₂, -CHF₂, and - C(O)NH₂.

In a fifth sub-aspect, the present disclosure provides the compounds of formula (I), formula (I-1), and formula (I-2) described above, wherein X₁, X₃, and X₄ are each N, X₂ and X₅ are C, X₆, X₈, and X₉ are each N, and X₇ is CH.

In some embodiments, the compound of the present disclosure has a structure of formula (I-iv): 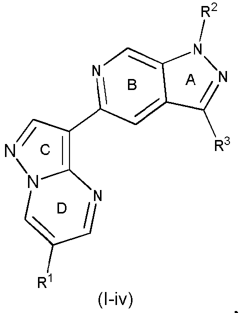 wherein R¹, R², and R³ are each as defined in any of the embodiments described above for the compounds of formula (I), formula (I-1), and formula (I-2), provided that R¹ is not hydrogen or deuterium.

In some embodiments, R¹ is selected from: halogen, CN, NO₂, -OR^{1f}, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, or -C(O)OR^{1f}.

In some embodiments, R¹ is selected from: halogen, CN, -(CR^{1a}R^{1b})ₚ-OR^{1f}, -(CR^{1a}R^{1b})ₚ-C(O)OR^{1f}, or - C(O)OR^{1f}.

In some such embodiments, p is 1.

In some such embodiments, R^{1a} and R^{1b} are each independently selected from hydrogen and C₁₋₄ alkyl, preferably each independently hydrogen; or R^{1a} and R^{1b}, together with the carbon atom to which they are both attached, form R⁹.

In some such embodiments, R⁹ is selected from: C₃₋₆ cycloalkyl, wherein the cycloalkyl is optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl), and N(C₁₋₄ alkyl)₂.

In some such embodiments, R⁹ is selected from: cyclopropyl, cyclobutyl, cyclopentyl, and cyclohexyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R^{1d} and R^{1e} are each independently selected from hydrogen and R¹¹; or R^{1a} and R^{1e}, together with the nitrogen atom to which they are both attached, form R¹⁰.

In some such embodiments, R¹¹ is selected from: C₃₋₆ cycloalkyl, and 3-6 membered heterocycloalkyl having 1-3 heteroatoms independently selected from nitrogen, oxygen, and sulfur, wherein the cycloalkyl and heterocycloalkyl are optionally substituted with one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂.

In some such embodiments, R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, and NH₂.

In some such embodiments, R¹⁰ is selected from: 3-6 membered heterocycloalkyl having 1 nitrogen heteroatom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂.

In some such embodiments, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

In some such embodiments, R^{1f} is selected from hydrogen and C₁₋₄ alkyl, preferably hydrogen.

In some particular embodiments, R¹ is selected from: F, Cl, Br, CN, OH, NH₂, C(O)OH, 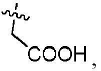 and

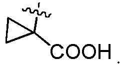

In some particular embodiments, R¹ is CN.

In some embodiments, R² is selected from: C₃₋₆ cycloalkyl, 4-6 membered heterocycloalkyl having 1 nitrogen heteroatom and optionally 1-2 additional heteroatoms independently selected from nitrogen, oxygen, and sulfur, phenyl, and 5-6 membered heteroaryl having 1 nitrogen heteroatom and optionally 1-2 additional heteroatoms independently selected from nitrogen, oxygen, and sulfur, wherein the cycloalkyl, heterocycloalkyl, phenyl, and heteroaryl are optionally substituted with one or more substituents independently selected from halogen, CN, OH, NH₂, NO₂, C₁₋₄ alkyl.

In some embodiments, R² is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, thiomorpholinyl, phenyl, pyrazolyl, oxazolyl, isoxazolyl, thiazolyl, isothiazolyl, oxadiazolyl, thiadiazolyl, pyridinyl, pyrazinyl, pyridazinyl, and pyrimidinyl, each of which is optionally substituted with 1 or 2 substituents independently selected from F, Cl, Br, CN, OH, NH₂, NO₂, and C₁-C₄ alkyl, and in the case where two substituents are attached to the same ring carbon atom of the cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, or thiomorpholinyl, the two substituents, together with the ring carbon atom to which they are attached, optionally form optionally substituted C₃₋₆ cycloalkyl.

In some embodiments, R² is selected from:

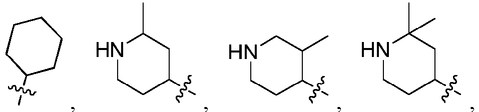

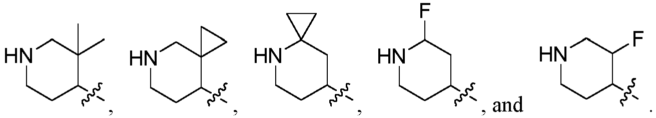

In some embodiments R² is selected from: 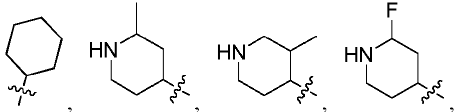 and

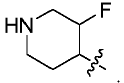

In some embodiments, R² is

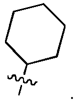

In some embodiments, R³ is selected from: halogen, CN, NO₂, C₁₋₆ alkyl, OH, NH₂, -CH₂F, -CHF₂, - CF₃, -CH₂Cl, -CHCl₂, and -CCl₃.

In some embodiments, R³ is -CHF₂.

In a sixth sub-aspect, the present disclosure provides the compounds of formula (I), formula (I-1), and formula (I-2) described above, wherein X₁ and X₅ are each N, X₂ and X₄ are C, X₃ is CH, X₆ is CH, and X₇, X₈, and X₉ are each N.

In some embodiments, the compound of the present disclosure has a structure of formula (I-v): 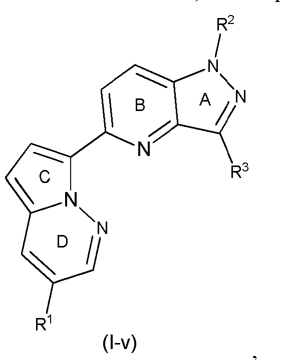 wherein R¹, R², and R³ are each as defined in any of the embodiments described above for the compounds of formula (I), formula (I-1), and formula (I-2), provided that R¹ is not hydrogen or deuterium.

In some embodiments, R¹, R², and R³ are each as defined in any of the embodiments according to the third sub-aspect, the fourth sub-aspect, and/or the fifth sub-aspect.

In some embodiments, R¹ is -C(O)NH₂; and/or, in some embodiments, R² is 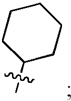 and/or, in some embodiments, R³ is -CHF₂.

In a seventh sub-aspect, the present disclosure provides the compounds of formula (I), formula (I-1), and formula (I-2) described above, wherein X₁ and X₅ are each N, X₂ and X₄ are C, X₃ is CH, and X₆, X₇, X₈, and X₉ are each N.

In some embodiments, the compound of the present disclosure has a structure of formula (I-vi): 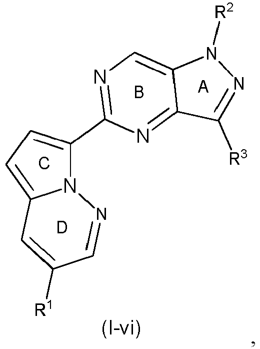 wherein R¹, R², and R³ are each as defined in any of the embodiments described above for the compounds of formula (I), formula (I-1), and formula (I-2), provided that R¹ is not hydrogen or deuterium.

In some embodiments, R¹, R², and R³ are each as defined in any of the embodiments according to the third sub-aspect, the fourth sub-aspect, and/or the fifth sub-aspect.

In some embodiments, R¹ is -C(O)NH₂; and/or, in some embodiments, R² is 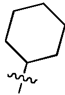 ; and/or, in some embodiments, R³ is -CHF₂.

In an eighth sub-aspect, the present disclosure provides the compounds of formula (I), formula (I-1), and formula (I-2) described above, wherein X₁ and X₂ are each N, X₃ is CH, X₄ and X₅ are each C, and X₆, X₇, X₈, and X₉ are each N.

In some embodiments, the compound of the present disclosure has a structure of formula (I-vii): 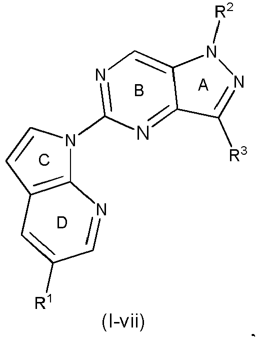 wherein R¹, R², and R³ are each as defined in any of the embodiments described above for the compounds of formula (I), formula (I-1), and formula (I-2), provided that R¹ is not hydrogen or deuterium.

In some embodiments, R¹, R², and R³ are each as defined in any of the embodiments according to the third sub-aspect, the fourth sub-aspect, and/or the fifth sub-aspect.

In some embodiments, R¹ is -CN; and/or, in some embodiments, R² is 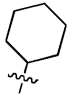 ; and/or, in some embodiments, R³ is -CHF₂.

In a ninth sub-aspect, the present disclosure provides the compounds of formula (I), formula (I-1), and formula (I-2) described above, wherein X₁, X₃, and X₄ are each N, X₂ and X₅ are C, and X₆, X₇, X₈, and X₉ are each N.

In some embodiments, the compound of the present disclosure has a structure of formula (I-viii): 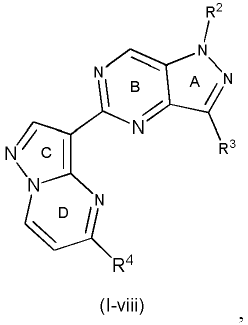 wherein R², R³, and R⁴ are each as defined in any of the embodiments described above for the compounds of formula (I), formula (I-1), and formula (I-2), provided that R⁴ is not hydrogen or deuterium.

In some embodiments, R² and R³ are each as defined in any of the embodiments according to the third sub-aspect, the fourth sub-aspect, and/or the fifth sub-aspect.

In some embodiments, R² is 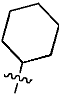 ; and/or, in some embodiments, R³ is -CHF₂.

In some embodiments, R⁴ is selected from: R⁷, halogen, CN, NO₂, OH, and NH₂. In some embodiments, R⁴ is selected from: R⁷, F, Cl, Br, CN, NO₂, OH, and NH₂. In some such embodiments, R⁷ is selected from: 7-10 membered saturated bridged heterocyclyl, wherein the bridged heterocyclyl has 1 nitrogen heteroatom and optionally 1-2 additional heteroatoms independently selected from nitrogen, oxygen, and sulfur and is optionally substituted with one or more substituents independently selected from halogen, CN, OH, NO₂, C₁₋₄ alkyl, C₁₄ haloalkyl, C₁₋₄ alkoxy, NH₂, NH(C₁₋₄ alkyl), and N(C₁₋₄ alkyl)₂. In some such embodiments, R⁷ is selected from: 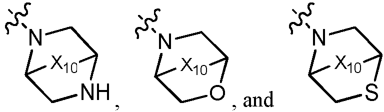 , wherein X₁₀ is CH₂, (CH₂)₂, or (CH₂)₃.

In some particular embodiments, R⁴ is selected from: CN and 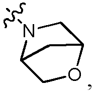 more preferably

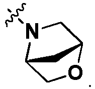

The present disclosure encompasses compounds resulting from any combination of the various embodiments.

In some embodiments, the present disclosure provides the compound of formula (I) or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof, wherein the compound is selected from:

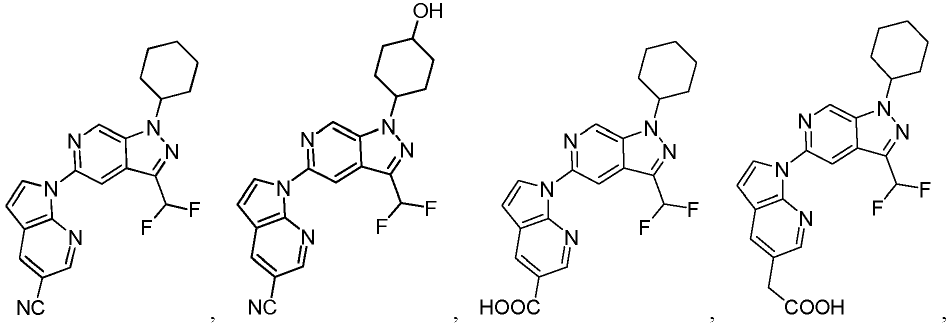

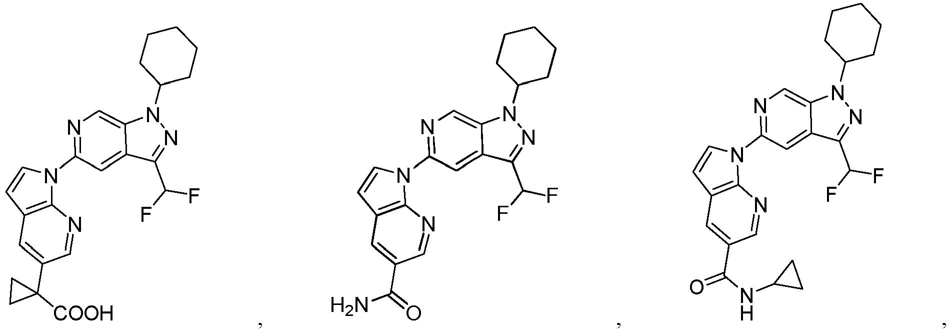

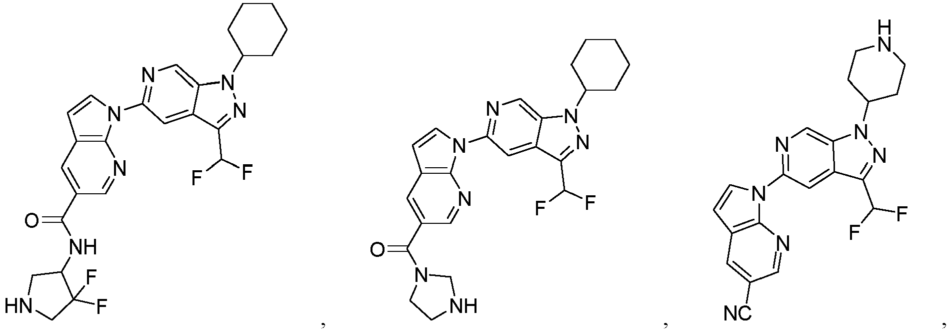

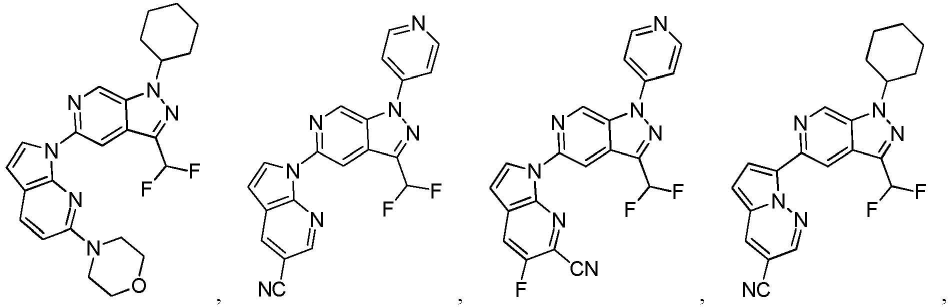

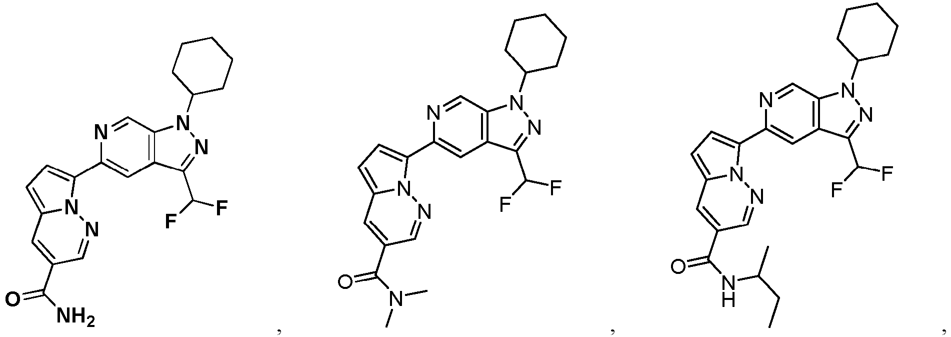

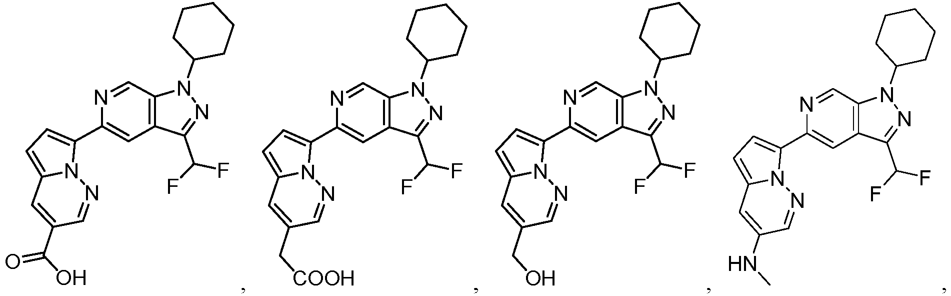

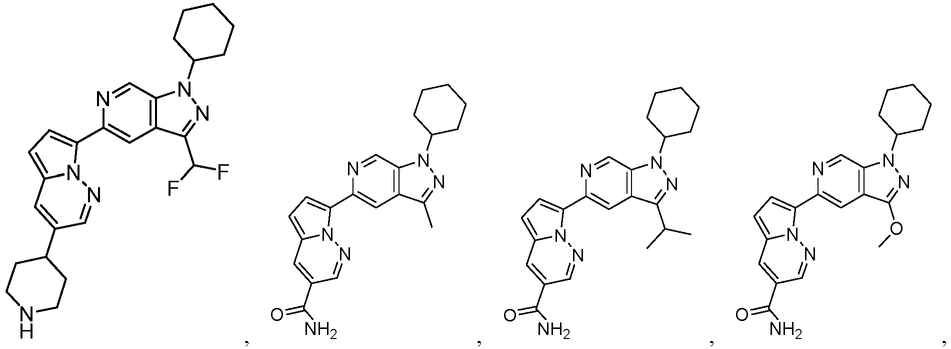

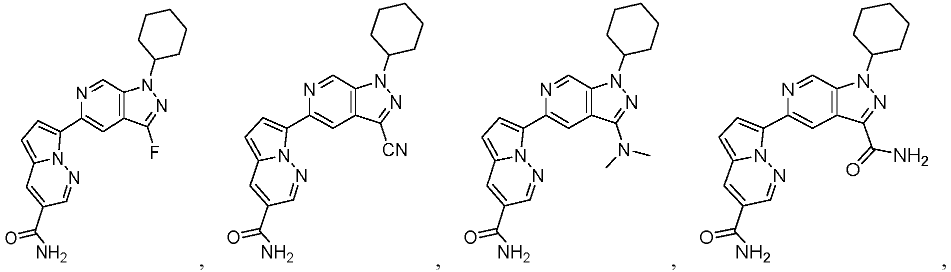

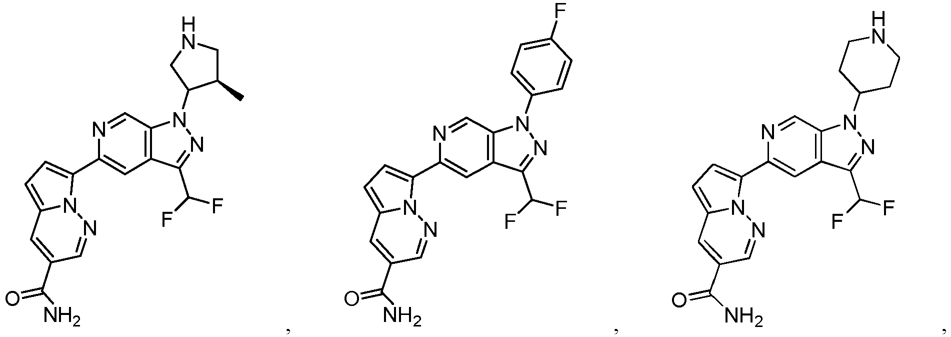

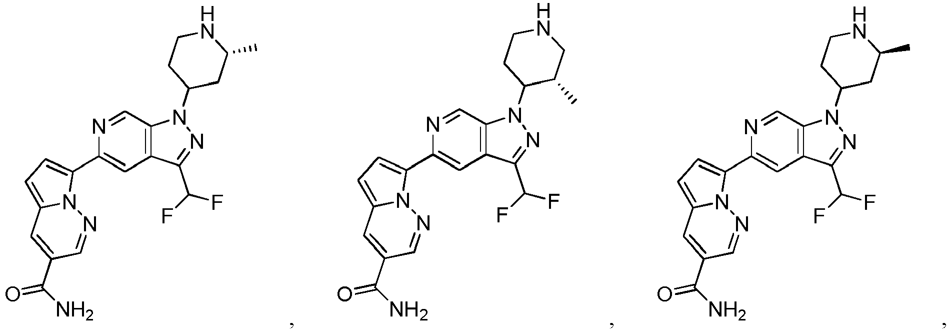

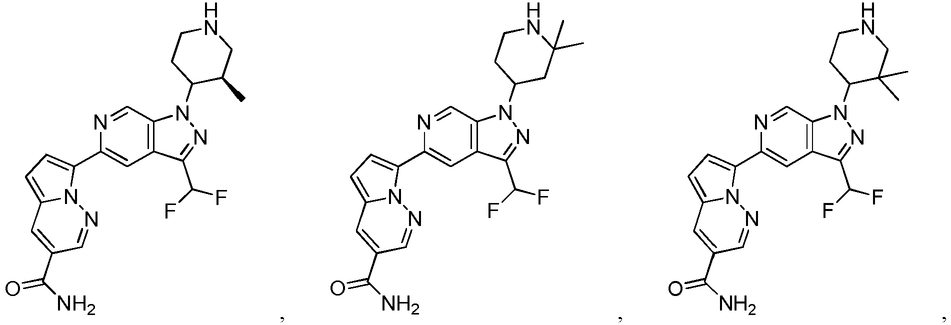

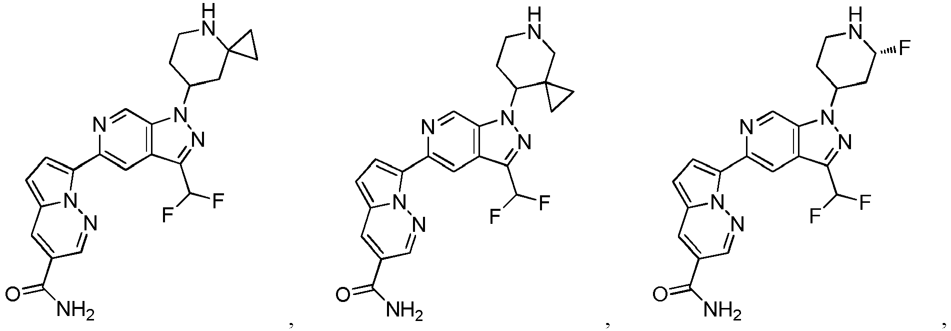

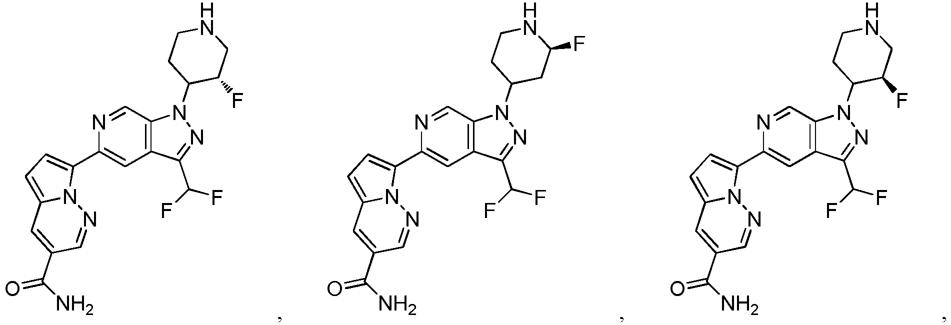

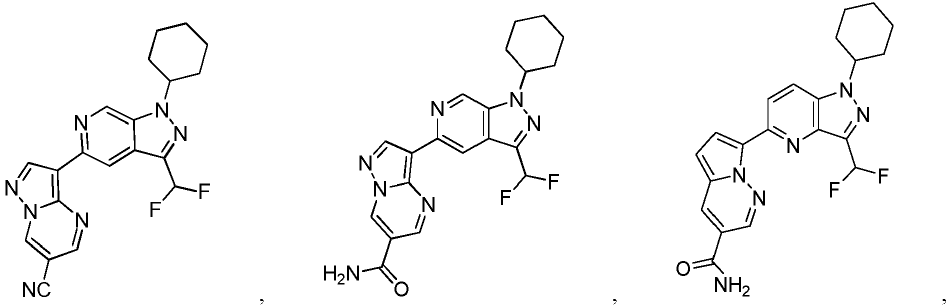

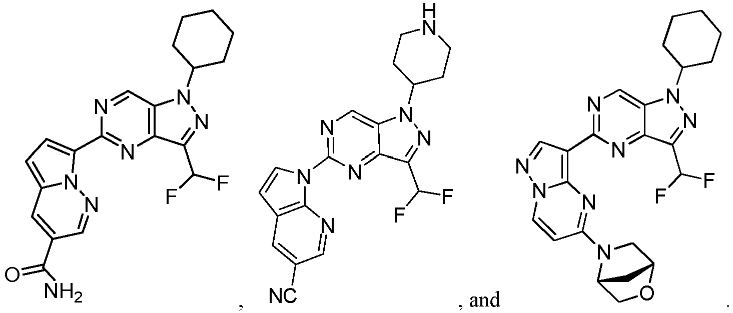

### PROTAC Molecule for Inhibiting and Degrading IRAK4

In another aspect, the present disclosure provides use of the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure described above in the preparation of a proteolysis targeting chimera (PROTAC).

In another aspect, the present disclosure provides a proteolysis targeting chimera (PROTAC) comprising a moiety with IRAK4 protein kinase inhibitory activity, wherein the moiety is derived from the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure described above.

### Pharmaceutical Composition and Treatment Method

In another aspect, the present disclosure provides a pharmaceutical composition comprising a therapeutically effective amount of the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure or the PROTAC molecule of the present disclosure, and one or more pharmaceutically acceptable carriers. The pharmaceutical composition is preferably a solid formulation, a liquid formulation, or a transdermal formulation.

In another aspect, the present disclosure provides use of the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure, or the PROTAC molecule of the present disclosure, or the pharmaceutical composition of the present disclosure in the preparation of a medicament.

In some embodiments, the compound of the present discourse, the PROTAC molecule of the present disclosure, the pharmaceutical composition of the present disclosure, or the medicament is used for treating a disease, a disorder, or a condition associated with IRAK4 protein kinase.

In another aspect, the present disclosure also provides a method for treating, alleviating a symptom of, or delaying the progression or onset of a disease, a disorder, or a condition associated with IRAK4 protein kinase, which comprises administering to an individual in need thereof an effective amount of the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure, the PROTAC molecule of the present disclosure, or the pharmaceutical composition of the present disclosure.

In yet another aspect, the present disclosure provides the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure, the PROTAC molecule of the present disclosure, or the pharmaceutical composition of the present disclosure for use in treating a disease, a disorder, or a condition associated with IRAK4 protein kinase.

In another aspect, the present disclosure further provides use of the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure, the PROTAC molecule of the present disclosure, or the pharmaceutical composition of the present disclosure in the preparation of a medicament as an IRAK4 inhibitor.

In yet another aspect, the present disclosure provides a method for inhibiting IRAK4 activity in an individual, which comprises administering to an individual in need thereof an effective amount of the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof of the present disclosure, the PROTAC molecule of the present disclosure, or the pharmaceutical composition of the present disclosure.

In some embodiments, the disease, the disorder, or the condition associated with IRAK4 protein kinase is selected from: an autoimmune condition, an inflammatory condition, cancer, graft rejection, thromboembolism, atherosclerosis, myocardial infarction, and metabolic syndrome.

In some embodiments, the inflammatory condition is selected from: osteoarthritis, gout, gouty arthritis, chronic obstructive pulmonary disease, periodic fever, atopic dermatitis, allergic eczema, lymphadenectasis, pyaemia, irritable bowel syndrome (IBD), ulcerative colitis, asthma, and allergy.

In some embodiments, the autoimmune condition is selected from: Crohn's disease, rheumatoid arthritis, systemic lupus erythematosus, lupus nephritis, cutaneous lupus, psoriasis, psoriatic arthritis, multiple sclerosis, neuropathic pain, ankylosing spondylitis, reactive arthritis, and systemic juvenile idiopathic arthritis.

In some embodiments, the graft rejection is selected from: graft-versus-host disease and allograft rejection.

In some embodiments, the cancer is selected from: brain cancer, kidney cancer, liver cancer, stomach cancer, vaginal cancer, ovarian cancer, gastric tumor, breast cancer, bladder cancer, colon cancer, prostate cancer, pancreatic cancer, lung cancer, cervical cancer, testicular cancer, skin cancer, bone cancer, thyroid cancer, sarcoma, glioblastoma, neuroblastoma, gastrointestinal cancer, neck and head tumor, adenoma, adenocarcinoma, keratoacanthoma, epidermoid carcinoma, large cell carcinoma, non-small cell lung cancer, Hodgkin's lymphoma and non-Hodgkin's lymphoma, follicular carcinoma, papillary carcinoma, seminoma, melanoma, acute myelogenous leukemia, chronic myelogenous leukemia, diffuse large B-cell lymphoma, activated B-cell-like diffuse large B-cell lymphoma, chronic lymphocytic leukemia, chronic lymphocytic lymphoma, primary effusion lymphoma, Burkitt lymphoma/leukemia, acute lymphocytic leukemia, B-cell prolymphocytic leukemia, lymphoplasmacytic lymphoma, Waldenstrom macroglobulinemia, splenic marginal zone lymphoma, intravascular large B-cell lymphoma, plasmacytoma, and multiple myeloma.

The term "pharmaceutically acceptable carrier" herein refers to a diluent, an adjuvant, an excipient, or a vehicle administered together with a therapeutic agent, which is suitable, within the scope of sound medical judgment, for contact with the tissues of humans and/or other animals without undue toxicity, irritation, allergic response, or other problems or complications that are not commensurate with a reasonable benefit/risk ratio.

Unless otherwise stated, the term "treat", "treating", or "treatment" as used herein means reversing, alleviating, or inhibiting the progression of a disorder or condition to which such term applies or one or more symptoms of such a disorder or condition, or preventing such a disorder or condition or one or more symptoms of such a disorder or condition.

As used herein, the term "individual" includes a human or non-human animal. Exemplary human individuals include human individuals with a disease (e.g., a disease described herein), which are referred to as patients, or normal individuals. In the present disclosure, "non-human animals" include all vertebrates, such as non-mammals (e.g., birds, amphibians, or reptiles) and mammals, such as non-human primates, livestock, and/or domesticated animals (e.g., sheep, dogs, cats, cows, pigs, etc.).

In another embodiment, the pharmaceutical composition of the present disclosure may further comprise one or more additional therapeutic agents or prophylactic agents.

### Examples

Embodiments of the present disclosure will be described in detail below with reference to examples, but those skilled in the art will appreciate that the following examples are only illustrative of the present disclosure and should not be construed as limiting the scope of the present disclosure. Experimental procedures without specified conditions in the examples are conducted according to conventional conditions or conditions recommended by the manufacturers. Reagents or instruments without specified manufacturers used herein are conventional products that are commercially available.

NMR was measured using a Bruker Avance III 400 nuclear magnetic resonance apparatus and chemical shifts (δ) were expressed in 10⁻⁶ (ppm). The solvents were deuterated methanol (CD₃OD), deuterated chloroform (CDCl₃), hexadeuterodimethyl sulfoxide (DMSO-d6), or the like, and the internal standard was tetramethylsilane (TMS).

MS was determined using an Agilent (ESI) mass spectrometer (Agilent 1260, Agilent 6125B).

High performance liquid chromatography (HPLC) determination conditions were as follows: Gilson high pressure liquid chromatograph (Gilson GX-281), C18 column (10 µM, 19 mm × 250 mm), ultraviolet detection wave band of 220 nm and 254 nm, and elution condition of 5-95% acetonitrile (containing 0.05% v/v formic acid or ammonium bicarbonate), gradient elution for 15 min.

A Biotage Isolera rapid purification system was used for reverse phase purification.

The thin layer chromatography separation and purification was performed using a thin layer chromatography silica gel plate (aluminum plate (20 cm × 20 cm × 1 mm) from Meck or GF 254 produced in Yantai).

Biotage Initiator+ (400 W, RT, ~300 °C) microwave reactor was used for the microwave reactions.

TLC or LCMS was commonly used for reaction monitoring, and the commonly used developer systems were as follows: dichloromethane/methanol, n-hexane/ethyl acetate, and petroleum ether/ethyl acetate, with the volume ratio of the solvents being adjusted according to the polarity of the compound or by adding triethylamine and the like.

The silica gel used for column chromatography was generally 100-200 mesh silica gel. Common eluent systems were as follows: dichloromethane/methanol and petroleum ether/ethyl acetate, with the volume ratio of the solvents being adjusted according to the polarity of the compound or by adding a small amount of triethylamine.

The reagents, solvents, etc., of the present disclosure are commercially available from Aldrich Chemical Company, Energy Chemical, J&K Scientific, Shanghai Bide Pharmatech Co., Ltd., PharmaBlock Sciences, and Shanghai Titan Scientific Co., Ltd.

In the conventional synthesis methods and synthesis examples of the compounds and intermediates of the present disclosure, the meanings of the abbreviations are as follows.

| Abbreviations | Meaning |
|---|---|
| DMF | N,N-dimethylformamide |
| DCM | Dichloromethane |
| Pd₂(dba)₃ | Tris(dibenzylideneacetone)dipalladium |
| Pd(PPh₃)₄ | Tetrakis(triphenylphosphine)palladium |
| tBuXPhos | 2-di-tert-butylphosphino-2',4',6'-triisopropylbiphenyl |
| Pd(PPh₃)₂Cl₂ | Bis(triphenylphosphine)palladium chloride |
| Me₂S | Dimethyl sulfide |
| MeOH | Methanol |
| DAST | Diethylaminosulfur trifluoride |
| DMA | N,N-Dimethylacetamide |
| Bu | Butyl |
| TBAI | Tetrabutylammonium iodide |
| NiCl₂.DME | Nickel chloride dimethoxyethane adduct |
| AcOH | Acetic acid |
| PCy₃ | Tricyclohexylphosphine |
| DMEDA | N,N-Dimethyl ethylenediamine |
| RT | Retention time |
| LC-MS | Liquid chromatography-mass spectrometry system |
| NMR | Nuclear magnetic resonance spectrum |
| HPLC | High performance liquid chromatography |

### Synthesis Examples

### Example 1: Preparation of Compound C1

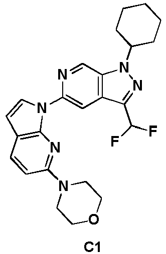

### Synthetic route

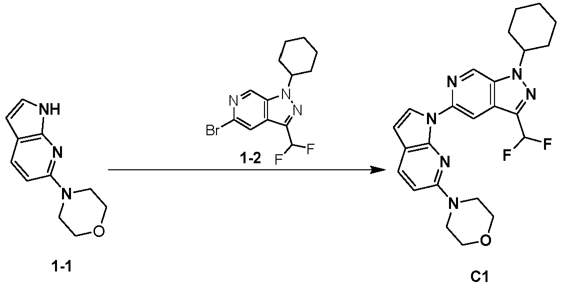

### (1) Preparation of compound 1-1

Compound 1-1 was prepared with reference to the following patent application and literature: 1) WO2012156334 A1; and 2) Storz, Thomas, et al., Synthesis (2008), (2), 201-214.

### (2) Preparation of compound 1-2

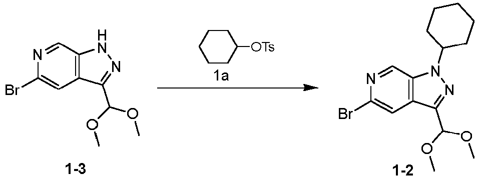

At room temperature, to a single-neck flask were added NaH (882.0 mg, 22.0 mmol) and N,N-dimethylformamide (20 mL), and the mixture was stirred in nitrogen atmosphere for suspending. After compound 1-3 (5.00 g, 18.4 mmol) was dissolved in N,N-dimethylformamide (20 mL), the solution was added dropwise slowly to the NaH suspension at 0 °C in nitrogen atmosphere for reaction. After the completion of the addition, the reaction was continued at 0 °C for 30 min, and compound 1a (9.72 g, 44.0 mmol) was added. The reaction solution was stirred at 25 °C for 16 h. Water (4 mL) was added to the reaction solution. After being stirred for 5 min, the reaction solution was concentrated under reduced pressure, and the residue was purified by column chromatography (0-5% ethyl acetate/petroleum ether) to obtain compound 1-2 (3.0 g).

LC-MS: m/z: 356.1 (M+H)⁺, RT = 0.983 min.

### (3) Preparation of compound C1

Compound 1-1 (200 mg, 0.98 mmol) and N,N-dimethylformamide (8 mL) were stirred until being dissolved, and compound 1-2 (325 mg, 0.98 mmol), tris(dibenzylideneacetone)dipalladium (45.1mg, 0.05 mmol), 2-di-tert-butylphosphino-2',4',6'-triisopropylbiphenyl (41.8 mg, 0.10 mmol), and potassium phosphate (626.60 mg, 2.95 mmol) were added in sequence. After being purged with nitrogen 3 times, the reaction solution was stirred at 90 °C for 16 h. The reaction mixture was filtered, the filtrate was concentrated under reduced pressure, and the crude product was purified by high performance liquid chromatography (formic acid/acetonitrile/water system) to obtain compound C1 (11.2 mg). LC-MS: m/z: 453.1 (M+H)⁺, RT = 1.169min.

### Example 2: Preparation of Compound C2

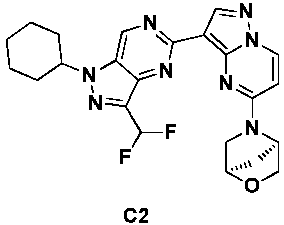

### Synthetic route

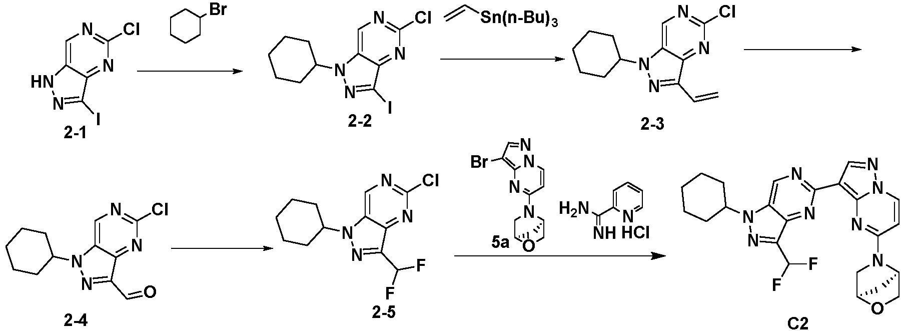

### (1) Preparation of compound 2-1

Compound 2-1 was prepared with reference to the method disclosed in WO2021254493 A1.

### (2) Preparation of compound 2-2

At room temperature, to a solution of compound 2-1 (2.7 g, 9.63 mmol) and bromocyclohexane (2.36 mL, 19.26 mmol) in DMF (30 mL) was added potassium carbonate (3.99 g, 28.89 mmol), and the resulting mixture was stirred at 80 °C for 18 h. The mixture was poured into water (100 mL) and extracted with ethyl acetate (100 mL × 3). The combined organic phases were washed with saturated brine (50 mL), dried over anhydrous sodium sulfate, filtered, and concentrated under reduced pressure to obtain a crude product. The crude product was purified by silica gel chromatography (eluent: petroleum ether:ethyl acetate = 10:1 to 1:1) to obtain compound 2-2 (1.00 g, 2.76 mmol). LC-MS: m/z :363 (M+H)⁺, RT = 2.925 min.

### (3) Preparation of compound 2-3

At room temperature, to a solution of compound 2-2 (1.00 g, 2.76 mmol) and tributyl(vinyl)tin (1.62 mL, 5.52 mmol) in 1,4-dioxane (20 mL) was added bis(triphenylphosphine)palladium chloride (0.97 g, 1.38 mmol). The mixture was purged with nitrogen and stirred at 100 °C for 18 h in nitrogen atmosphere. The mixture was poured into water (100 mL) and extracted with ethyl acetate (50 mL × 3). The combined organic phases were washed with saturated brine (50 mL), dried over anhydrous sodium sulfate, filtered, and concentrated to obtain a crude product. The crude product was purified by silica gel chromatography (eluent: petroleum ether:ethyl acetate = 10:1 to 1:1) to obtain compound 2-3 (500 mg, 1.90 mmol). LC-MS: m/z :262.9 (M+H)⁺, RT = 1.042 min.

### (4) Preparation of compound 2-4

A solution of compound 2-3 (500 mg, 1.90 mmol) in dichloromethane (10 mL) and methanol (10 mL) was purged with nitrogen at -78 °C. Ozone was continuously bubbled into the mixture at -78 °C, and the mixture was stirred for 1 h. The reaction solution was quenched with dimethyl sulfide (355 mg, 5.71 mmol) and extracted with ethyl acetate (30 mL × 3). The combined organic phases were washed with saturated brine (50 mL), dried over anhydrous sodium sulfate, filtered, and concentrated to obtain compound 2-4 (540 mg, 2.04 mmol).

LC-MS: m/z :265 (M+H)⁺, RT = 2.017 min.

### (5) Preparation of compound 2-5

At -30 °C, to a solution of compound 2-4 (540 mg, 2.04 mmol) in dichloromethane (10 mL) was added DAST (0.81 mL, 6.12 mmol), and the mixture was stirred at -30 °C for 18 h. The mixture was poured into water (50 mL) and extracted with ethyl acetate (50 mL × 3). The combined organic phases were washed with saturated brine (50 mL), dried over anhydrous sodium sulfate, filtered, and concentrated to obtain a crude product. The crude product was purified by silica gel chromatography (eluent: petroleum ether:ethyl acetate = 10:1 to 1:1) to obtain compound 2-5 (180 mg, 0.63 mmol).

¹H NMR (400 MHz, DMSO-*d₆*) δ 9.68 (s, 1H), 7.46 (t, *J* = 52.0 Hz, 1H), 4.98-4.86 (m, 1H), 2.10-2.05 (m, 2H), 1.90-1.86 (m, 3H), 1.73-1.70 (m, 1H), 1.55-1.44 (m, 2H), 1.35-1.23 (m, 2H).

### (6) Preparation of compound C2

At room temperature, to a solution of compound 2-5 (70 mg, 0.24) and compound 5a in DMA (4 mL) were added 2-amidinopyridine hydrochloride (76.0 mg, 0.48 mmol), zinc powder (62.0 mg, 0.95 mmol), tetrabutylammonium iodide (87.6 mg, 0.24 mmol), and nickel chloride dimethoxyethane adduct (208 mg, 0.95 mmol), and the mixture was purged with nitrogen 3 times and stirred at 65 °C for 16 h in nitrogen atmosphere. The mixture was cooled to room temperature and filtered, and the filtrate was diluted with water (50 mL) and extracted with ethyl acetate (40 mL × 2). The organic phase was washed with saturated brine (40 mL), dried over anhydrous sodium sulfate, and concentrated under reduced pressure to obtain a crude product. The crude product was subjected to high performance liquid chromatography (ammonium bicarbonate/acetonitrile/water system) to obtain compound C2 (5.5 mg).

¹H NMR (400 MHz, DMSO-*d₆*) δ 9.59 (s, 1H), 8.73 (d, *J =* 7.7 Hz, 1H), 8.52 (s, 1H), 7.39 (t, *J =* 53.4 Hz, 1H), 7.06 (s, 1H), 4.99-4.70 (m, 2H), 3.81 (t, *J =* 12.6 Hz, 2H), 3.60 (d, *J* = 9.8 Hz, 2H), 2.10 - 1.70 (m, 9H), 1.73 (d, *J* = 12.4 Hz, 1H), 1.57 -1.46 (m, 2H), 1.36 - 1.24 (m, 1H).

LC-MS: m/z :467.2 (M+H)⁺, RT = 2.889 min.

### Example 3: Preparation of Compounds C3i and C3ii

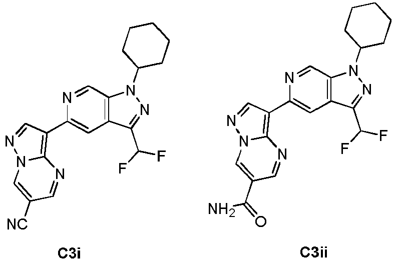

### Synthetic route:

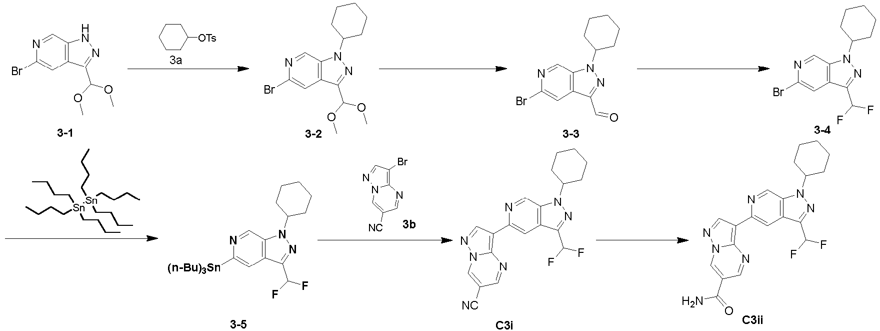

### (1) Preparation of compound 3-2

At room temperature, to a 100-mL single-neck flask were added NaH (882.0 mg, 22.0 mmol) and N,N-dimethylformamide (20 mL), and the mixture was stirred in nitrogen atmosphere for suspending. After compound 3-1 (5.00 g, 18.4 mmol) was dissolved in N,N-dimethylformamide (20 mL), the solution was added dropwise slowly to the NaH suspension at 0 °C in nitrogen atmosphere for reaction. After the completion of the addition, the reaction was continued at 0 °C for 30 min. Compound 3a (9.72 g, 44.0 mmol) was added, and the reaction solution was stirred at 25 °C for 16 h. Water (4 mL) was added to the reaction solution. After being stirred for 5 min, the reaction solution was concentrated under reduced pressure, and the residue was purified by column chromatography (0-5% ethyl acetate/petroleum ether) to obtain compound 3-2 (3.0 g).

LC-MS: m/z: 356.1 (M+H)⁺, RT = 0.983 min.

### (2) Preparation of compound 3-3

At room temperature, to a 100-mL single-neck flask were added compound 3-2 (3.20 g, 9.03 mmol), acetic acid (50 mL), and water (10 mL), and the reaction solution was stirred at 80 °C for 2 h. The reaction solution was then concentrated under reduced pressure. The residue was dissolved in ethyl acetate (60 mL) and washed with a saturated sodium bicarbonate solution (50 mL). The organic phase was dried over anhydrous sodium sulfate and filtered, and the filtrate was concentrated under reduced pressure to obtain compound 3-3 (2.50 g). The crude product was directly used in the next step. LC-MS: m/z: 307.9(M+H)⁺.

### (3) Preparation of compound 3-4

At room temperature, to a 100-mL single-neck flask were added compound 3-3 (1.70 g,5.52 mmol) and dichloromethane (40 mL), and the mixture was stirred until being dissolved. Diethylaminosulfur trifluoride (4.45 g, 27.6 mmol) was added at 0 °C, and the reaction solution was stirred at 25 °C for 16 h. The reaction solution was poured into ice water (30 mL) and extracted with dichloromethane (20 mL × 3). The organic phases were combined, dried over anhydrous sodium sulfate, filtered, and concentrated to obtain a crude product. The crude product was separated by column chromatography (petroleum ether/ethyl acetate = 20/1) to obtain compound 3-4 (0.90 g).

¹H NMR (400 MHz, CDCl₃) δ 8.80 (d, *J =* 1.1 Hz, 1H), 7.99 (s, 1H), 6.95 (t, *J* = 54.3 Hz, 1H), 4.54 - 4.47 (m, 1H), 2.14 - 1.95 (m, 7H), 1.87 - 1.74 (m, 1H), 1.57 - 1.45 (m, 2H), 1.42 - 1.25 (m, 1H).

LC-MS: m/z: 331.8(M+H)⁺, RT = 1.025 min.

### (4) Preparation of compound 3-5

At room temperature, to a 100-mL single-neck flask were added compound 3-4 (1.90 g, 5.75 mmol) and dioxane (50 mL), and the mixture was stirred until being dissolved. Hexabutylditin (4.00 g, 6.91 mmol), tris(dibenzylideneacetone)dipalladium (263.5 mg, 0.29 mmol), tricyclohexylphosphine (161.5 mg, 0.58 mmol), and lithium chloride (1.46 g, 34.5 mmol) were added, and the reaction solution was purged with nitrogen 3 times and stirred at 100 °C for 16 h. The reaction solution was filtered, the filtrate was concentrated under reduced pressure, and the residue was purified by column chromatography (0-5% ethyl acetate/petroleum ether) to obtain compound 3-5 (2.20 g). LC-MS: m/z: 542.1 (M+H)⁺, RT = 1.083 min.

### (5) Preparation of compound C3i

At room temperature, to a 40-mL single-neck flask were added compound 3b (300.0 mg, 1.35 mmol) and toluene (10 mL), and the mixture was stirred sufficiently. Compound 3-5 (727 mg, 1.35 mmol) and tetrakis(triphenylphosphine)palladium (155 mg, 0.13 mmol) were added. The reaction solution was stirred at 120 °C for 16 h in nitrogen atmosphere. After the reaction was completed as detected by LC-MS, the reaction solution was concentrated under reduced pressure. The crude product was separated by column chromatography (petroleum ether/ethyl acetate = 4/1) to obtain product C3i (120.0 mg).

¹H NMR (400 MHz, CDCl₃) δ 9.16 -9.04 (m, 3H), 8.82 (s, 1H), 8.75 - 8.68 (m, 1H), 7.18 - 6.90 (m, 1H), 4.66 - 4.51 (m, 1H), 2.20 - 1.98 (m, 6H), 1.87 -1.79 (m, 1H), 1.56 - 1.47 (m, 2H), 1.43 - 1.34 (m, 1H).

LC-MS: m/z :394.1(M+H)⁺, RT = 1.01 min.

### (6) Preparation of compound C3ii

At room temperature, to an 8-mL single-neck flask were added compound C3i (70.0 mg, 0.18 mmol) and sulfuric acid (1.5 mL). The reaction solution was stirred at 25 °C for 1 h in nitrogen atmosphere. After the reaction was completed as detected by LC-MS, the reaction solution was poured into ice water (50 mL), and the pH was adjusted to 9-10 with a saturated sodium carbonate solution. Ethyl acetate (30 mL × 3) was added for extraction. The organic phases were combined, dried over anhydrous sodium sulfate, filtered, and concentrated to obtain a crude product. The crude product was separated by column chromatography (petroleum ether/ethyl acetate = 3/1) to obtain product C3ii (18.9 mg).

¹H NMR (400 MHz, DMSO-*d₆*) δ 9.70 - 9.61 (m, 1H), 9.50 - 9.41 (m, 1H), 9.21 - 9.12 (m, 1H), 9.04 - 8.96 (m, 1H), 8.86 - 8.78 (m, 1H), 8.32 - 8.20 (m, 1H), 7.87 - 7.76 (m, 1H), 7.63 - 7.33 (m, 1H), 2.14 - 2.04 (m, 2H), 1.97 - 1.85 (m, 4H), 1.79 - 1.71 (m, 1H),1.61 - 1.48 (m, 2H), 1.38 - 1.30 (m, 1H).

LC-MS: m/z :412.1(M+H)⁺, RT = 0.908 min.

### Example 4: Preparation of Compounds C4i and C4ii

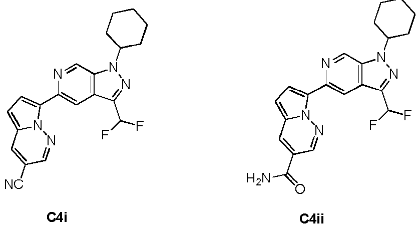

### Synthetic route

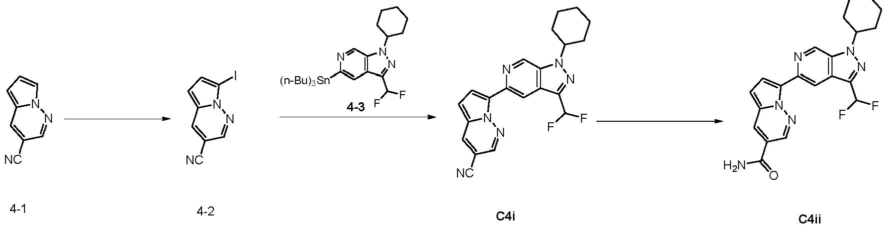

### (1) Preparation of compound 4-1

Compound 4-1 was prepared with reference to the method disclosed in WO2015117563 A1.

### (2) Preparation of compound 4-2

At room temperature, to acetonitrile (10 mL) were added compound 4-1 (300 mg, 2.10 mmol) and N-iodosuccinimide (707 mg, 3.14 mmol). The reaction solution was stirred at 60 °C for 1 h. After the reaction was completed as detected by TLC, a sodium sulfite solution (10 mL) was carefully added to the reaction solution. The mixture was then extracted with ethyl acetate (100 mL), and the organic phase was washed with saturated brine (100 mL × 2), dried over anhydrous sodium sulfate, filtered, and concentrated under reduced pressure. The residue was subjected to flash chromatography (10% ethyl acetate in petroleum ether) to obtain compound 4-2 (270 mg, 1.00 mmol).

¹H NMR (400 MHz, CDCl₃) δ 8.27 (d, *J =* 2.1 Hz, 1H), 8.05 (d, *J =* 2.1 Hz, 1H), 7.24 (d, *J* = 4.7 Hz, 1H), 6.95 (d, *J* = 4.7 Hz, 1H).

### (3) Preparation of C4i

At room temperature, to a 40-mL IKA flask was added compound 4-2 (50 mg, 0.19 mmol) and toluene (1 mL) in sequence, then compound 4-3 (110.46 mg, 0.20 mmol) and tetrakis(triphenylphosphine)palladium (21.48 mg, 0.02 mmol) were added to the reaction flask, and finally the mixture was purged by bubbling with nitrogen for 1 min. The reaction solution was stirred at 120 °C for 16 h. After the starting materials were completely consumed as detected by LC-MS, water (2 mL) and ethyl acetate (2 mL) were added to the reaction solution, followed by extraction with ethyl acetate (15 mL × 3). The organic phase was concentrated under reduced pressure, and the residue was purified by high performance liquid chromatography (water/ammonia/acetonitrile) to obtain compound C4i (3.87 mg, 0.01 mmol).

¹H NMR (400MHz, DMSO-*d₆*) δ 9.51 (br s, 1H), 9.11 (br s, 1H), 8.89 - 8.70 (m, 2H), 7.91 (br s, 1H), 7.72 - 7.33 (m, 1H), 7.15 (br s, 1H), 4.93 (br s, 1H), 2.08 (br s, 2H), 1.91 (br s, 4H), 1.73 (br s, 1H), 1.55 (br d, *J=*8.6 Hz, 2H), 1.29 (br s, 1H).

LC-MS: m/z :393.1 (M+H)⁺, RT = 1.051 min.

### (4) Preparation of C4ii

At room temperature, to a 40-mL IKA flask was added compound C4i (50 mg, 0.13 mmol), which was dissolved in hydrochloric acid (0.5 mL) and acetic acid (0.5 mL). The reaction solution was stirred at 20 °C for 16 h. After the starting materials were completely consumed as detected by LC-MS, water (10 mL) and ethyl acetate (5 mL) were added to the reaction solution, followed by extraction with ethyl acetate (30 mL × 3). The organic phase was concentrated under reduced pressure, and the residue was purified by high performance liquid chromatography (water/ammonia/acetonitrile) to obtain C4ii (6.8 mg, 0.02 mmol).

¹H NMR (400MHz, DMSO-*d₆*) δ 9.49 (s, 1H), 9.19 (s, 1H), 8.89 (d, *J*=2.2 Hz, 1H), 8.64 (d, *J*=2.2 Hz, 1H), 8.11 (br s, 1H), 7.83 (d, *J*=4.6 Hz, 1H), 7.65 - 7.34 (m, 2H), 7.04 (d, *J*=4.6 Hz, 1H), 5.07 - 4.78 (m, 1H), 2.10 (br d, *J*=9.8 Hz, 2H), 1.99 - 1.85 (m, 4H), 1.74 (br d, *J*=11.9 Hz, 1H), 1.63 - 1.46 (m, 2H), 1.44 - 1.24 (m, 1H).

LC-MS: XT221418-51-P1A1, m/z: 411.3 (M+H)⁺, RT = 0.954 min.

### Example 5: Preparation of Compounds C5i and C5ii

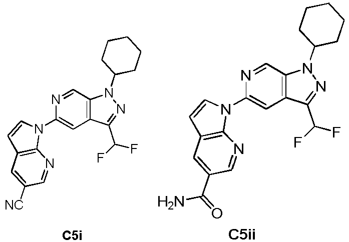

### Synthetic route:

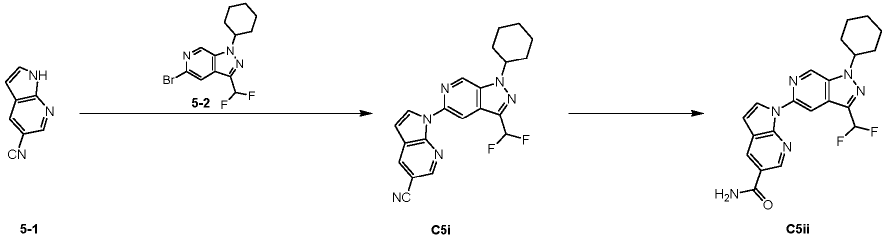

### (1) Preparation of C5i

At room temperature, to a 40-mL IKA flask were added compound 5-1 (346.84 mg, 2.42 mmol) and *N,N*-dimethylformamide (5 mL) in sequence, and the mixture was stirred until being dissolved. Compound 5-2 (800.0 mg, 2.42 mmol), N,N-dimethyl ethylenediamine (2.14 mg, 0.02 mmol), cuprous iodide (4.61 mg, 0.02 mmol), and potassium phosphate (1028.58 mg, 4.85 mmol) were added in sequence. The reaction solution was purged with nitrogen 3 times and stirred at 110 °C for 12 h. The reaction solution was concentrated under reduced pressure, and the residue was subjected to column chromatography (0-50% petroleum ether and tetrahydrofuran) to obtain C5i (150 mg).

LC-MS: m/z :393.3 (M+H)⁺, RT = 1.108 min.

### (2) Preparation of C5ii

At room temperature, to an 8-mL IKA flask were added compound C5i (120 mg, 0.31 mmol) and concentrated sulfuric acid (2 mL) in sequence, and the mixture was stirred until being dissolved. The reaction solution was stirred at 25 °C for 1 h. After the reaction was completed as detected, the reaction solution was added dropwise slowly to a saturated sodium carbonate solution in an ice-water bath, extracted three times with ethyl acetate, and concentrated under reduced pressure, and the residue was added to water and acetonitrile, and the mixture was lyophilized to obtain C5ii (55.68 mg).

¹H NMR (400 MHz, DMSO-*d₆*) δ 9.50 - 9.37 (m, 1H), 9.14 (s, 1H), 8.97 (d, *J* = 1.9 Hz, 1H), 8.61 (d, *J* = 2.0 Hz, 1H), 8.50 (d, *J =* 3.8 Hz, 1H), 8.12 (br s, 1H), 7.68 - 7.30 (m, 2H), 6.89 (d, *J =* 3.9 Hz, 1H), 5.01 - 4.89 (m, 1H), 2.16 - 2.06 (m, 2H), 1.98 - 1.87 (m, 3H), 1.75 (br d, *J =* 12.5 Hz, 1H), 1.63 - 1.46 (m, 2H), 1.37 - 1.17 (m, 2H).

LC-MS: m/z :411.0 (M+H)⁺, RT = 3.558 min.

Other compounds of the present disclosure can be prepared by methods analogous to those described in the examples above (with appropriate modification, if necessary).

### Biological Assays:

### Experimental Example 1: IRAK4 Kinase Inhibition Assay

In this assay, the Cisbio HTRF KinEASE-STK SI kit (62S1PEB) was used. The assay/detection buffer, STK Substrate 1-biotin, Streptavidin-XL665, and STK Antibody-Crypatete were all contained in the kit.

After the compound/DMSO was added to a 384-well plate (PerkinElmer 6008280) using Echo 665, 5 µL of 2-fold reaction concentration of IRAK4 kinase protein (final concentration: 10 nM) dissolved with an assay buffer [1 volume of 5× kinase buffer + 20 mM magnesium chloride (Sigma) solution + 1 mM DTT (Alfa Aesar) solution + 4 volumes of deionized water] was added. The shallow well plate was sealed, shaken, centrifuged at 1000 rpm for 1 min, and then incubated at room temperature for 5 min. Then, 5 µL of 2-fold reaction concentration of a 1:1 mixture of STK Substrate 1-biotin (final concentration: 1 µM) and ATP (Sigma A1852) (final concentration: 200 µM) formulated with the assay buffer was added. The shallow well plate was sealed, shaken, centrifuged at 1000 rpm for 1 min, and then incubated at 37 °C for 2 h, followed by the addition of 10 µL of a 1:1 mixture of Streptavidin-XL665 (final concentration: 62.5 nM) and STK-antibody-cryptate dissolved with a detection buffer. The shallow well plate was sealed, shaken, centrifuged at 1000 rpm for 1 min, and then incubated at room temperature for 1 h, and then the value at the wavelength of 620 nm/665 nm was read using a microplate reader. The data were analyzed using Prism 9.3 software, and the IC₅₀ value of IRAK4 kinase inhibition was fitted using a four parameter Logistic equation. The test results are shown in Table 1.

**Table 1. IRAK4 inhibitory activity of compounds**

| Test compound | IC₅₀ (uM) |
|---|---|
| C5i | D |
| C5ii | B |
| C3i | B |
| C1 | C |
| C4ii | A |

| | |
|---|---|
| Note: A < 1 uM; 1 uM ≤ B ≤ 5 uM; 5 uM < C ≤ 10 uM; 10 uM < D. | |

### Experimental Example 2: Detection of Protein Degradation by HiBit Fluorescence

The HiBiT-containing tag was connected to an IRAK4 plasmid (Beijing Tsingke Biotech Co., Ltd.) using the HiBiT tag technology, and the plasmid was transfected into HEK293T cells (Chinese Academy of Sciences, 1101HUM-PUMC000212). The HiBiT fluorescence level was detected to characterize the degradation level of the compound for IRAK4. 300,000 HEK293T cells were seeded onto a 6-well plate (Corning 3516) with 1 mL of fresh medium, and the plate was then incubated overnight in a constant temperature incubator at 37 °C and 5% CO₂. 3 µL of X-tremeGENE^{™} 9 DNA Transfection Reagent (Roche #06365787001) and 1 µg of pcdna3.1-IRAK4-HiBiT (Beijing Tsingke Biotech Co., Ltd.) were added to 200 µL of Opti-MEM (Gibico 31985062) and well mixed gently, the 6-well plate incubated overnight was taken out and left to stand at room temperature for 15 min, and then the obtained mixture was added dropwise to the 6-well plate. After 6 h of transient transfection, cells were digested with trypsin and centrifuged at 1000 rpm for 3 min, and then 5000 HEK293T cells were seeded onto a 384-well plate (Corning 3765) with 40 µL of fresh medium, and the plate was incubated overnight in a constant temperature incubator at 37 °C and 5% CO₂. Then, 40 nL of compound was added to the 384-well plate using an Echo 655 Liquid Handler (a 1:3 serial dilution from a maximum final concentration of 10 µM for a total of 10 concentrations). The plate was then incubated for 24 h at 37 °C and 5% CO₂. The 3 84-well plate was then taken out and allowed to equilibrate to room temperature. 40 µL of Nano-Glo^{®} HiBiT Lytic Detection System (Promega N3030) was added to each well, followed by incubation at room temperature for 15 min. The fluorescence value was recorded using the EnVision Xcite Multilabel Reader (PerkinElmer 2105-0020). The data were analyzed using Prism 9.3 software, and the DC₅₀ value of IRAK4 degradation was fitted using a four parameter Logistic equation.

In addition to those described herein, it will be apparent to those skilled in the art that various modifications of the present disclosure can be made based on the foregoing description. Such modifications are also intended to fall within the scope of the appended claims. The references (including all patents, patent applications, journal articles, books, and any other publications) cited in the present disclosure are each incorporated herein by reference in its entirety.

## Claims

1. A compound of formula (I): 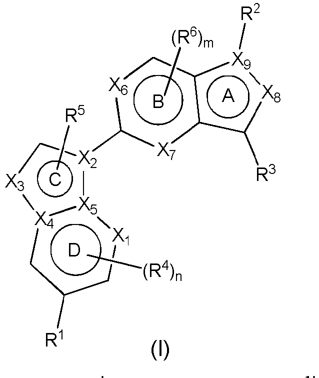
or a stereoisomer, a tautomer, a diastereoisomer, a racemate, a cis-trans isomer, an isotope-labeled compound (preferably a deuteride), an N-oxide, a metabolite, an ester, a prodrug, a crystalline form, a hydrate, a solvate, or a pharmaceutically acceptable salt thereof,
wherein:
X₁, X₂, X₃, X₄, X₅, X₆, X₇, X₈, and X₉ are each independently C, CR, or N, provided that valency of all atoms is met and X₄ and X₅ are not both N at the same time;
R, R¹, R⁴, and R⁵ are each independently selected from: hydrogen, deuterium, R⁷, halogen, CN, NO₂, C₁₋₆ alkyl, -OR^{1f}, -SR^{1f}, -NR^{1d}R^{1e}, -S(O)₂R^{1f}, -S(O)R^{1f}, -S(O)₂-NR^{1d}R^{1e}, -S(O)-NR^{1d}R^{1e}, -P(O)(OR^{1f})₂, - P(O)(NR^{1d}R^{1e})₂, -CF(R^{1f})₂, -CF₂(R^{1f}), -CF₃, -CCl(R^{1f})₂, -CCl₂(R^{1f}), -CCl₃, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, -C(O)R^{1f}, -C(O)OR^{1f}, -C(O)NR^{1d}R^{1e}, -C(O)NR^{1f}-OR^{1f}, -OC(O)R^{1f}, - OC(O)NR^{1d}R^{1e}, -NR^{1f}-C(O)OR^{1f}, -NR^{1f}-C(O)R^{1f}, -NR^{1f}-C(O)NR^{1d}R^{1e}, and -NR^{1f}-S(O)₂R^{1f}, wherein
p is 1, 2, or 3;
n is 0, 1, or 2, wherein when n is 1 or 2, R⁴ is attached to an available ring member of ring D; and
R⁵ is attached to an available ring member of ring C;
R² is selected from: saturated or partially unsaturated C₃₋₇ cyclohydrocarbyl, 3-7 membered saturated or partially unsaturated heterocyclyl having 1-3 heteroatoms independently selected from nitrogen, oxygen, and sulfur, C₆₋₁₀ aryl, and 5-10 membered heteroaryl having 1-4 heteroatoms independently selected from nitrogen, oxygen, and sulfur, wherein the cyclohydrocarbyl, heterocyclyl, aryl, and heteroaryl are optionally substituted with 1, 2, or more substituents independently selected from halogen, CN, OH, SH, NO₂, C₁₋₆ alkyl, C₁₋₆ haloalkyl, C₁₋₆ alkoxy, C₁₋₆ alkylthio, -NR^{2d}R^{2e}, -C(O)OR^{2f}, and -C(O)NR^{2d}R^{2e}, and in the case where two substituents are attached to the same ring carbon atom of the cyclohydrocarbyl or heterocyclyl, the two substituents, together with the ring carbon atom to which they are attached, optionally form saturated or partially unsaturated optionally substituted C₃₋₇ cyclohydrocarbyl or 3-7 membered saturated or partially unsaturated optionally substituted heterocyclyl having 1-3 heteroatoms independently selected from nitrogen, oxygen, and sulfur;
R³ and R⁶ are each independently selected from: H, R⁸, halogen, CN, NO₂, C₁₋₆ alkyl, -OR^{3f}, -SR^{3f}, - NR^{3d}R^{3e}, -S(O)₂R^{3f}, -S(O)R^{3f}, -S(O)₂-NR^{3d}R^{3e}, -S(O)-NR^{3d}R^{3e}, -P(O)(OR^{3f})₂, -P(O)(NR^{3d}R^{3e})₂, -CF(R^{3f})₂,-CF₂(R^{3f}), -CF₃, -CCl(R^{3f})₂, -CCl₂(R^{3f}), -CCl₃, -(CR^{3a}R^{3b})_{q}-OR^{3f}, -(CR^{3a}R^{3b})_{q}-C(O)OR^{3f}, -(CR^{3a}R^{3b})_{q}-NR^{3d}R^{3e}, -C(O)R^{3f}, -C(O)OR^{3f}, and -C(O)NR^{3d}R^{3e}, wherein
q is 1, 2, or 3;
m is 0, 1, 2, or 3, wherein when m is 1, 2, or 3, R⁶ is attached to an available ring member of ring B;
R^{1a}, R^{1b}, R^{3a}, and R^{3b} are each independently selected from hydrogen, deuterium, halogen, CN, OH, SH, NO₂, C₁₋₆ alkyl, C₁₋₆ haloalkyl, C₁₋₆ alkoxy, C₁₋₆ alkylthio, and -NR^{7d}R^{7e}; or, R^{1a} and R^{1b}, or R^{3a} and R^{3b}, together with the carbon atom to which they are both attached, form R⁹;
R^{1d}, R^{1e}, R^{2d}, R^{2e}, R^{3d}, R^{3e}, R^{7d}, and R^{7e} are each independently selected from hydrogen, deuterium, R¹¹, C₁₋₆ alkyl, and C₁₋₆ haloalkyl; or, R^{1d} and R^{1e}, or R^{2d} and R^{2e}, or R^{3d} and R^{3e}, or R^{7d} and R^{7e}, together with the nitrogen atom to which they are both attached, form R¹⁰;
R^{1f}, R^{2f}, and R^{3f} are independently selected from hydrogen, deuterium, C₁₋₆ alkyl, C₁₋₆ haloalkyl, and R¹²;
R⁷, R⁸, R⁹, R¹¹, and R¹² are each independently selected from: saturated or partially unsaturated C₃₋₇ cyclohydrocarbyl, 3-10 membered saturated or partially unsaturated heterocyclyl having 1-3 heteroatoms independently selected from nitrogen, oxygen, and sulfur, C₆₋₁₀ aryl, and 5-10 membered heteroaryl having 1-4 heteroatoms independently selected from nitrogen, oxygen, and sulfur, wherein the cyclohydrocarbyl, heterocyclyl, aryl, and heteroaryl are optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e};
R¹⁰ is selected from: 3-7 membered saturated or partially unsaturated heterocyclyl having 1 nitrogen heteroatom and optionally 1-2 additional heteroatoms independently selected from nitrogen, oxygen, and sulfur, and 5-10 membered heteroaryl having 1 nitrogen heteroatom and optionally 1-3 additional heteroatoms independently selected from nitrogen, oxygen, and sulfur, wherein the heterocyclyl and heteroaryl are optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{10d}R^{10e}; and
R^{8d}, R^{8e}, R^{10d}, and R^{10e} are each independently selected from hydrogen, deuterium, C₁₋₆ alkyl, and C₁₋₆ haloalkyl.

2. The compound according to claim 1, wherein:
R¹ and R⁴ are not both hydrogen or deuterium at the same time;
and/or
R^{1a}, R^{1b}, R^{3a}, and R^{3b} are each independently selected from hydrogen, halogen, CN, OH, SH, NO₂, C₁₋₄ alkyl, C₁₋₄ haloalkyl, C₁₋₄ alkoxy, C₁₋₄ alkylthio, and -NR^{7d}R^{7e}; or, R^{1a} and R^{1b}, or R^{3a} and R^{3b}, together with the carbon atom to which they are both attached, form R⁹;
and/or
R^{1d}, R^{1e}, R^{2d}, R^{2e}, R^{3d}, R^{3e}, R^{7d}, and R^{7e} are each independently selected from hydrogen, R¹¹, C₁₋₄ alkyl, and C₁₋₄ haloalkyl; or, R^{1d} and R^{1e}, or R^{2d} and R^{2e}, or R^{3d} and R^{3e}, or R^{7d} and R^{7e}, together with the nitrogen atom to which they are both attached, form R¹⁰;
and/or
R^{1f}, R^{2f}, and R^{3f} are independently selected from hydrogen, C₁₋₄ alkyl, C₁₋₄ haloalkyl, and R¹²;
and/or
R⁷, R⁸, R⁹, R¹¹, and R¹² are each independently selected from: saturated or partially unsaturated C₃₋₆ cyclohydrocarbyl, 3-10 membered saturated or partially unsaturated heterocyclyl having 1-3 heteroatoms independently selected from nitrogen, oxygen, and sulfur, C₆₋₁₀ aryl, and 5-6 membered heteroaryl having 1-3 heteroatoms independently selected from nitrogen, oxygen, and sulfur, wherein the cyclohydrocarbyl, heterocyclyl, aryl, and heteroaryl are optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e};
and/or
R¹⁰ is selected from: 3-6 membered saturated or partially unsaturated heterocyclyl having 1 nitrogen heteroatom and optionally 1-2 additional heteroatoms independently selected from nitrogen, oxygen, and sulfur, and 5-6 membered heteroaryl having 1 nitrogen heteroatom and optionally 1-2 additional heteroatoms independently selected from nitrogen, oxygen, and sulfur, wherein the heterocyclyl and heteroaryl are optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{10d}R^{10e};
and/or
R^{8d}, R^{8e}, R^{10d}, and R^{10e} are each independently selected from hydrogen, C₁₋₆ alkyl, and C₁₋₆ haloalkyl.

3. The compound according to claim 1 or 2, wherein:
R¹ is not hydrogen; or
R¹ is selected from: R⁷, halogen, CN, NO₂, C₁₋₄ alkyl, -OR^{1f}, -SR^{1f}, -NR^{1d}R^{1e}, -S(O)₂R^{1f}, -S(O)R^{1f}, - S(O)₂-NR^{1d}R^{1e}, -S(O)-NR^{1d}R^{1e}, -P(O)(OR^{1f})₂, -P(O)(NR^{1d}R^{1e})₂, -CF(R^{1f})₂, -CF₂(R^{1f}), -CF₃, -CCl(R^{1f})₂, - CCl₂(R^{1f}), -CCl₃, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, -C(O)R^{1f}, -C(O)OR^{1f}, - C(O)NR^{1d}R^{1e}, -C(O)NR^{1f}-OR^{1f}, -OC(O)R^{1f}, -OC(O)NR^{1d}R^{1e}, -NR^{1f}-C(O)OR^{1f}, -NR^{1f}-C(O)R^{1f}, -NR^{1f}-C(O)NR^{1d}R^{1e}, or -NR^{1f}-S(O)₂R^{1f}; or
R¹ is selected from: R⁷, halogen, CN, NO₂, C₁₋₄ alkyl, -OR^{1f}, -SR^{1f}, -NR^{1d}R^{1e}, -CF(R^{1f})₂, -CF₂(R^{1f}), - CF₃, -CCl(R^{1f})₂, -CCl₂(R^{1f}), -CCl₃, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, - C(O)OR^{1f}, or -C(O)NR^{1d}R^{1e}; or
R¹ is selected from: R⁷, halogen, CN, -OR^{1f}, -NR^{1d}R^{1e}, -(CR^{1a}R^{1b})ₚ-OR^{1f}, -(CR^{1a}R^{1b})ₚ-C(O)OR^{1f}, - C(O)OR^{1f}, or -C(O)NR^{1d}R^{1e}.

4. The compound according to any one of claims 1 to 3, wherein:
R⁷ is selected from: C₃₋₆ cycloalkyl, 4-6 membered saturated or partially unsaturated heterocyclyl having 1-3 heteroatoms independently selected from nitrogen, oxygen, and sulfur, phenyl, and 5-6 membered heteroaryl having 1-3 heteroatoms independently selected from nitrogen, oxygen, and sulfur, wherein the cycloalkyl, heterocyclyl, phenyl, and heteroaryl are optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e}; or
R⁷ is selected from: C₃₋₆ cycloalkyl, 4-6 membered heterocycloalkyl having 1-3 heteroatoms independently selected from nitrogen, oxygen, and sulfur, phenyl, and 5-6 membered heteroaryl having 1-3 heteroatoms independently selected from nitrogen, oxygen, and sulfur, wherein the cycloalkyl, heterocycloalkyl, phenyl, and heteroaryl are optionally substituted with one or more substituents independently selected from halogen, CN, OH, NO₂, C₁₋₄ alkyl, C₁₋₄ haloalkyl, C₁₋₄ alkoxy, and -NR^{8d}R^{8e};
wherein: preferably, R^{8d} and R^{8e} are each independently selected from hydrogen and C₁₋₆ alkyl; or, preferably, R^{8d} and R^{8e} are each independently selected from hydrogen and C₁₋₄ alkyl;
or
R⁷ is selected from: 4-6 membered heterocycloalkyl having 1 N atom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
R⁷ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
R⁷ is selected from: azetidinyl, pyrrolidinyl, and piperidinyl.

5. The compound according to any one of claims 1 to 4, wherein:
p is 1;
and/or
R^{1a} and R^{1b} are each independently selected from hydrogen and C₁₋₆ alkyl; or
R^{1a} and R^{1b} are each independently selected from hydrogen and C₁₋₄ alkyl; or
R^{1a} and R^{1b} are each independently hydrogen; or
R^{1a} and R^{1b}, together with the carbon atom to which they are both attached, form R⁹;
wherein:
preferably, R⁹ is selected from: C₃₋₆ cycloalkyl, 3-6 membered saturated or partially unsaturated heterocyclyl having 1-3 heteroatoms independently selected from nitrogen, oxygen, and sulfur, C₆₋₁₀ aryl, and 5-6 membered heteroaryl having 1-3 heteroatoms independently selected from nitrogen, oxygen, and sulfur, wherein the cycloalkyl, heterocyclyl, aryl, and heteroaryl are optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e};
wherein, preferably, R^{8d} and R^{8e} are each independently selected from hydrogen and C₁₋₆ alkyl; or, preferably, R^{8d} and R^{8e} are each independently selected from hydrogen and C₁₋₄ alkyl;
or
preferably, R⁹ is selected from: C₃₋₆ cycloalkyl, wherein the cycloalkyl is optionally substituted with one or more substituents independently selected from F, Cl, Br, CN, OH, NO₂, C₁₋₄ alkyl, C₁₋₄ haloalkyl, C₁₋₄ alkoxy, NH₂, -NH(C₁₋₄ alkyl) and N(C₁₋₄ alkyl)₂; or
preferably, R⁹ is selected from: cyclopropyl, cyclobutyl, cyclopentyl, and cyclohexyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
preferably, R⁹ is cyclopropyl.

6. The compound according to any one of claims 1 to 5, wherein:
R^{1d} and R^{1e} are each independently selected from hydrogen, R¹¹, and C₁₋₆ alkyl; or
R^{1d} and R^{1e} are each independently selected from hydrogen, R¹¹, and C₁₋₄ alkyl; or
R^{1d} and R^{1e} are each independently selected from hydrogen, R¹¹, methyl, ethyl, propyl, isopropyl, n-butyl, isobutyl, sec-butyl, and tert-butyl; or
R^{1d} and R^{1e}, together with the nitrogen atom to which they are both attached, form R¹⁰;
wherein:
preferably, R¹¹ is selected from: C₃₋₆ cycloalkyl, 3-6 membered saturated or partially unsaturated heterocyclyl having 1-3 heteroatoms independently selected from nitrogen, oxygen, and sulfur, C₆₋₁₀ aryl, and 5-6 membered heteroaryl having 1-3 heteroatoms independently selected from nitrogen, oxygen, and sulfur, wherein the cycloalkyl, heterocyclyl, aryl, and heteroaryl are optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e};
wherein, preferably, R^{8d} and R^{8e} are each independently selected from hydrogen and C₁₋₆ alkyl; or, preferably, R^{8d} and R^{8e} are each independently selected from hydrogen and C₁₋₄ alkyl;
or
preferably, R¹¹ is selected from: C₃₋₆ cycloalkyl, and 3-6 membered heterocycloalkyl having 1 nitrogen heteroatom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂;
or
preferably, R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
preferably, R¹¹ is selected from: cyclopropyl, cyclobutyl, cyclopentyl, cyclohexyl, azetidinyl, pyrrolidinyl, and piperidinyl, each of which is optionally substituted with one or more substituents independently selected from F and Cl; or
preferably, R¹¹ is selected from cyclopropyl and 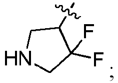 and/or
preferably, R¹⁰ is selected from: 3-6 membered heterocycloalkyl having 1 nitrogen heteroatom and optionally 1-2 additional heteroatoms independently selected from nitrogen, oxygen, and sulfur, and 5-6 membered heteroaryl having 1 nitrogen heteroatom and optionally 1-2 additional heteroatoms independently selected from nitrogen, oxygen, and sulfur, wherein the heterocycloalkyl and heteroaryl are optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
preferably, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄)alkyl, and N(C₁₋₄ alkyl)₂; or
preferably, R¹⁰ is selected from: 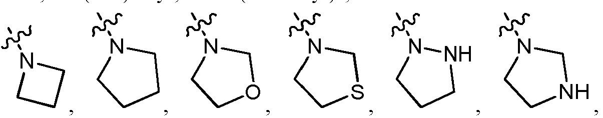 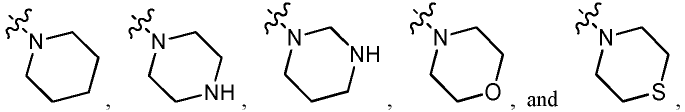 each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
preferably, R¹⁰ is selected from: 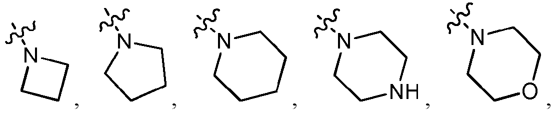 and 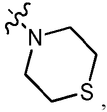 each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂.

7. The compound according to any one of claims 1 to 6, wherein:
R^{1f} is selected from hydrogen and C₁₋₆ alkyl; or
R^{1f} is selected from hydrogen and C₁₋₄ alkyl; or
R^{1f} is hydrogen.

8. The compound according to any one of claims 1 to 7, wherein R¹ is selected from: F, Cl, Br, CN, OH, NH₂, -NHCH₃, -C(O)OH, -C(O)NH₂, -CH₂NH₂, -CH₂OH, 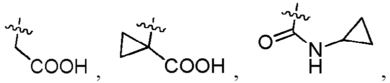 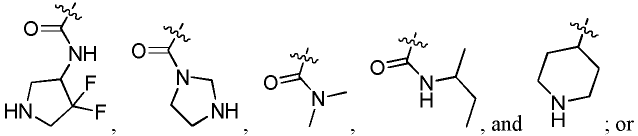 R¹ is selected from -CN, -C(O)NH₂, F, -NHCH₃, -C(O)OH,

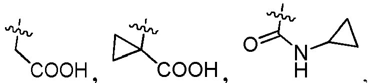

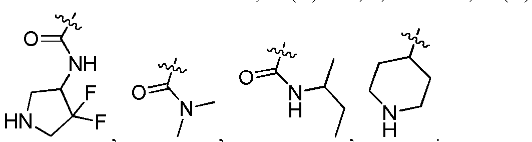

9. The compound according to any one of claims 2 to 8, wherein the compound has a structure of formula (I-1): 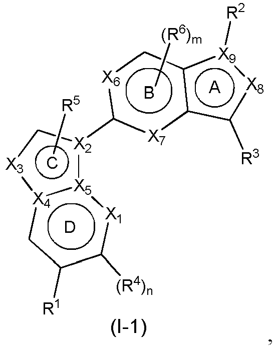 wherein n is 0 or 1.

10. The compound according to claim 1 or 2, wherein:
R¹ is selected from hydrogen and deuterium, and n is 1 or 2;
or
R¹ is hydrogen, and n is 1;
or
the compound has a structure of formula (I-2): 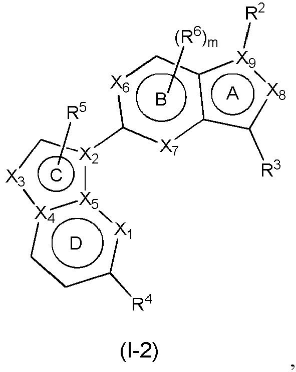 provided that R⁴ is not hydrogen or deuterium.

11. The compound according to any one of claims 1 to 10, wherein:
R⁴ is selected from: hydrogen, R⁷, halogen, CN, NO₂, -OR^{1f}, -SR^{1f}, and -NR^{1d}R^{1e};
wherein:
preferably, R^{1d} and R^{1e} are each independently selected from hydrogen and C₁₋₆ alkyl; or
preferably, R^{1d} and R^{1e} are each independently selected from hydrogen and C₁₋₄ alkyl;
and/or
wherein:
preferably, R^{1f} is selected from hydrogen and C₁₋₆ alkyl; or
preferably, R^{1f} is selected from hydrogen and C₁₋₄ alkyl;
or
R⁴ is selected from: hydrogen, R⁷, F, Cl, Br, CN, NO₂, OH, and NH₂; or
R⁴ is selected from: hydrogen, R⁷, and CN;
wherein:
preferably, R⁷ is selected from: 4-6 membered saturated monocyclic heterocyclyl, 8-10 membered saturated fused bicyclic heterocyclyl, 6-11 membered saturated monospiroheterocyclyl, and 7-10 membered saturated bridged heterocyclyl, each of which has 1-3 heteroatoms independently selected from nitrogen, oxygen, and sulfur and is optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, and -NR^{8d}R^{8e}; or
preferably, R⁷ is selected from: 4-6 membered saturated monocyclic heterocyclyl and 7-10 membered saturated bridged heterocyclyl, wherein the monocyclic heterocyclyl and bridged heterocyclyl have 1 nitrogen heteroatom and optionally 1-2 additional heteroatoms independently selected from nitrogen, oxygen, and sulfur and are optionally substituted with one or more substituents independently selected from halogen, CN, OH, NO₂, C₁₋₄ alkyl, C₁₋₄ haloalkyl, C₁₋₄ alkoxy, and -NR^{8d}R^{8e};
wherein, preferably, R^{8d} and R^{8e} are each independently selected from hydrogen and C₁₋₆ alkyl; or, preferably, R^{8d} and R^{8e} are each independently selected from hydrogen and C₁₋₄ alkyl;
or
preferably, R⁷ is selected from: 4-6 membered saturated monocyclic heterocyclyl (e.g., azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, or thiomorpholinyl) and 7-10 membered saturated bridged heterocyclyl, wherein the monocyclic heterocyclyl and bridged heterocyclyl have 1 nitrogen heteroatom and optionally 1-2 additional heteroatoms independently selected from nitrogen, oxygen, and sulfur and are optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄)alkyl, and N(C₁₋₄ alkyl)₂; or
preferably, R⁷ is selected from: 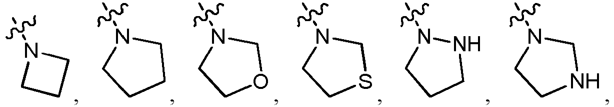 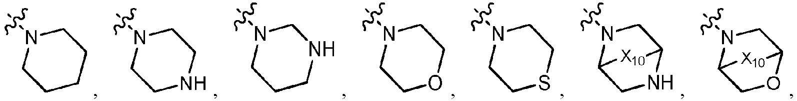 and 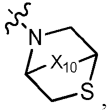 each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂., wherein X₁₀ is CH₂, (CH₂)₂, or (CH₂)₃; or
preferably, R⁷ is selected from: 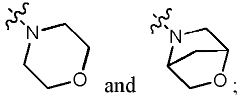
preferably, R⁴ is selected from: hydrogen, CN, 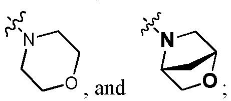
provided that when R¹ is hydrogen or deuterium, R⁴ is not hydrogen.

12. The compound according to any one of claims 1 to 11, wherein:
R and R⁵ are each independently selected from hydrogen;
and/or
R² is selected from: C₃₋₆ cycloalkyl, 4-6 membered saturated or partially unsaturated heterocyclyl having 1-3 heteroatoms independently selected from nitrogen, oxygen, and sulfur, C₆₋₁₀ aryl, and 5-6 membered heteroaryl having 1-3 heteroatoms independently selected from nitrogen, oxygen, and sulfur, wherein the cycloalkyl, heterocyclyl, aryl, and heteroaryl are optionally substituted with 1, 2, or more substituents independently selected from halogen, CN, OH, SH, NO₂, C₁₋₆ alkyl, C₁₋₆ haloalkyl, C₁₋₆ alkoxy, C₁₋₆ alkylthio, -NR^{2d}R^{2e}, -C(O)OR^{2f}, and -C(O)NR^{2d}R^{2e}, and in the case where two substituents are attached to the same ring carbon atom of the cycloalkyl or heterocyclyl, the two substituents, together with the ring carbon atom to which they are attached, optionally form optionally substituted C₃₋₆ cycloalkyl or optionally substituted 4-6 membered heterocycloalkyl having 1-3 heteroatoms independently selected from nitrogen, oxygen, and sulfur, wherein preferably, R^{2d} and R^{2e} are each independently selected from hydrogen, C₁₋₆ alkyl, and C₁₋₆ haloalkyl, and/or R^{2f} is selected from hydrogen and C₁₋₆ alkyl; or
R² is selected from: C₃₋₆ cycloalkyl, 4-6 membered heterocycloalkyl having 1 nitrogen heteroatom and optionally 1-2 additional heteroatoms independently selected from nitrogen, oxygen, and sulfur, phenyl, and 5-6 membered heteroaryl having 1 nitrogen heteroatom and optionally 1-2 additional heteroatoms independently selected from nitrogen, oxygen, and sulfur, wherein the cycloalkyl, heterocycloalkyl, phenyl, and heteroaryl are optionally substituted with 1, 2, or more substituents independently selected from halogen, CN, OH, NH₂, NO₂, C₁₋₄ alkyl, -C(O)OH, -C(O)O-C₁₋₄ alkyl, -C(O)NH₂, -C(O)NH(C₁₋₄ alkyl), and - C(O)NH(C₁₋₄ alkyl)₂, and in the case where two substituents are attached to the same ring carbon atom of the cycloalkyl or heterocycloalkyl, the two substituents, together with the ring carbon atom to which they are attached, optionally form optionally substituted C₃₋₆ cycloalkyl; or
R² is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, thiomorpholinyl, phenyl, pyrazolyl, oxazolyl, isoxazolyl, thiazolyl, isothiazolyl, oxadiazolyl, thiadiazolyl, pyridinyl, pyrazinyl, pyridazinyl, and pyrimidinyl, each of which is optionally substituted with 1, 2, or more substituents independently selected from F, Cl, Br, CN, OH, NH₂, NO₂, methyl, ethyl, isopropyl, tert-butyl, C(O)OH, -C(O)OCH₃, -C(O)OCH₂CH₃, -C(O)NH₂, -C(O)NHCH₃, and - C(O)N(CH₃)₂, and in the case where two substituents are attached to the same ring carbon atom of the cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, morpholinyl, or thiomorpholinyl, the two substituents, together with the ring carbon atom to which they are attached, optionally form optionally substituted C₃₋₆ cycloalkyl; or
R² is selected from: cyclohexyl, pyrrolidinyl, piperidinyl, phenyl, and pyridinyl, each of which is optionally substituted with 1 or 2 substituents independently selected from F, Cl, Br, OH, NH₂, and methyl, and in the case where two substituents are attached to the same ring carbon atom of the pyrrolidinyl or piperidinyl, the two substituents, together with the ring carbon atom to which they are attached, optionally form optionally substituted C₃₋₆ cycloalkyl; or
R² is selected from: 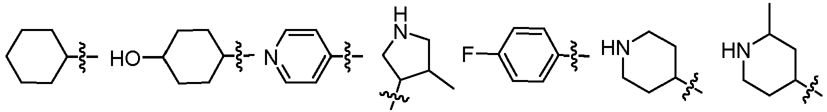 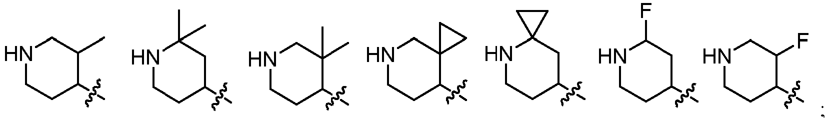 or
R² is selected from: 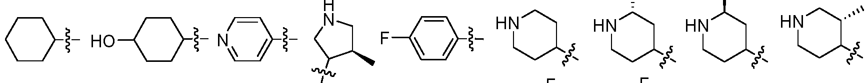 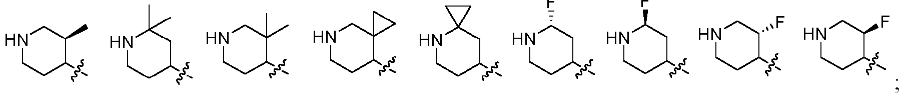 and/or
R³ is selected from: halogen, CN, NO₂, C₁₋₆ alkyl, -OR^{3f}, -SR^{3f}, -NR^{3d}R^{3e}, -S(O)₂-NR^{3d}R^{3e}, -S(O)-NR^{3d}R^{3e}, -CF(R^{3f})₂, -CF₂(R^{3f}), -CF₃, -CCl(R^{3f})₂, -CCl₂(R^{3f}), -CCl₃, -C(O)OR^{3f}, and -C(O)NR^{3d}R^{3e}; or
R³ is selected from: halogen, CN, C₁₋₄ alkyl, -OR^{3f}, -NR^{3d}R^{3e}, -CF(R^{3f})₂, -CF₂(R^{3f}), -CF₃, -CCl(R^{3f})₂,-CCl₂(R^{3f}), -CCl₃, -C(O)OR^{3f}, and -C(O)NR^{3d}R^{3e}; or
R³ is selected from: halogen, CN, C₁₋₄ alkyl, -OR^{3f}, -NR^{3d}R^{3e}, -CF(R^{3f})₂, -CF₂(R^{3f}), -CF₃, -CCl(R^{3f})₂,-CCl₂(R^{3f}), -CCl₃, and -C(O)NR^{3d}R^{3e}.
wherein:
preferably, R^{3d} and R^{3e} are each independently selected from hydrogen, deuterium, R¹¹, C₁₋₄ alkyl, and C₁₋₄ haloalkyl, wherein R¹¹ is preferably selected from: C₃₋₆ cycloalkyl, 4-6 membered saturated or partially unsaturated heterocyclyl having 1-3 heteroatoms independently selected from nitrogen, oxygen, and sulfur, C₆₋₁₀ aryl, and 5-6 membered heteroaryl having 1-3 heteroatoms independently selected from nitrogen, oxygen, and sulfur, wherein the cycloalkyl, heterocyclyl, aryl, and heteroaryl are optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, and -NR^{8d}R^{8e}, wherein preferably, R^{8d} and R^{8e} are each independently selected from hydrogen, C₁₋₄ alkyl and C₁₋₄ haloalkyl; or
preferably, R^{3d} and R^{3e} are each independently selected from hydrogen, methyl, and ethyl;
and/or
wherein:
preferably, R^{3f} is selected from hydrogen and C₁₋₆ alkyl; or
preferably, R^{3f} is selected from hydrogen and C₁₋₄ alkyl;
or
wherein:
preferably, R^{3d} and R^{3e}, together with the nitrogen atom to which they are both attached, form R¹⁰, wherein R¹⁰ is preferably selected from: 3-6 membered heterocycloalkyl having 1 nitrogen heteroatom and optionally 1-2 additional heteroatoms independently selected from nitrogen, oxygen, and sulfur, and 5-6 membered heteroaryl having 1 nitrogen heteroatom and optionally 1-2 additional heteroatoms independently selected from nitrogen, oxygen, and sulfur, wherein the heterocycloalkyl and heteroaryl are optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂;
or
R³ is selected from: halogen, OH, SH, NH₂, CN, C₁₋₄ alkyl, -OC₁₋₄ alkyl, -NH(C₁₋₄ alkyl), -N(C₁₋₄ alkyl)₂, -CHF₂, -CH₂F, -CF₃, -CH₂Cl, -CHCl₂, -CCl₃, -C(O)NH₂, -C(O)NH(C₁₋₄ alkyl), and -C(O)N(C₁₋₄ alkyl)₂;
or
R³ is selected from: F, Cl, Br, OH, NH₂, CN, methyl, ethyl, propyl, isopropyl, n-butyl, isobutyl, sec-butyl, tert-butyl, methoxy, ethoxy, -N(CH₃)₂, -CHF₂, -CHCl₂, and -C(O)NH₂;
and/or
R⁶ is selected from: halogen, CN, NO₂, C₁₋₄ alkyl, -OR^{3f}, -SR^{3f}, -NR^{3d}R^{3e}, -CF(R^{3f})₂, -CF₂(R^{3f}), -CF₃, - CCl(R^{3f})₂, -CCl₂(R^{3f}), and -CCl₃, wherein R^{3f} is preferably selected from hydrogen and C₁₋₆ alkyl, or R^{3f} is preferably selected from hydrogen and C₁₋₄ alkyl; or
R⁶ is selected from: F, Cl, Br, OH, NH₂, CN, NO₂, methyl, ethyl, propyl, isopropyl, n-butyl, isobutyl, sec-butyl, tert-butyl, methoxy, ethoxy, -CH₂F, -CHF₂, -CF₃, -CH₂Cl, -CHCl₂, and -CCl₃;
and/or
m is 0.

13. The compound according to any one of claims 1 to 12, wherein:
at least 2 of X₁, X₂, X₃, X₄, and X₅ are N, provided that X₄ and X₅ are not both N at the same time; or
3 of X₁, X₂, X₃, X₄, and X₅ are N, provided that X₄ and X₅ are not both N at the same time; or
4 of X₁, X₂, X₃, X₄, and X₅ are N, provided that X₄ and X₅ are not both N at the same time;
wherein preferably, X₁ is N;
and/or
at least 2 of X₆, X₇, X₈, and X₉ are N; or
at least 3 of X₆, X₇, X₈, and X₉ are N;
at least 4 of X₆, X₇, X₈, and X₉ are N;
wherein preferably, X₈ and X₉ are each N;
preferably, 2 or 3 of X₁, X₂, X₃, X₄, and X₅ are N, and X₁ is N, provided that X₄ and X₅ are not both N at the same time; and
3 or 4 of X₆, X₇, X₈, and X₉ are N, and X₈ and X₉ are each N.

14. The compound according to any one of claims 1 to 13, wherein:
X₁ and X₂ are each N, X₃ is CH, X₄ and X₅ are each C, X₆, X₈, and X₉ are each N, and X₇ is CH;
and/or
The compound has a structure of formula (I-i) or formula (I-ii): 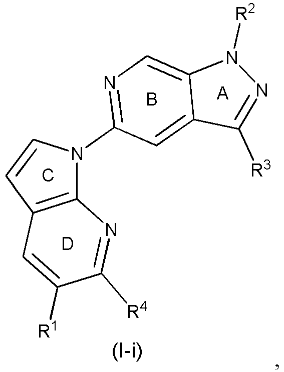
wherein: R¹, R², R³, and R⁴ are each as defined in any one of claims 1 to 13, provided that R¹ is not hydrogen or deuterium; 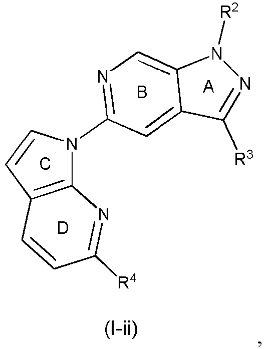
wherein: R², R³, and R⁴ are each as defined in any one of claims 1 to 13, provided that R⁴ is not hydrogen or deuterium;
wherein:
preferably, R¹ is selected from: halogen, CN, NO₂, -OR^{1f}, -(CR^{1a}R^{1b})ₚ-OR^{1f}, -(CR^{1a}R^{1b})ₚ-C(O)OR^{1f}, - (CR^{1a}R^{1b})ₚ-NR^{1d}R^{1e}, -C(O)OR^{1f}, or -C(O)NR^{1d}R^{1e}; or
R¹ is selected from: halogen, CN, -(CR^{1a}R^{1b})ₚ-OR^{1f}, -(CR^{1a}R^{1b})ₚ-C(O)OR^{1f}, -C(O)OR^{1f}, or - C(O)NR^{1d}R^{1e};
wherein:
preferably, p is 1;
and/or
preferably, R^{1a} and R^{1b} are each independently selected from hydrogen and C₁₋₄ alkyl, preferably each independently hydrogen; or R^{1a} and R^{1b}, together with the carbon atom to which they are both attached, form R⁹;
wherein:
preferably, R⁹ is selected from: C₃₋₆ cycloalkyl, wherein the cycloalkyl is optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl), and N(C₁₋₄ alkyl)₂; or
R⁹ is selected from: cyclopropyl, cyclobutyl, cyclopentyl, and cyclohexyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
preferably, R⁹ is cyclopropyl;
and/or
wherein:
preferably, R^{1d} and R^{1e} are each independently selected from hydrogen and R¹¹; or R^{1d} and R^{1e}, together with the nitrogen atom to which they are both attached, form R¹⁰;
wherein:
preferably, R¹¹ is selected from: C₃₋₆ cycloalkyl, and 3-6 membered heterocycloalkyl having 1-3 heteroatoms independently selected from nitrogen, oxygen, and sulfur, wherein the cycloalkyl and heterocycloalkyl are optionally substituted with one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂; or
R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, and NH₂; or
R¹¹ is selected from: cyclopropyl, cyclobutyl, cyclopentyl, cyclohexyl, azetidinyl, pyrrolidinyl, piperidinyl, piperazinyl, and morpholinyl, each of which is optionally substituted with one or more substituents independently selected from F, Cl, Br, CN, OH NO₂, and NH₂; or
R¹¹ is selected from cyclopropyl and 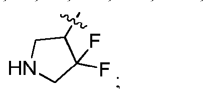 and/or
preferably, R¹⁰ is selected from: 3-6 membered heterocycloalkyl having 1 nitrogen heteroatom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
preferably, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl_{3,} methoxy, ethoxy, and NH₂; or
preferably, R¹⁰ is selected from: 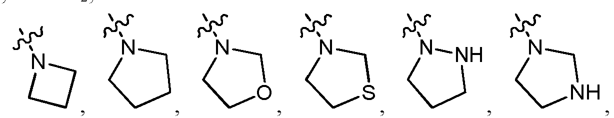 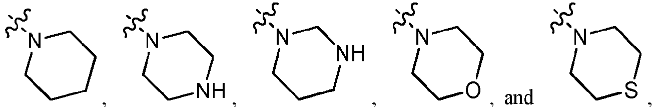 and preferably 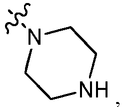 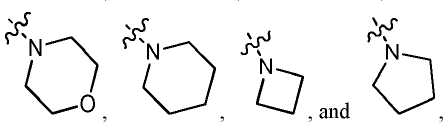 each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂;
and/or
wherein:
R^{1f} is selected from hydrogen and C₁₋₄ alkyl, preferably hydrogen;
or
R¹ is selected from: F, Cl, Br, CN, OH, NH₂, C(O)OH, -C(O)NH₂, 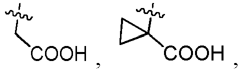 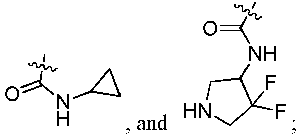
preferably, R¹ is selected from CN and -C(O)NH₂;
and/or
wherein:
preferably, R² is selected from: C₃₋₆ cycloalkyl, 4-6 membered heterocycloalkyl having 1 nitrogen heteroatom and optionally 1-2 additional heteroatoms independently selected from nitrogen, oxygen, and sulfur, phenyl, and 5-6 membered heteroaryl having 1 nitrogen heteroatom and optionally 1-2 additional heteroatoms independently selected from nitrogen, oxygen, and sulfur, wherein the cycloalkyl, heterocycloalkyl, phenyl, and heteroaryl are optionally substituted with 1, 2, or more substituents independently selected from halogen, CN, OH, NH₂, NO₂, C₁₋₆ alkyl, and C₁₋₆ alkoxy, and in the case where two substituents are attached to the same ring carbon atom of the cycloalkyl or heterocycloalkyl, the two substituents, together with the ring carbon atom to which they are attached, optionally form optionally substituted C₃₋₆ cycloalkyl; or
R² is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, thiomorpholinyl, phenyl, pyrazolyl, oxazolyl, isoxazolyl, thiazolyl, isothiazolyl, oxadiazolyl, thiadiazolyl, pyridinyl, pyrazinyl, pyridazinyl, and pyrimidinyl, each of which is optionally substituted with 1 or 2 substituents independently selected from F, Cl, Br, CN, OH, NH₂, NO₂, and C₁-C₄ alkyl, and in the case where two substituents are attached to the same ring carbon atom of the cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, or thiomorpholinyl, the two substituents, together with the ring carbon atom to which they are attached, optionally form optionally substituted C₃₋₆ cycloalkyl; or
R² is selected from: cyclopentyl, cyclohexyl, pyrrolidinyl, piperidinyl, phenyl, and pyridinyl, each of which is optionally substituted with 1 or 2 substituents independently selected from F, Cl, Br, CN, OH, NH₂, NO₂, methyl, ethyl, and isopropyl, and in the case where two substituents are attached to the same ring carbon atom of the pyrrolidinyl or piperidinyl, the two substituents, together with the ring carbon atom to which they are attached, optionally form optionally substituted C₃₋₆ cycloalkyl; or
R² is selected from: 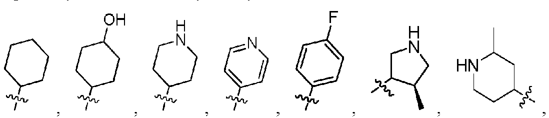 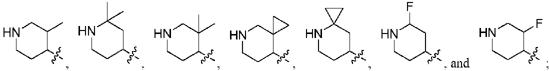 or
R² is selected from: 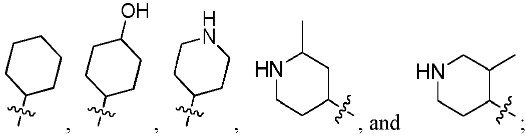 or
R² is 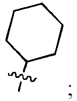 and/or
wherein:
preferably, R³ is selected from: halogen, CN, NO₂, C₁₋₆ alkyl, OH, NH₂, -CH₂F, -CHF₂, -CF₃, -CH₂Cl, - CHCl₂, and -CCl₃; or
R³ is -CHF₂;
and/or
wherein:
preferably, R⁴ is selected from: hydrogen, R⁷, halogen, CN, NO₂, OH, and NH₂; or
R⁴ is selected from: hydrogen, R⁷, F, Cl, Br, CN, NO₂, OH, and NH₂;
wherein:
preferably, R⁷ is selected from: 4-6 membered saturated monocyclic heterocyclyl, wherein the heterocyclyl has 1 nitrogen heteroatom and optionally 1-2 additional heteroatoms independently selected from nitrogen, oxygen, and sulfur and is optionally substituted with one or more substituents independently selected from halogen, CN, OH, SH, NO₂, C₁₋₆ alkyl, C₁₋₆ haloalkyl, C₁₋₆ alkoxy, C₁₋₆ alkylthio, NH₂, NH(C₁₋₄ alkyl), and N(C₁₋₄ alkyl)₂; or
R⁷ is selected from: 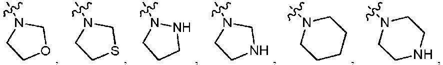 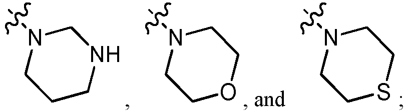
preferably, R⁴ is selected from: hydrogen, CN, and 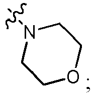
provided that when R¹ is hydrogen, R⁴ is not hydrogen.

15. The compound according to any one of claims 1 to 13, wherein:
X₁ and X₅ are each N, X₂ and X₄ are C, X₃ is CH, X₆, X₈, and X₉ are each N, and X₇ is CH; and/or
the compound has a structure of formula (I-iii): 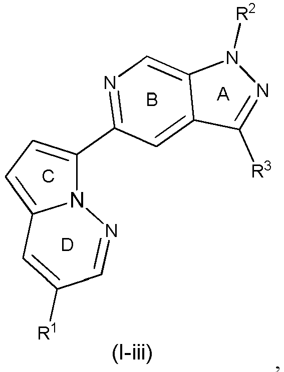
wherein: R¹, R², and R³ are each as defined in any one of claims 1 to 13, provided that R¹ is not hydrogen or deuterium;
preferably, R¹ is selected from: R⁷, halogen, NO₂, -OR^{1f}, -NR^{1d}R^{1e}, -(CR^{1a}R^{1b})ₚ-OR^{1f}, -(CR^{1a}R^{1b})ₚ-C(O)OR^{1f}, -(CR^{1a}R^{1b})ₚ-NR^{1d}R^{1e}, -C(O)OR^{1f}, or -C(O)NR^{1d}R^{1e}; or
R¹ is selected from: R⁷, halogen, -NR^{1d}R^{1e}, -(CR^{1a}R^{1b})ₚ-OR^{1f}, -(CR^{1a}R^{1b})ₚ-C(O)OR^{1f}, -C(O)OR^{1f}, or - C(O)NR^{1d}R^{1e};
wherein:
preferably, p is 1;
and/or
wherein:
preferably, R⁷ is selected from: 4-6 membered heterocycloalkyl having 1 N atom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
R⁷ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂; or
R⁷ is piperidinyl;
and/or
wherein:
preferably, R^{1a} and R^{1b} are each independently selected from hydrogen and C₁₋₄ alkyl, preferably each independently hydrogen; or R^{1a} and R^{1b}, together with the carbon atom to which they are both attached, form R⁹;
wherein:
preferably, R⁹ is selected from: C₃₋₆ cycloalkyl, wherein the cycloalkyl is optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl), and N(C₁₋₄ alkyl)₂; or
R⁹ is selected from: cyclopropyl, cyclobutyl, cyclopentyl, and cyclohexyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂;
and/or
wherein:
preferably, R^{1d} and R^{1e} are each independently selected from hydrogen, C₁₋₄ alkyl, and R¹¹; or R^{1d} and R^{1e}, together with the nitrogen atom to which they are both attached, form R¹⁰;
wherein:
preferably, R¹¹ is selected from: C₃₋₆ cycloalkyl, and 3-6 membered heterocycloalkyl having 1-3 heteroatoms independently selected from nitrogen, oxygen, and sulfur, wherein the cycloalkyl and heterocycloalkyl are optionally substituted with one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂; or
R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, and NH₂;
and/or
preferably, R¹⁰ is selected from: 3-6 membered heterocycloalkyl having 1 nitrogen heteroatom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
preferably, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂;
and/or
wherein:
R^{1f} is selected from hydrogen and C₁₋₄ alkyl, preferably hydrogen;
preferably, R¹ is selected from: F, Cl, Br, OH, NH₂, -NHCH₃, C(O)OH, -C(O)NH₂, -CH₂OH, 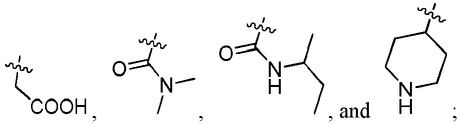
more preferably, R¹ is selected from -CN and -C(O)NH₂;
and/or
wherein:
preferably, R² is selected from: C₃₋₆ cycloalkyl, 4-6 membered heterocycloalkyl having 1 nitrogen heteroatom and optionally 1-2 additional heteroatoms independently selected from nitrogen, oxygen, and sulfur, phenyl, and 5-6 membered heteroaryl having 1 nitrogen heteroatom and optionally 1-2 additional heteroatoms independently selected from nitrogen, oxygen, and sulfur, wherein the cycloalkyl, heterocycloalkyl, phenyl, and heteroaryl are optionally substituted with 1, 2, or more substituents independently selected from halogen, CN, OH, NH₂, NO₂, C₁₋₆ alkyl, and C₁₋₆ alkoxy, and in the case where two substituents are attached to the same ring carbon atom of the cycloalkyl or heterocycloalkyl, the two substituents, together with the ring carbon atom to which they are attached, optionally form optionally substituted C₃₋₆ cycloalkyl; or
R² is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, thiomorpholinyl, phenyl, pyrazolyl, oxazolyl, isoxazolyl, thiazolyl, isothiazolyl, oxadiazolyl, thiadiazolyl, pyridinyl, pyrazinyl, pyridazinyl, and pyrimidinyl, each of which is optionally substituted with 1 or 2 substituents independently selected from F, Cl, Br, CN, OH, NH₂, NO₂, and C₁-C₄ alkyl, and in the case where two substituents are attached to the same ring carbon atom of the cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, or thiomorpholinyl, the two substituents, together with the ring carbon atom to which they are attached, optionally form optionally substituted C₃₋₆ cycloalkyl; or
R² is selected from: cyclopentyl, cyclohexyl, pyrrolidinyl, piperidinyl, phenyl, and pyridinyl, each of which is optionally substituted with 1 or 2 substituents independently selected from F, Cl, Br, CN, OH, NH₂, NO₂, methyl, ethyl, and isopropyl, and in the case where two substituents are attached to the same ring carbon atom of the pyrrolidinyl or piperidinyl, the two substituents, together with the ring carbon atom to which they are attached optionally form optionally substituted C₃₋₆ cycloalkyl; or 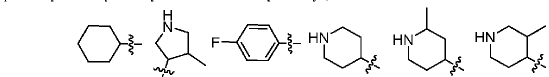
R² is selected from: 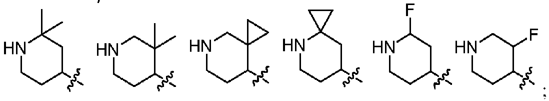 or
R² is selected from: 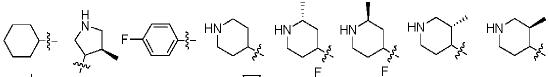 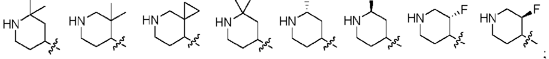 and/or
wherein:
preferably, R³ is selected from: halogen, CN, C₁₋₄ alkyl, -OR^{3f}, -SR^{3f}, -NR^{3d}R^{3e}, -CF(R^{3f})₂, -CF₂(R^{3f}), - CF₃, -CCl(R^{3f})₂, -CCl₂(R^{3f}), -CCl₃, and -C(O)NR^{3d}R^{3e};
wherein: preferably, R^{3d} and R^{3e} are each independently selected from hydrogen and C₁₋₄ alkyl, preferably hydrogen and methyl;
and/or
preferably, R^{3f} is selected from hydrogen and C₁₋₄ alkyl, preferably hydrogen and methyl;
preferably, R³ is selected from: halogen, OH, SH, NH₂, CN, C₁₋₄ alkyl, -OC₁₋₄ alkyl, -NH(C₁₋₄ alkyl), - N(C₁₋₄ alkyl)₂, -CHF₂, -CH₂F, -CF₃, -CH₂Cl, -CHCl₂, -CCl₃, -C(O)NH₂, -C(O)NH(C₁₋₄ alkyl), and - C(O)N(C₁₋₄ alkyl)₂; or
R³ is selected from: F, OH, NH₂, CN, methyl, ethyl, isopropyl, tert-butyl, methoxy, -N(CH₃)₂, -CHF₂, and -C(O)NH₂; or
R³ is selected from: F, CN, methyl, isopropyl, methoxy, -N(CH₃)₂, -CHF₂, and -C(O)NH₂.

16. The compound according to any one of claims 1 to 13, wherein:
X₁, X₃, and X₄ are each N, X₂ and X₅ are C, X₆, X₈, and X₉ are each N, and X₇ is CH;
and/or
the compound has a structure of formula (I-iv): 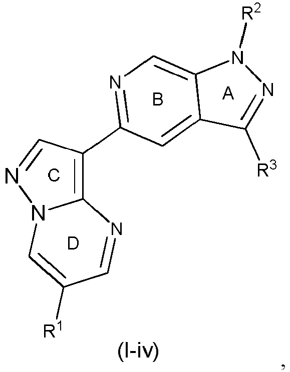
wherein: R¹, R², and R³ are each as defined in any one of claims 1 to 13, provided that R¹ is not hydrogen or deuterium;
preferably, R¹ is selected from: halogen, CN, NO₂, -OR^{1f}, -(CR^{1a}R^{1b})ₚ-OR^{1f}, -(CR^{1a}R^{1b})ₚ-C(O)OR^{1f}, - (CR^{1a}R^{1b})ₚ-NR^{1d}R^{1e}, or -C(O)OR^{1f}; or
R¹ is selected from: halogen, CN, -(CR^{1a}R^{1b})ₚ-OR^{1f}, -(CR^{1a}R^{1b})ₚ-C(O)OR^{1f}, or -C(O)OR^{1f};
wherein:
preferably, p is 1;
and/or
preferably, R^{1a} and R^{1b} are each independently selected from hydrogen and C₁₋₄ alkyl, preferably each independently hydrogen; or R^{1a} and R^{1b}, together with the carbon atom to which they are both attached, form R⁹;
wherein:
preferably, R⁹ is selected from: C₃₋₆ cycloalkyl, wherein the cycloalkyl is optionally substituted with one or more substituents independently selected from halogen, CN, OH, SH, NO₂, C₁₋₄ alkyl, C₁₋₄ haloalkyl, C₁₋₄ alkoxy, C₁₋₄ alkylthio, NH₂, NH(C₁₋₄ alkyl), and N(C₁₋₄ alkyl)₂; or
R⁹ is selected from: cyclopropyl, cyclobutyl, cyclopentyl, and cyclohexyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂;
and/or
wherein:
preferably, R^{1d} and R^{1e} are each independently selected from hydrogen and R¹¹; or R^{1d} and R^{1e}, together with the nitrogen atom to which they are both attached, form R¹⁰;
wherein:
preferably, R¹¹ is selected from: C₃₋₆ cycloalkyl, and 3-6 membered heterocycloalkyl having 1-3 heteroatoms independently selected from nitrogen, oxygen, and sulfur, wherein the cycloalkyl and heterocycloalkyl are optionally substituted with one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂; or
R¹¹ is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, and NH₂;
and/or
preferably, R¹⁰ is selected from: 3-6 membered heterocycloalkyl having 1 nitrogen heteroatom and optionally 1-2 additional heteroatoms independently selected from nitrogen, oxygen, and sulfur, wherein the heterocycloalkyl is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, NH₂, NH(C₁₋₄ alkyl), and N(C₁₋₄ alkyl)₂; or
preferably, R¹⁰ is selected from: azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, and thiomorpholinyl, each of which is optionally substituted with one or more substituents independently selected from F, Cl, Br, CN, OH, NO₂, methyl, ethyl, propyl, isopropyl, n-butyl, isobutyl, sec-butyl, tert-butyl, CHF₂, CH₂F, CF₃, CHCl₂, CH₂Cl, CCl₃, methoxy, ethoxy, and NH₂;
and/or
wherein:
R^{1f} is selected from hydrogen and C₁₋₄ alkyl, preferably hydrogen;
or
R¹ is selected from: F, Cl, Br, CN, OH, NH₂, C(O)OH, 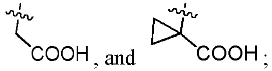 or
R' is CN;
and/or
wherein:
preferably, R² is selected from: C₃₋₆ cycloalkyl, 4-6 membered heterocycloalkyl having 1 nitrogen heteroatom and optionally 1-2 additional heteroatoms independently selected from nitrogen, oxygen, and sulfur, phenyl, and 5-6 membered heteroaryl having 1 nitrogen heteroatom and optionally 1-2 additional heteroatoms independently selected from nitrogen, oxygen, and sulfur, wherein the cycloalkyl, heterocycloalkyl, phenyl, and heteroaryl are optionally substituted with 1, 2, or more substituents independently selected from halogen, CN, OH, NH₂, NO₂, C₁₋₆ alkyl, and C₁₋₆ alkoxy, and in the case where two substituents are attached to the same ring carbon atom of the cycloalkyl or heterocycloalkyl, the two substituents, together with the ring carbon atom to which they are attached, optionally form optionally substituted C₃₋₆ cycloalkyl; or
R² is selected from: cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, thiomorpholinyl, phenyl, pyrazolyl, oxazolyl, isoxazolyl, thiazolyl, isothiazolyl, oxadiazolyl, thiadiazolyl, pyridinyl, pyrazinyl, pyridazinyl, and pyrimidinyl, each of which is optionally substituted with 1 or 2 substituents independently selected from F, Cl, Br, CN, OH, NH₂, NO₂, and C₁-C₄ alkyl, and in the case where two substituents are attached to the same ring carbon atom of the cyclopropyl, cyclobutyl, cyclopentyl, cyclohexyl, azetidinyl, pyrrolidinyl, oxazolidinyl, thiazolidinyl, pyrazolidinyl, imidazolidinyl, piperidinyl, piperazinyl, hexahydropyrimidinyl, triazinyl, morpholinyl, or thiomorpholinyl, the two substituents, together with the ring carbon atom to which they are attached, optionally form optionally substituted C₃₋₆ cycloalkyl; or
R² is selected from: 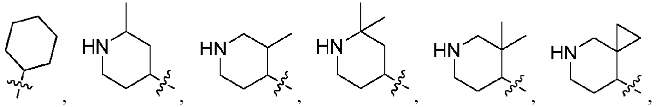 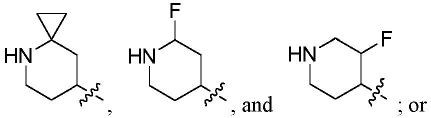
R² is selected from: 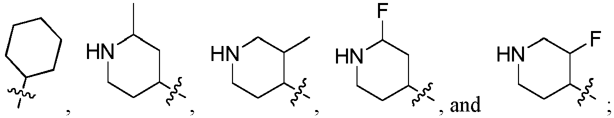 or
R² is 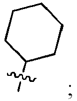 and/or
wherein:
preferably, R³ is selected from: halogen, CN, NO₂, C₁₋₆ alkyl, OH, NH₂, -CH₂F, -CHF₂, -CF₃, -CH₂Cl, - CHCl₂, and -CCl₃; or
R³ is -CHF₂.

17. The compound according to any one of claims 1 to 13, wherein:
X₁ and X₅ are each N, X₂ and X₄ are C, X₃ is CH, X₆ is CH, and X₇, X₈, and X₉ are each N;
and/or
the compound has a structure of formula (I-v): 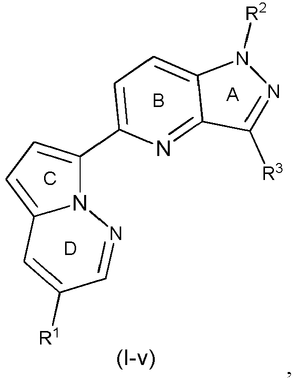
wherein: R¹, R², and R³ are each as defined in any one of claims 1 to 13, provided that R¹ is not hydrogen or deuterium;
preferably, R¹, R², and R³ are each as defined in claim 14, 15, or 16; or
R¹ is -C(O)NH₂; and/or R² is 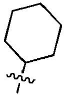 and/or R³ is -CHF₂; or
wherein:
X₁ and X₅ are each N, X₂ and X₄ are C, X₃ is CH, and X₆, X₇, X₈, and X₉ are each N;
and/or
the compound has a structure of formula (I-vi): 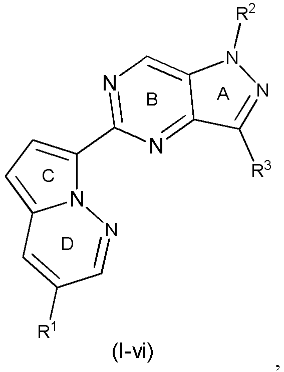
R¹, R², and R³ are each as defined in any one of claims 1 to 13, provided that R¹ is not hydrogen or deuterium;
wherein:
preferably, R¹, R², and R³ are each as defined in claim 14, 15, or 16; or
R¹ is -C(O)NH₂; and/or R² is 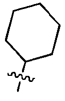 and/or R³ is -CHF₂; or
wherein:
X₁ and X₂ are each N, X₃ is CH, X₄ and X₅ are each C, and X₆, X₇, X₈, and X₉ are each N;
and/or
the compound has a structure of formula (I-vii): 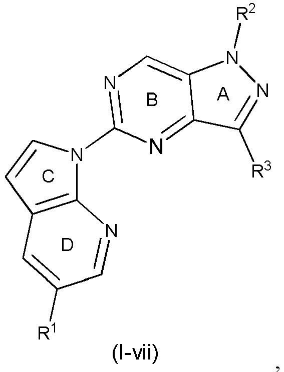
wherein: R¹, R², and R³ are each as defined in any one of claims 1 to 13, provided that R¹ is not hydrogen or deuterium;
preferably, R¹, R², and R³ are each as defined in claim 14, 15, or 16; or
R¹ is -CN; and/or R² is 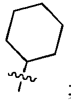 and/or R³ is -CHF₂.

18. The compound according to any one of claims 1 to 13, wherein:
X₁, X₃, and X₄ are each N, X₂ and X₅ are C, and X₆, X₇, X₈, and X₉ are each N;
and/or
the compound has a structure of formula (I-viii): 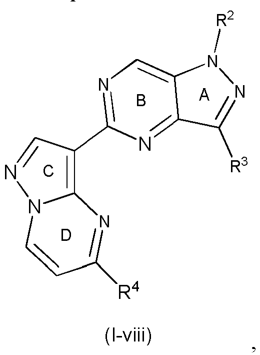
wherein: R², R³, and R⁴ are each as defined in any one of claims 1 to 13, provided that R⁴ is not hydrogen or deuterium;
preferably, R² and R³ are each as defined in claim 14, 15, or 16; or
R² is 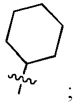 and/or R³ is -CHF₂;
and/or
preferably, R⁴ is selected from: R⁷, halogen, CN, NO₂, OH, and NH₂;
or
R⁴ is selected from: R⁷, F, Cl, Br, CN, NO₂, OH, and NH₂;
wherein:
preferably, R⁷ is selected from: 7-10 membered saturated bridged heterocyclyl, wherein the bridged heterocyclyl has 1 nitrogen heteroatom and optionally 1-2 additional heteroatoms independently selected from nitrogen, oxygen, and sulfur and is optionally substituted with one or more substituents independently selected from halogen, CN, OH, NO₂, C₁₋₄ alkyl, C₁₋₄ haloalkyl, C₁₋₄ alkoxy, NH₂, NH(C₁₋₄ alkyl), and N(C₁₋₄ alkyl)₂; or
R⁷ is selected from: 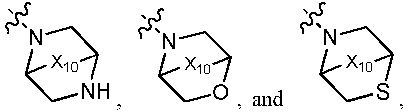 wherein X₁₀ is CH₂, (CH₂)₂, or (CH₂)₃;
preferably, R⁴ is selected from: CN and 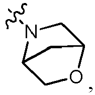 more preferably

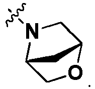

19. The compound according to claim 1, wherein the compound is selected from:

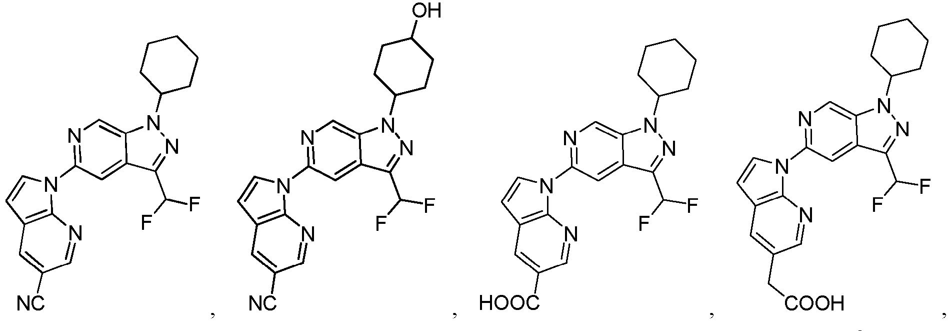

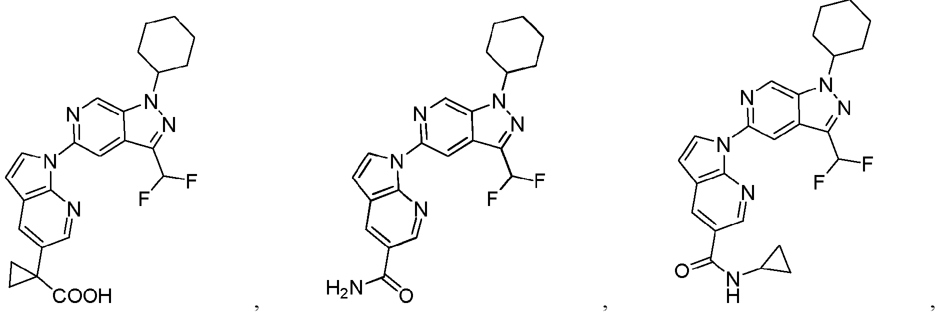

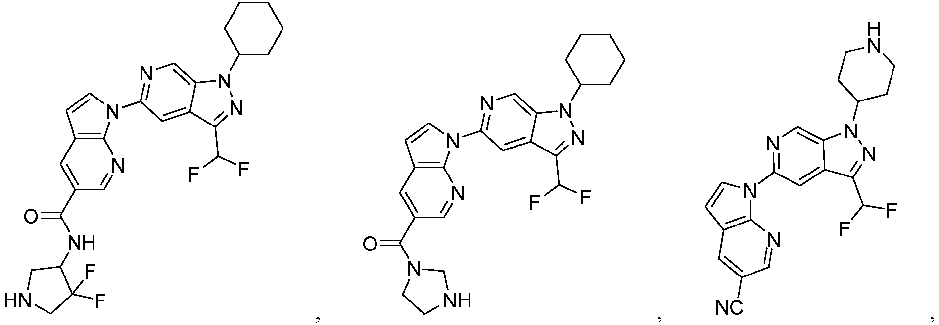

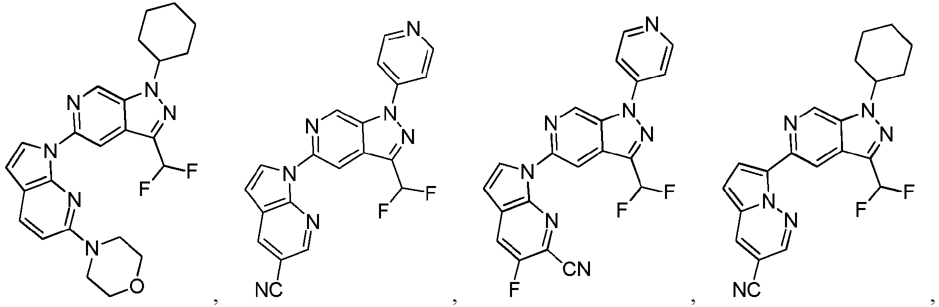

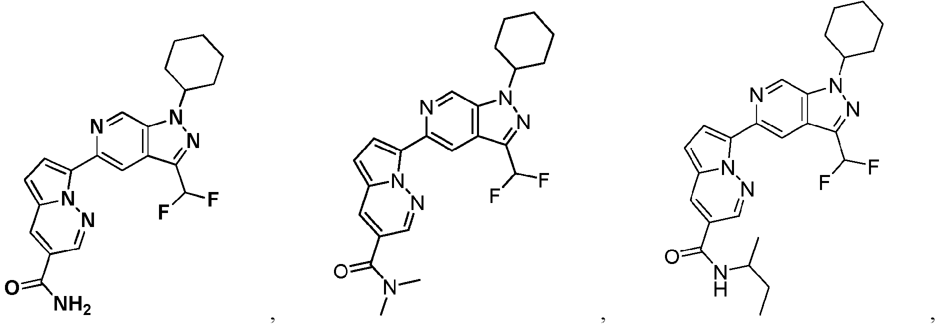

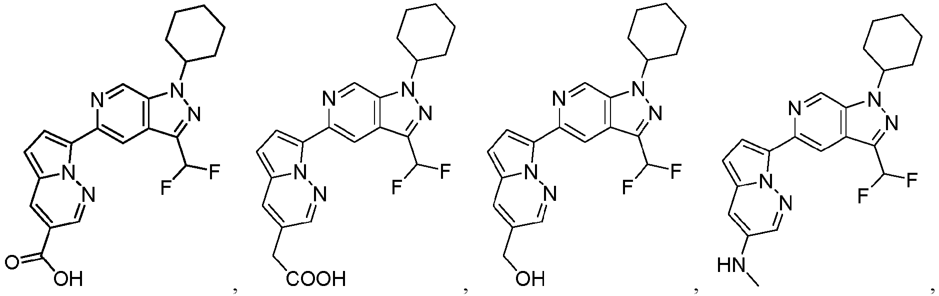

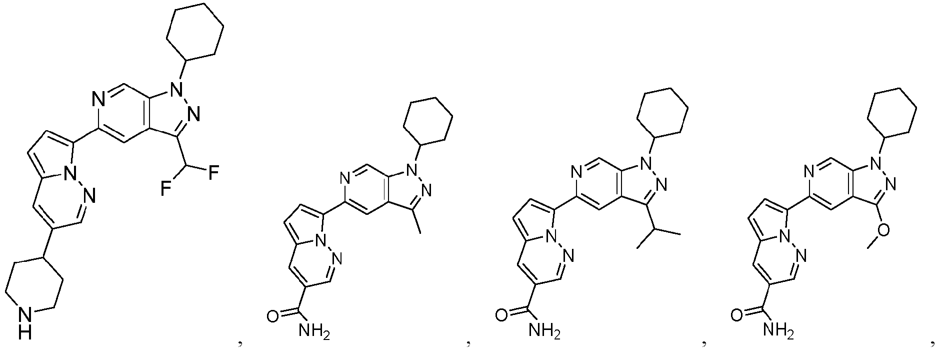

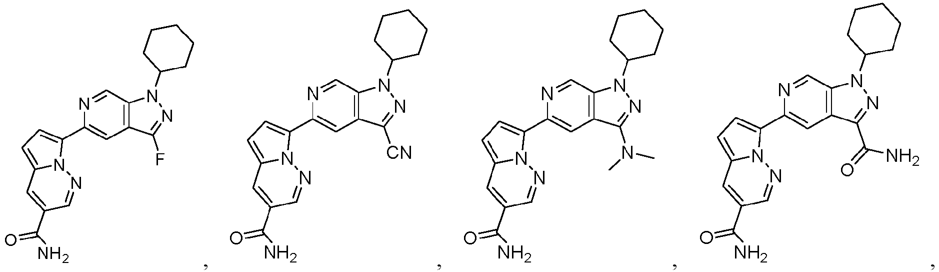

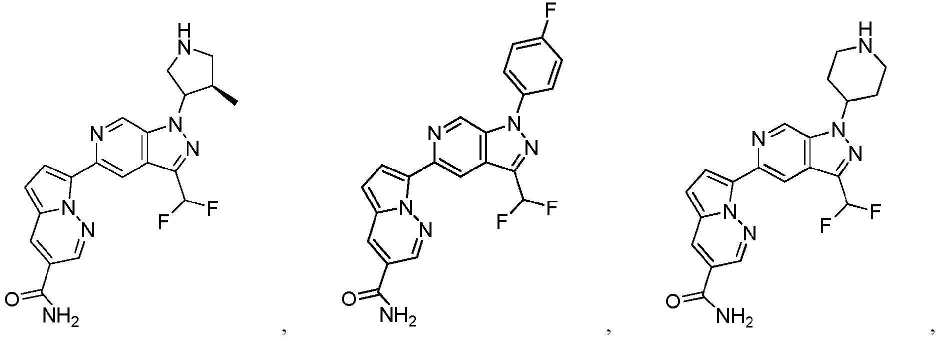

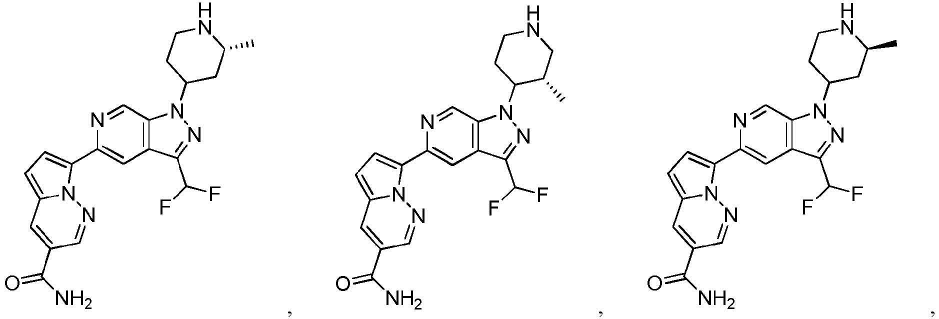

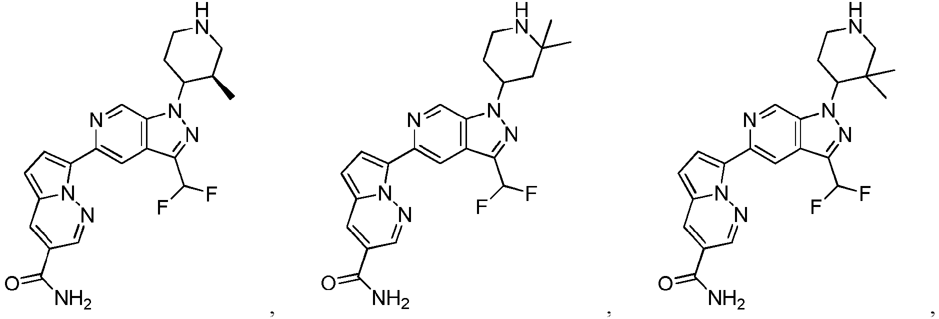

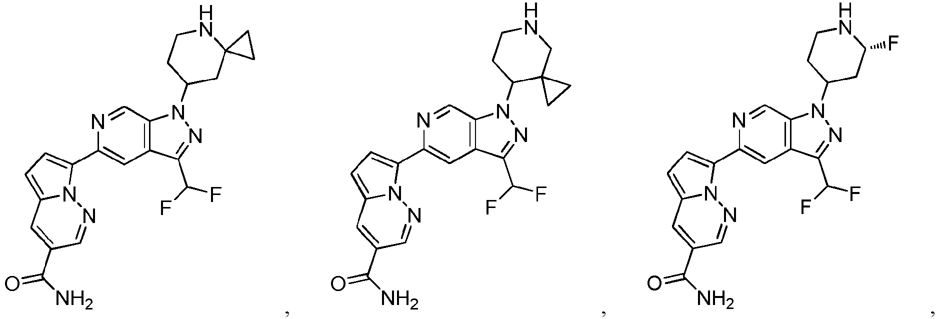

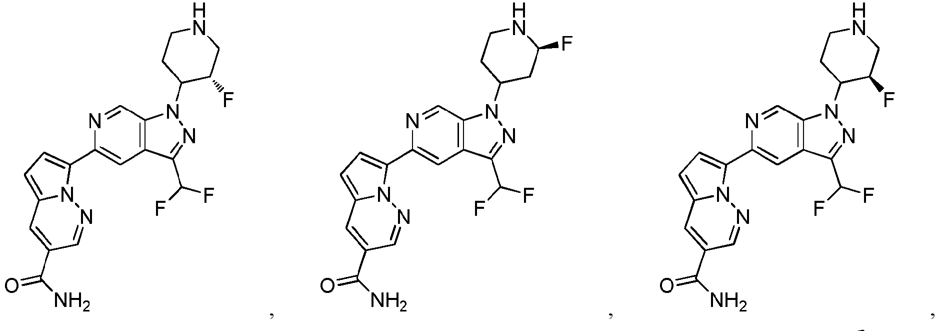

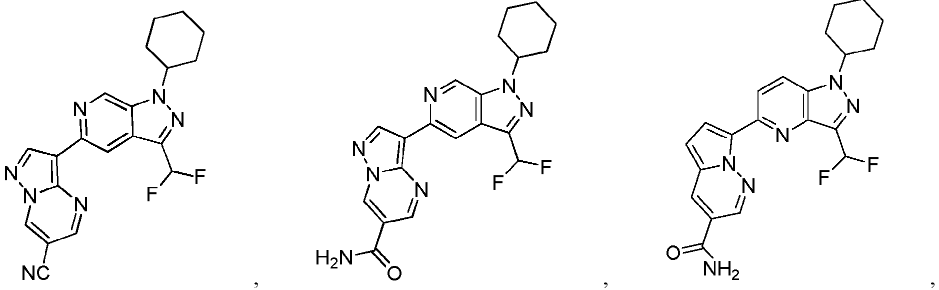

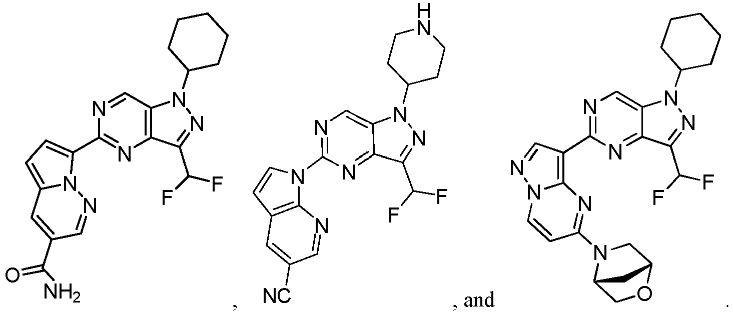

20. Use of the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof according to any one of claims 1 to 19 in the preparation of a proteolysis targeting chimera (PROTAC).

21. A proteolysis targeting chimera (PROTAC), comprising a moiety with IRAK4 protein kinase inhibitory activity, wherein the moiety is derived from the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof according to any one of claims 1 to 19.

22. A pharmaceutical composition, comprising the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof according to any one of claims 1 to 19, or the proteolysis targeting chimera according to claim 21, and a pharmaceutically acceptable excipient, carrier, or diluent.

23. Use of the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof according to any one of claims 1 to 19, or the proteolysis targeting chimera according to claim 21, or the pharmaceutical composition according to claim 22, in the preparation of a medicament for treating a disease, a disorder, or a condition associated with IRAK4 protein kinase.

24. A method for treating a disease, a disorder, or a condition associated with IRAK4 protein kinase, comprising administering to an individual in need thereof a therapeutically effective amount of the compound or the stereoisomer, the tautomer, the diastereoisomer, the racemate, the cis-trans isomer, the isotope-labeled compound (preferably the deuteride), the N-oxide, the metabolite, the ester, the prodrug, the crystalline form, the hydrate, the solvate, or the pharmaceutically acceptable salt thereof according to any one of claims 1 to 19, or the proteolysis targeting chimera according to claim 21, or the pharmaceutical composition according to claim 22.

25. The pharmaceutical composition according to claim 22, the use according to claim 23, or the method according to claim 24, wherein the disease, the disorder, or the condition associated with IRAK4 protein kinase is selected from: an autoimmune condition, an inflammatory condition, cancer, graft rejection, thromboembolism, atherosclerosis, myocardial infarction, and metabolic syndrome;
wherein:
preferably, the inflammatory condition is selected from:
osteoarthritis, gout, gouty arthritis, chronic obstructive pulmonary disease, periodic fever, atopic dermatitis, allergic eczema, lymphadenectasis, pyaemia, irritable bowel syndrome (IBD), ulcerative colitis, asthma, and allergy; and/or
preferably, the autoimmune condition is selected from: Crohn's disease, rheumatoid arthritis, systemic lupus erythematosus, lupus nephritis, cutaneous lupus, psoriasis, psoriatic arthritis, multiple sclerosis, neuropathic pain, ankylosing spondylitis, reactive arthritis, and systemic juvenile idiopathic arthritis; and/or
preferably, the graft rejection is selected from: graft-versus-host disease and allograft rejection; and/or
preferably, the cancer is selected from: brain cancer, kidney cancer, liver cancer, stomach cancer, vaginal cancer, ovarian cancer, gastric tumor, breast cancer, bladder cancer, colon cancer, prostate cancer, pancreatic cancer, lung cancer, cervical cancer, testicular cancer, skin cancer, bone cancer, thyroid cancer, sarcoma, glioblastoma, neuroblastoma, gastrointestinal cancer, neck and head tumor, adenoma, adenocarcinoma, keratoacanthoma, epidermoid carcinoma, large cell carcinoma, non-small cell lung cancer, Hodgkin's lymphoma and non-Hodgkin's lymphoma, follicular carcinoma, papillary carcinoma, seminoma, melanoma, acute myelogenous leukemia, chronic myelogenous leukemia, diffuse large B-cell lymphoma, activated B-cell-like diffuse large B-cell lymphoma, chronic lymphocytic leukemia, chronic lymphocytic lymphoma, primary effusion lymphoma, Burkitt lymphoma/leukemia, acute lymphocytic leukemia, B-cell prolymphocytic leukemia, lymphoplasmacytic lymphoma, Waldenstrom macroglobulinemia, splenic marginal zone lymphoma, intravascular large B-cell lymphoma, plasmacytoma, and multiple myeloma.
